# EUROPÄISCHE PATENTSCHRIFT

(11) **EP 1 149 914 B1**
(45) Veröffentlichungstag und Bekanntmachung des Hinweises auf die Patenterteilung: **18.06.2008**
(21) Anmeldenummer: 01107702.1
(22) Anmeldetag: 29.03.2001
(51) Int. Cl.: C12N 15/62, C07K 16/00

(54) **Fusionsprotein zur gleichzeitigen Erkennung von humanen Autoantikörpern**
A fusion protein that recognizes human autoantibodies simultaneously
Proteine fusionnèe qui reconnaît les auto-anticorps humain simultanement

(30) Priorität: 10.04.2000 DE 10017782; 25.05.2000 DE 10025840
(43) Veröffentlichungstag der Anmeldung: 31.10.2001
(73) Patentinhaber: Labor Dr. Merk und Kollegen GmbH, 88416 Ochsenhausen (DE)
(72) Erfinder: Richter, Wiltrud, Orthopäd. Uniklinik Heidelberg, 69118 Heidelberg (DE); Rickert, Mathias, Orthopäd. Uniklinik Heidelberg, 69118 Heidelberg (DE); Rapp, Ingrid, Beim Braunland 1 88416 Ochsenhausen (DE); Dangel, Werner, Beim Braunland 1 88416 Ochsenhausen (DE)
(74) Vertreter: Vossius & Partner

(56) Entgegenhaltungen:
- EP-A- 0 940 470
- WO-A-95/07992
- US-A- 5 998 366
- US-A- 5 998 584

## Beschreibung

Die vorliegende Erfindung betrifft ein Fusionsprotein, das am N-Terminus ein oder mehr Epitope umfasst, die einen Autoantikörper gegen das Inselzellantigen IA2 spezifisch binden, und am C-Terminus Epitope umfasst, die einen Antikörper gegen die Glutamatdecarboxylase GAD65 spezifisch binden, wobei
(i) das Fusionsprotein spezifisch an die nachfolgend unter (a), (b), (c) und (d) definierten Antikörper bindet:
   (a) MICA 2 (SEQ ID NR: 9 und 11) oder MICA 7 (SEQ ID NR: 29 und 31);
   (b) MICA 4 (SEQ ID NR: 17 und 19) oder MICA 6 (SEQ ID NR: 25 und 27);
   (c) MICA 1 (SEQ ID NR: 5 und 7) oder MICA 3 (SEQ ID NR: 13 und 15); und
   (d) MICA 9 (SEQ ID NR: 33 und 35); wobei
(ii) Epitope des Inselzellantigens IA2 Epitope aus der Aminosäuresequenz der Aminosäuren 606 bis 979 von SEQ ID NO: 2 oder einer Allelvariation davon sind und Epitope der Glutamatdecarboxylase GAD65 Epitope aus der Aminosäuresequenz der Aminosäuren 1 bis 585 von SEQ ID NO: 1 oder einer Allelvariation davon sind.
Ferner betrifft die Erfindung Polynucleotide, die das Fusionsprotein codieren. Die Erfindung betrifft ebenfalls Verfahren und Wirtszellen zur Herstellung des Fusionsproteins. Insbesondere betrifft die Erfindung diagnostische Kits, die ein erfindungsgemäßes Protein enthalten und deren Verwendung zum Nachweis von Autoimmunantikörpern, vorzugweise zum Nachweis von Diabetes mellitus Typ 1.

Diabetes mellitus Typ 1 ist eine der am weitesten verbreiteten Stoffwechselkrankheiten. In den Vereinigten Staaten sind ungefähr 0,3 bis 0,4% der Bevölkerung an Diabetes mellitus Typ 1 erkrankt und epidemiologische Studien belegen, daß dieser Anteil steigt. Diabetes mellitus Typ 1 entsteht durch die selektive Zerstörung von insulinproduzierenden β-Zellen des Pankreas durch Lymphozyten, die die Pankreasinsellzellen infiltrieren. Die typische Diabetes mellitus Typ 1 Hyperglykämie tritt erst auf, wenn mindestens 80% der insulinproduzierenden β-Zellen verloren wurden. Die verbleibenden β-Zellen werden in den folgenden Jahren zerstört. Obwohl Insulintherapien den Diabetes mellitus Typ 1-Patienten ein normales Leben erlauben, ist dieser Ersatz nur unzureichend und führt nicht zu einer vollständigen Wiederherstellung der Stoffwechselhornöostase. Folglich leiden Diabetes mellitus Typ 1-Patienten häufig an den Folgen von Disfunktion des Auges, der Niere, des Herzens oder anderer Organe. Deshalb ist es wünschenswert, die Zeitspanne zwischen der Latenszeit, in der die β-Zell-Zerstörung beginnt und dem tatsächlichen Erfordernis einer Insulintherapie zu verlängern, bzw. Therapien zu entwickeln, die die Insulinabhängigkeit ganz verhindern.

Viele Diabetes mellitus Typ 1. Patienten haben in ihren Seren Antikörper gegen beispielsweise die Glutamatdecarboxylase 65 (GAD65), das Inselzellantigen 2 (IA2) und/oder gegen Insulin.
GAD65 und IA2 sind zwei Proteine, die neben verschiedenen Körpergeweben vor allem im zentralen Nervensystem und in den β-Zellen des Pankreas exprimiert werden (Schranz, 1998). Autoantikörper gegen diese zwei Proteine können bei Patienten mit Diabetes mellitus Typ 1 und gesunden Patienten mit einem erhöhten Risiko Diabetes mellitus Typ 1 zu entwickeln diagnostiziert werden. Mehr als 80% der Patienten, bei denen Diabetes mellitus Typ 1 frisch manifestiert ist, haben wenigstens einen der genannten Autoantikörper (Leslie, 1999). Das Risiko bei Verwandten von Diabetes mellitus Typ 1 Patienten selbst an Diabetes mellitus Typ 1 zu erkranken erhöht sich mit steigender Anzahl verschiedener Autoantikörper (Dittler, 1998; Verge, 1996). Folgestudien haben gezeigt, daß gesund bleibende Personen, in Bezug auf Diabetes mellitus Typ 1, zeitweise einen Autoantikörper gegen die oben genannten Antigene aufweisen können. In Kombination treten die genannten Autoantikörper aber bei dauernd gesunden Personen nur äußerst selten auf.

Des weiteren gibt es Hinweise darauf, daß insulinabhängiger Diabetes mellitus (Diabetes mellitus Typ 1) zusammen mit anderen nicht-β-Zellen spezifischen Autoimmunkrankheiten auftritt. So wurde von Pietropaolo, 1998, gezeigt, daß IA2-Autoantikörper bei AITD (autoimmune thyroid diseases) zusammen mit Diabetes mellitus Typ 1 nachweisbar sind und GAD65-Autoantikörper in 67,7% der Patienten mit AITD, die ebenfalls Diabetes mellitus Typ 1 haben und bei 5,5% der Patienten mit PBC (primary biliary cirrhosis) auftreten.

Ein diagnosischer Test, mit dem der Beginn der β-Zellen-Zerstörung festgestellt werden könnte, würde Behandlungen zur Wiederherstellung der Toleranz gegen β-Zellen mit Immunsupressiva frühzeitig erlauben und somit zu einer Verzögerung im Beginn der durch Insulinzugabe bewirkten Folgen erlauben, bzw. die Insulinabhängigkeit ganz verhindern.
Für eine frühzeitige Behandlung, die die oben genannten Folgeerkrankungen verzögert oder verhindert, ist daher eine rechtzeitige Risikoabschätzung an Diabetes mellitus Typ 1 zu erkranken wichtig (Dittler, 1998; Borg, 1997; Christie, 1997).

Bisher sind eine Anzahl verschiedener Testmethoden zum Nachweis von Diabetes mellitus Typ 1 entwickelt worden. Diese Tests basieren zum Großteil auf Radio-Immuno-Assays (RIA) oder Enzmye Linked lmmunosorbant Assay (ELISA). Dabei wird auf Autoantikörper (Pietropaolo, 1998; Wiest-Ladenburger, 1997) getestet.

Einige dieser Testmethoden beziehen den Nachweis von Autoantikörpern gegen Insulin oder Preproinsulin mit ein. Etwa 20% der Seren von neu an Diabetes mellitus Typ 1 erkrankten Personen enthalten Insulin Autoantikörper (Sabbah, 1996). Untersuchungen haben ergeben, daß unter Umständen die Generierung von Autoantikörpern gegen Insulin über Prepro- oder Proinsulin (PPINS oder PINS) verläuft (Snorgaard, 1996). Neuere Forschungsergebnisse sind aber sehr kontrovers, was den Nutzen von Autoantikörpern gegen Insulin, Prepro- oder Proinslulin in Bezug auf die Risikoabschätzung an Diabetes mellitus Typ 1 zu erkranken, angeht (Ellis, 1999; Semana, 1999; Bosi, 1998; Harrop, 1992). Bevor nicht eindeutiger der Nutzen von Autoantikörpern gegen Insulin, Prepro- oder Proinsulin bei der Risikoabschätzung an Diabetes mellitus Typ 1 zu erkranken geklärt ist, sollte auf die Einbeziehung dieser Autoantikörper im Testsystem verzichtet werden.

Petersen (1994) verwendet ein in vitro Transkription/Translation-Vertahren zur Produktion von radioaktiv markierten Autoantigenen während in einem anderen Verfahren Biotin markierte GAD65 zu Patientenseren gegeben und danach die Formation von Immunkomplexen detektiert und quantifiziert wurde (Mehta, 1996). Diese Verfahren besitzen jedoch einen geringen Informationsgehalt.

Aus den vorstehenden Ausführungen wird deutlich, daß ein Bedarf an diagnostischen Assays und Verfahren besteht, mit denen frühzeitig Diabetes mellitus Typ 1 und andere Autoimmunkrankheiten, wie z.B. Stiff-Man-Syndrom oder polyglanduläres Autoimmunsyndrom, mit einer hohen Spezifität und Sensibilität schnell und einfach nachzuweisen sind.

Diese Aufgabe wird durch die Bereitstellung der in den Patentansprüchen bezeichneten Ausführungsformen gelöst.

Die vorliegende Erfindung betrifft somit ein Fusionsprotein, das am N-Terminus ein oder mehr Epitope umfasst, die einen Autoantikörper gegen das Inselzellantigen IA2 spezifisch binden, und am C-Terminus Epitope umfasst, die einen Antikörper gegen die Glutamatdecarboxylase GAD65 spezifisch binden, wobei
(i) das Fusionsprotein spezifisch an die nachfolgend unter (a), (b), (c) und (d) definierten Antikörper bindet:
   (a) MICA 2 (SEQ ID NR: 9 und 11) oder MICA 7 (SEQ ID NR: 29 und 31);
   (b) MICA 4 (SEQ ID NR: 17 und 19) oder MICA 6 (SEQ ID NR: 25 und 27);
   (c) MICA 1 (SEQ ID NR: 5 und 7) oder MICA 3 (SEQ ID NR: 13 und 15); und
   (d) MICA 9 (SEQ ID NR: 33 und 35); wobei
(ii) Epitope des Inselzellantigens IA2 Epitope aus der Aminosäuresequenz der Aminosäuren 606 bis 979 von SEQ ID NO: 2 oder einer Allelvariation davon sind und Epitope der Glutamatdecarboxylase GAD65 Epitope aus der Aminosäuresequenz der Aminosäuren 1 bis 585 von SEQ ID NO: 1 oder einer Allelvariation davon sind.

Der Begriff "spezifisch bindet" betrifft die Affinität der Antikörper zu dem Epitop. Vorzugsweise weist der Antikörper eine Affinität von mindestens 10⁻⁷ M, vorzugsweise 10⁻⁸ M, mehr bevorzugt 10⁻⁹ M, besonders bevorzugt 10⁻¹⁰ M und am meisten bevorzugt 10⁻¹¹ M auf.
Der Begriff "Epitop" im Sinne der vorliegenden Erfindung umfaßt den Bereich eines Antigens, der mit einem Antikörper bindet. In einem Polymer, wie z.B. einem Protein, umfaßt der Begriff "Epitop" sowohl lineare Bereiche, die eine von einem Antikörper erkennbare Struktur bilden können, als auch Bereiche, die aufgrund der Faltung des zugrundeliegenden Moleküls, z.B. der Tertiär- oder Quartärstruktur eines Proteins, von einem Antikörper erkannt werden (Konformationsepitope).

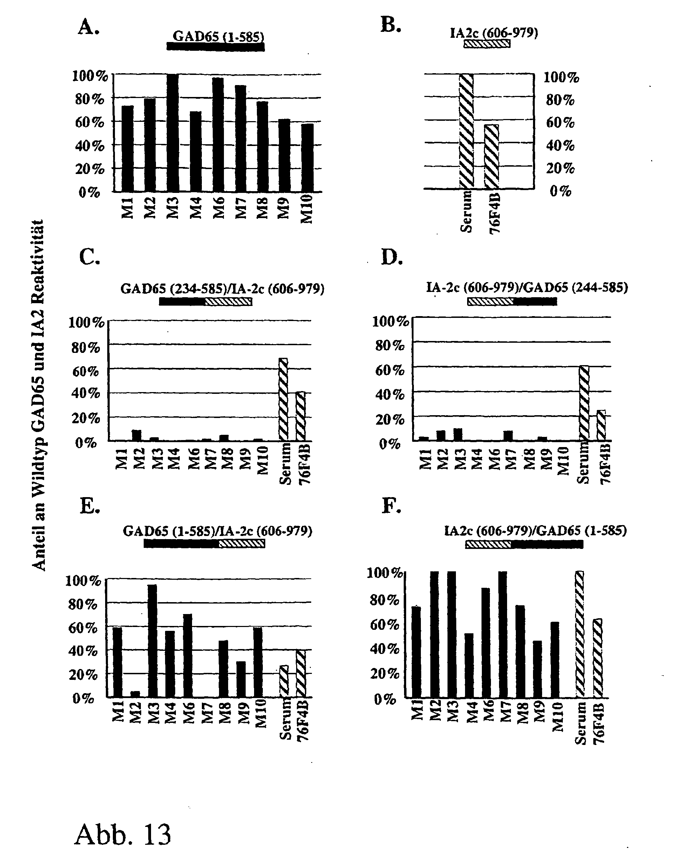

In EP-A 0 940 470 wird vorgeschlagen, Epitope der Autoantigene GAD65, PPINS und IA2 beliebig miteinander zu verbinden, um so Fusionsproteine zum Nachweis von Autoantikörpern, die diese Epitope spezifisch binden, herzustellen. Insbesondere wird vorgeschlagen, bestimmte Fusionsproteine herzustellen und zu verwenden, die Aminosäuresequenzen von GAD65, 1A2 und PPINS Protein enthalten. Es wird allerdings nicht beschrieben, ob sich diese Fusionsproteine zum gleichzeitigen Nachweis der jeweiligen Autoantikörper eignen. In einleitenden Experimenten zu der vorliegenden Erfindung wurde versucht, ein Fusionsprotein herzustellen, das N-terminal die Aminosäuren 1 bis 585 der GAD65 und C-terminal die Aminosäuren 606 bis 979 des IA2 umfaßt. Jedoch konnte dieses Fusionsprotein nicht in SF9-Zellen exprimiert werden, einem Expressionssystem wie es in der EP-A 0 940 470 vorgeschlagen wird (siehe Beispiel 2). Statt dessen wurde dann versucht, das Fusionsprotein in einem in vitro Transkriptions/Translationssystem in Retriculozyten-Lysat zu synthetisieren. Die Integrität der Konformation der GAD65-Domänen von GAD65/IA2-Fusionsproteinen wurde mit humanen monoklonalen Diabetes-assoziierten Antikörpern in Radioimmunassays geprüft. Diese Antikörper definieren 9 Konformationsepitope in GAD65, wovon 7 in den Aminosäuren 240 und 585 der GAD65 liegen (Richter, 1993; Syren, 1996; Schwartz, 1999). Um die Konformation des IA2-Teils zu testen wurde ein monoklonaler Mausantikörper (76F4B) verwendet, der ein membrannahes zytoplasmatisches Epitop erkennt. Das hergestellte Fusionsprotein bindet jedoch in dem durchgeführten Konformationsbestimmungsassays alle verschiedenen monoklonalen Antikörper gegen GAD65 und gegen IA2 gar nicht oder nur schlecht. Das Fusionsprotein scheint somit eine Faltung zu besitzen, die die Bindung und den Nachweis von Autoantikörpern nur bedingt erlaubt. Insbesondere wurde mit den Autoantikörpern MICA 7 keine und mit MICA 2 nur eine sehr geringe Reaktivität beobachtet (siehe Abb. 13). Das Fusionsprotein weist zudem eine stark verringerte Antikörperbindung an fast allen anderen bekannten GAD65 und IA2-Epitopen auf. Dagegen reagieren die zur Kontrolle einzeln exprimierten Proteine GAD65₍₁₋₅₈₅₎ und IA2₍₆₀₆₋₉₇₉₎ mit allen getesteten Antikörpern sehr gut.

Um auf die Größe des Proteins zurückzuführende Faltungsprobleme auszuschließen, wurde in einem weiteren Experiment ein verkürztes GAD65/IA2-Fusionsprotein getestet (Abb. 13). Dieses Fusionsprotein bindet jedoch keinen getesteten GAD65-Autoantikörper und die getesteten IA2-Autoantikörper nur sehr schlecht. Somit ist auch ein verkürztes GAD65/IA2-Fusionsprotein nicht dazu geeignet, Autoantikörper, z.B. in Seren nachzuweisen. Die vorstehend diskutierten Experimente weisen daher daraufhin, daß GAD65 und IA2 Protein negativ interagieren und dies dazu führt, daß Fusionen von diesen beiden Proteinen immunologisch inaktiv oder deren Fähigkeit Autoantikörper zu binden zumindest stark reduziert sind.

Im Rahmen der vorliegenden Erfindung wurde jedoch in einem neuen experimentellen Ansatz überraschenderweise festgestellt, daß ein Fusionsprotein, bei dem die Reihenfolge der Autoimmunepitope geändert wurde, die Fähigkeit besitzt, alle bekannten GAD65-Epitope und die getesteten IA2-Epitope zu bilden. Epitopkonformationsstudien zeigen, daß die Antikörper MICA 1-10 und 76F4b mit einem Fusionsprotein reagieren, bei dem die IA2-Epitope N-terminal und die GAD65-Epitope C-terminal vorliegen. In Abb. 13 wird gezeigt, daß das erfindungsgemäße Fusionsprotein zudem die monoklonalen Antikörper mit einer Reaktvität binden, die jeweils mindestens 70% der Wildtypreaktivität beträgt. Bei dem erfindungsgemäßen Fusionsprotein bilden sich die Konformationsepitope in der korrekten Faltung und erlauben so überraschenderweise den Nachweis von Autoimmunantikörpern mit hoher Sensitivität und Spezifität.
Der vorliegenden Erfindung liegt somit die überraschende Beobachtung zu Grunde, daß die Abfolge der Epitope in dem erfindungsgemäßen Fusionsprotein entscheidend für die Reaktivität des Proteins mit Antikörpern ist.

In den dieser Erfindung zugrunde liegenden Experimenten wurde beobachtet, daß verglichen mit Wildtyp GAD65 die Fusionsproteine GAD65₍₂₃₄₋₅₈₅₎/IA2₍₆₀₆₋₉₇₉₎ und IA2₍₆₀₆₋₉₇₉₎/GAD 65₍₂₃₄₋₅₈₅₎ vollständig die Reaktivität mit MICA 1 bis 10 verlieren, obwohl die in Fusionsproteinen vorhandenen GAD65-Sequenzen erwarten ließen, daß alle MICA außer MICA 8 und MICA 9 reagieren würden (Syren, 1996). Die Erkennung der IA2 Domänen war in beiden Konstrukten konserviert, zeigte jedoch eine im Vergleich zum Wildtyp IA2 Molekül reduzierte Aktivität. Dementsprechend umfaßt das Fusionsprotein vorzugsweise längere Aminosäuresequenzen oder betrifft Fusionsproteine, die die Epitope der GAD65 nachbilden können.

Ob das erfindungsgemäße Fusionsprotein mit einem Antikörper reagiert, kann mit dem in den Beispielen aufgeführten Testsystem bestimmt werden. Dem Fachmann sind weitere Testsysteme bekannt. Zum Beispiel Immunpräzipitation, ELISA, Radioimmunassay (RIA), Immundiffusionstest, BIA-core Testverfahren.

Das erfindungsgemäße Fusionsprotein kann als Ausgangspunkt für die Entwicklung von analogen Proteinen dienen, die mit Autoantikörpern reagieren. Verfahren hierfür sind dem Fachmann bekannt. So können Faltungssimulationen und Computermodulierungen von Strukurmotiven, insbesondere von Epitopen, des erfindungsgemäßen Fusionsproteins mit bekannten Computerprogrammen durchgeführt werden (Olszewski, Proteins 25 (1996), 286-299: Hoffman, Comput. Appl. Biosci. 11 (1995), 675-679). "Computer modelling" der Proteinfaltung kann für Konformations- und Energieanalysen von Peptiden und Proteinen verwendet werden (Monge, J. Mol. Biol. 247 (1995), 995-1012; Renouf, Adv. Exp. Meta. Biol. 376 (1995), 37-45). Insbesondere können bestimmte Programme verwendet werden, um die Wechselwirkungen zwischen Antikörpern und den Konformationsepitopen zu identifizieren. Weitere Computersysteme zum Design von Proteinen und Peptiden sind im Stand der Technik beschrieben (z.B. in Berry, Biochem. Soc. Trans. 22 (1994), 1033-1036; Wodaka, Ann. N. Y. Acad. Sci. 501 (1987), 1-13; Pabo, Biochemistry 25 (1986), 5987-5991). Die Ergebnisse der oben genannten Computeranalysen können verwendet werden, um z.B. Peptidomimetics des erfindungsgemäßen Proteins oder Fragmente davon herzustellen. Solche Pseudopolypeptidanaloga der natürlichen Aminosäuresequenz des Proteins können effizient das Vorgängerprotein kopieren (Benkirane, J. Biol. Chem. 271 (1996), 33218-33224). Weiterhin können dreidimensionale und/oder kristallographische Strukturen des Fusionsproteins verwendet werden, um die Wechselwirkung zwischen den Antikörpern und Epitopen zu untersuchen. Der Fachmann kann aufgrund der sich aus den genannten Verfahren erhaltenen Informationen und aufgrund des Fachwissens über den Einfluß von Aminosäuresequenzen auf die Struktur eines Peptids das erfindungsgemäße Fusionsprotein in seiner Aminosäuresequenz verändern ohne die Konformation der jeweiligen Epitope zu verändern oder die Antikörperbindung durch Änderungen in der Primärstruktur erleichtern oder verbessern. Nicht jede dieser Veränderungen wird zu einem korrekt gefalteten Fusionsprotein führen (Beispiel 3). Mit Hilfe der in den Beispielen genannten Testsysteme läßt sich jedoch routinemäßig die Konformation und somit die Antikörper-Epitopwechselwirkung des Fusionsproteins bestimmen.

In einer besonders bevorzugten Ausführungsform bindet das Fusionsprotein spezifisch die im Patentanspruch genannten MICA-Antikörper. Die Sequenzen der variablen Regionen der
schweren und leichten Antikörperketten von MICA 1, 2, 3, 4, 5, 6, 7, 9 und 10 sind bekannt (SEQ ID N0:5-40).
GAD65 besitzt mehrere verschiedene Epitope, die von Autoantikörpern erkannt werden. Epitopstudien haben gezeigt, daß die unterschiedlichen autoreaktiven Epitope praktisch über die gesamte Oberfläche des Proteins verteilt sind und meist Konformationsepitope darstellen.
IA2 gehört zur Proteinfamilie der Protein Tyrosin-Phosphatasen (Krueger, 1990). Alle Vertreter dieser Proteinfamilie sind Transmembranproteine. Bis heute sind nur autoreaktive Epitope von Diabetes mellitus Typ 1 Autoantikörpern gefunden worden, die zum cytoplasmatischen Teil von IA2 (As 601-979) gehören. Epitopstudien am IA2 Molekül haben gezeigt, daß die Autoantikörper entweder den membrannahen Abschnitt (As 606-682), die PTP ähnliche Domäne (As 777-979) am C-Terminus, oder beide Bereiche zusammen erkennen. Wie beim GAD65 Molekül sind auch beim IA2 Molekül die meisten gefunden autoreaktiven Epitope Konformationsepitope (Lampasona, 1996; Xie, 1997).
Söhnlein, 2000, berichtet, daß GAD65 Antikörper mit 3 Konformations- (EP-1, EP-2, EP-3) und zwei linearen Epitopclustern (Aminosäuren 1-124 und 535-585) reagieren. Die Autoimmunantikörper MICA 4, 6 und 10 kompetitieren miteinander und binden somit sterisch benachbarte Epitope innerhalb des Epitopclusters EP-1. Ein unterschiedliches aber identisches Kompetitionsmuster wird für die Antikörper MICA 1 und 3 beobachtete (Epitopcluster EP-2). Die Antikörper MICA 2, 5, 7, 8 und 9 kompetitieren ebenfalls um die Bindung mit GAD65 und definieren somit ein drittes Epitopcluster (EP-3). Vorzugsweise weist das Fusionsprotein dementsprechend mindestens eines dieser Epitopcluster auf. Die in den Beispielen gezeigten Konformationsstudien zeigen zudem, daß die Konformation der durch die Autoantikörper MICA 2 und 7 erkannten Epitope unabhängig von der Integrität der restlichen Konformationsepitope ist. Damit das erfindungsgemäße Fusionsprotein alle autoreaktiven Bereiche des GAD65 Proteinmoleküls abdeckt, hat das erfindungsgemäße Fusionsprotein vorzugsweise die oben genannten Epitopcluster. In einer Ausführungsform reagiert das erfindungsgemäße Fusionsprotein jedoch nur mit einer der oben genannten Gruppen an monoklonalen Antikörpern und erkennt somit eine bestimmte Gruppe an Autoimmunantikörpern. Ein solches Fusionsprotein kann z.B. für die Bestimmung von Änderungen in der Epitoperkennung von Autoantikörpern während eines Krankheitsverlaufs verwendet werden oder um unterschiedliche Autoimmunkrankheiten wie z.B. Diabetes, PAS (polyendocrine autoimmune syndrome), "stiff man" Syndrom, nichtkrankheitsspezifische Epitope oder Epitopprofile zu detektieren (Söhnlein, 1999).

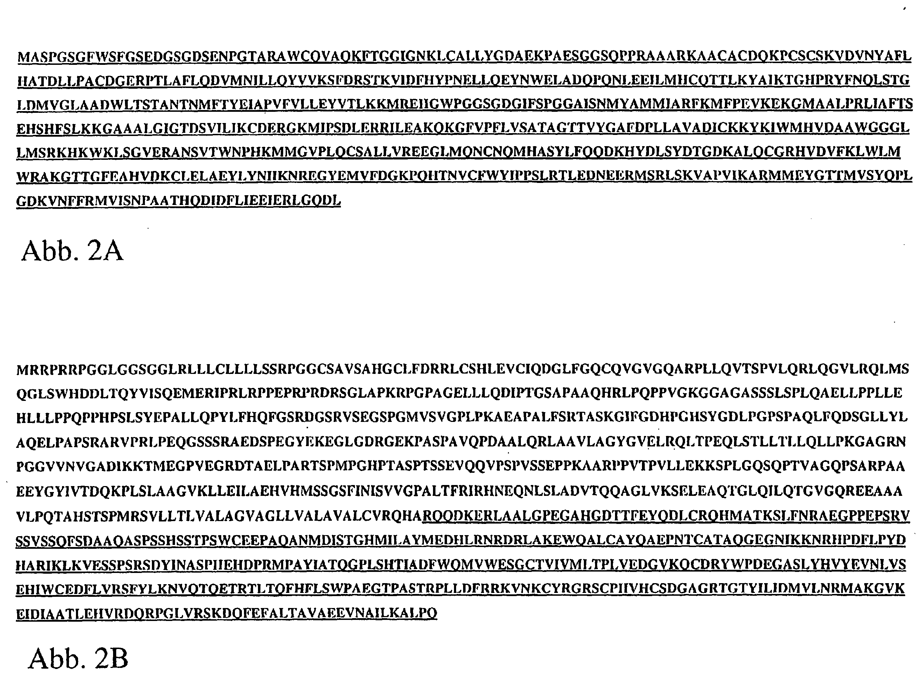

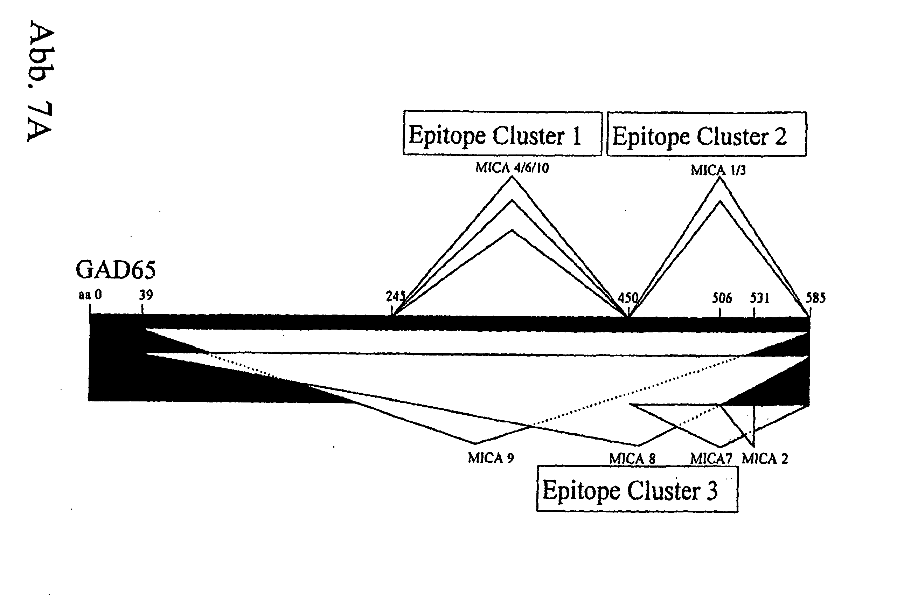

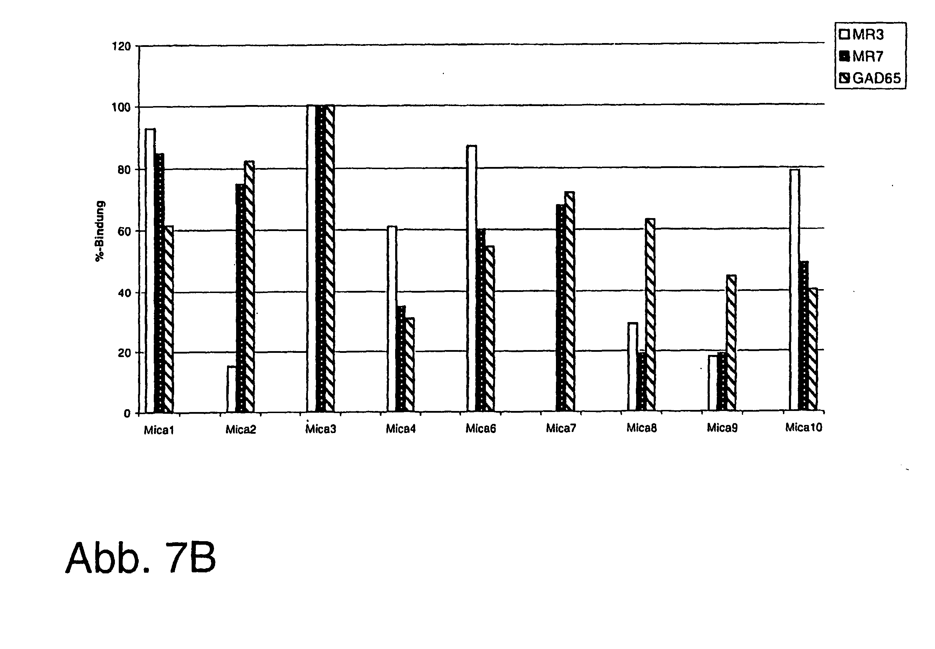

In einer weiteren bevorzugten Ausführungsform bindet das erfindungsgemäße Fusionsprotein mit den Antikörpern MICA 1, 2, 3, 4, 6, 7 und 9 spezifisch und besitzt somit ein weiteres Spektrum an Autoimmunepitopen.
Epitopstudien an den genannten Autoantigenen haben gezeigt, daß die autoreaktiven Epitope mit wenigen Ausnahmen Konformationsepitope sind. Um ein möglichst breites Spektrum an Autoantikörpern detektieren zu können besitzt das erfindungsgemäße Fusionsprotein vorzugsweise alle bisher gefundenen autoreaktiven Epitope der genannten Autoantigene ins Fusionsantigen integriert (Schwarz, 1999; Syren, 1996). Bei Deletion von As 1-102 gehen z.B. nachweislich Konformationsepitope verloren (Syren, 1996). Vorzugsweise besitzt daher das Fusionsprotein alle drei beschriebenen GAD 65-Epitopcluster (EP1-EP3, Söhnlein, 2000) und beim IA2-Anteil den intrazellulären antigenen Bereich. Um das zu erreichen, umfaßt der GAD65-Proteinanteil des Fusionsproteins vorzugsweise Aminosäuren 1 bis 585 und der IA2 Proteinanteil die Aminosäuren 606 bis 979 (siehe Abb. 2a und 2b und die Abb. 7a und b).

Zum Nachweis von IA2-Autoimmunantikörpern bindet das erfindungsgemäße Fusionsprotein auch spezifisch einen oder mehrere der in der Literatur beschriebenen Antikörper gegen IA2, z.B. in Lampasona, J. of Immunology 157 (1996), 2707-2711; Xie, J. of Immunology 159 (1997), 3662-3667.

Aminosäuresequenzen, die die Epitope des erfindungsgemäßen Proteins umfassen, können prinzipiell aus jedem Organismus stammen, allelische Varianten der in GAD65 und IA2, insbesondere der in SEQ ID Nr. 1 bis 4, umfaßten Epitope darstellen oder aufgrund der oben beschriebenen oder dem Fachmann bekannten Verfahren hergestellte Derivate dieser Epitope umfassen, solange diese Sequenz mit einem Autoantikörper gegen GAD65 und/oder IA2 spezifisch reagiert. Abweichungen zu den in SEC ID Nr. 1 bis 4 umfaßten Epitopen können dabei z.B. durch Deletionen, durch Substitutionen, Insertionen oder Rekombinationen entstanden sein.
Der Fachmann weiß, daß bei Konformationsepitopen die zugrundeliegende primäre Sequenz an Positionen, die für die Konformationsbildung und/oder die Wechselwirkung mit einem Antikörper unerheblich ist, nur gering konserviert ist und entsprechend geändert werden kann.

In einer weiteren Ausführungsform betrifft die vorliegende Erfindung ein Fusionsprotein, in dem ein oder mehr Epitope der GAD65 und ein oder mehr Epitope des IA2 ohne Verbindungspeptid miteinander verbunden sind.
Der Fachmann weiß, daß bereits geringe Änderungen der Primärstruktur eines Proteins zu Konfirmationsänderungen führen kann und somit Änderungen in der Epitoppräsentation dieser Proteine. Deshalb wird der Fachmann vorzugsweise vermeiden, Sequenzen des erfindungsgemäßen Fusionsproteins ohne vorherige Analyse, z.B. mit den oben beschriebenen Verfahren zu ändern und vorzugsweise ein Fusionsprotein einsetzen, in dem die verschiedenen Epitope so miteinander verbunden sind wie im Wildtyp, oder zumindest das Einsetzen neuer Sequenzen, z.B. Linker, die mit nicht-krankheitsrelevanten Antikörpern reagieren können, vermeiden.

In einer weiteren Ausführungsform ist das erfindungsgemäße Fusionsprotein an eine Festphase gekoppelt.
Das erfindungsgemäße Fusionsprotein kann direkt oder indirekt an eine feste Phase gekoppelt werden. Für eine indirekte Koppelung wird das Fusionsprotein über ein weiteres Molekül, z.B. einen Antikörper, mit der Festphase verbunden. Vorzugsweise werden biotinylierte monoklonale Antikörper an eine Streptavidin-beschichtete Mikrotiterplatte oder eine andere Festphase, z.B. eine Säule, gebunden. Durch die Inkubation der Festphase mit dem erfindungsgemäßen Fusionsprotein kann dann das Fusionsprotein immobilisiert werden. Das immobilisierte Fusionsprotein kann mit z.B. auf Antikörper zu testenden Proben inkubiert werden und deren Bindung detektiert werden, um Autoimmunkrankheiten zu diagnostizieren oder prognostizieren.

Das erfindungsgemäße Fusionsprotein kann auch direkt an die Festphase gebunden werden, z.B. durch direkte Adsorption oder die Einführung von kurzen Sequenzen, die mit der Festphase reagieren können. Ein Protein kann z.B. über kurze eingeführte Lysinsequenzen und einen Biotinrest an eine feste Streptavidinphase binden. In der EP-A 0 940 470 sind solche Linker beschrieben. In der Literatur sind weitere Gruppen bekannt, mit der das erfindungsgemäße Protein direkt kovalent an eine Festphase gebunden werden kann.

In einer weiteren Ausführungsform ist das erfindungsgemäße Fusionsprotein markiert. Eine solche Markierung kann z.B. eine Biotinylierung, einen fluoreszierende, chemilumineszierenden oder biolumineszierenden Stoff, ein Kolloidalmetal, ein Enzym, eine radioaktive Markierung und/oder einen Protein-taq umfassen. Solche Markierungen umfassen z.B. Enzymmarkierungen, z.B. Glykoseoxidase, Radioisotope, wie z.B. 125-I, 121-I, 14-C, 35-S, 3-H, 112-In oder 99m-Tc, fluoreszierende Markierungen, wie z.B. Fluoreszein, Rhodamin oder GFP oder Biotin. Ebenfalls umfaßt sind indirekte Markierungen durch bindende Proteine, wie z.B. Antikörper, die z.B. mit alkaliner Phosphatase, Peroxidase oder einem anderen Enzym oder Molekül zum Nachweis verbunden sind. Z.B. zum Nachweis von gebundenen Autoantikörpern oder monoklonalen Antikörpern in einem Test zur Diagnose oder Prognose einer der genannten Autoimmunkrankheiten kann das erfindungemäße Fusionsprotein oder der Antikörper, der dieses Fusionsprotein erkennt, markiert werden.

Eine weitere Ausführungsform umfaßt ein Polynucleotid, das das erfindungsgemäße Fusionsprotein codiert.

Vorzugsweise ist das erfindungsgemäße Polynucleotid DNA oder RNA.

Die vorliegende Erfindung betrifft weiterhin einen Vektor, insbesondere Plasmide, Cosmide, Viren, Bakteriophagen, und andere in der Gentechnik übliche Vektoren, die ein erfindungsgemäßes Polynukleotid umfassen.
In einer bevorzugten Ausführungsform ist der erfindungsgemäße Vektor ein Expressionsvektor.
Der Begriff "Expressionsvektor" umfaßt im Sinne der vorliegenden Erfindung sowohl prokaryontische als auch eukaryontische Expressionsvektoren. Die notwendigen regulatorischen Elemente zur Expression eines Polypeptids sind dem Fachmann wohl bekannt und können den Expressionsbedingungen entsprechend ausgewählt werden. Der Begriff "Expression" kann dabei Transkription als auch Transkription und Translation bedeuten. Regulatorische Elemente umfassen dabei insbesondere Promotoren. Für die Expression eines erfindungsgemäßen Polynucleotids in prokaryontischen Zellen stehen eine Reihe von Promotoren zur Verfügung, z.B. der E. coli lac- oder trp-Promotor, der P_{R}- oder P_{L}-Promotor des Lambda-Phagen, lacl, lacZ, T3, T7, gpt, etc. Eukaryontische Promotoren sind beispielsweise der CMV immediate early-Promotor, der HSV-Promotor, der Thymidinkinase-Promotor, der SV40-Promotor, der LTRs von Retroviren und der Maus Metallothionin-Promotor. Es ist bereits eine Vielzahl von Expressionsvektoren für die Expression in prokaryontischen oder eukaryontischen Zellen beschrieben, z.B. für Eukaryonten pKK223-3 (Pharmacia Fine Chemicals, Uppsala, Schweden), GEM1 (Promega Biotec, Madison, WI, USA), pSV2CAT oder pOG44 und für Prokaryonten pQE70, pQE60, pBluescript SK, etc. Neben Promotoren können erfindungsgemäße Vektoren auch Elemente zur weiteren Steigerung der Transkription oder Translation enthalten, wie z.B. sogenannte Transkriptions-Enhancer. Beispiele dafür sind der SV40-Enhancer, der Polyoma-Enhancer, der Cytomegalovirus early Promoter-Enhancer und Adenovirus-Enhancer.

In einer weiteren Ausführungsform betrifft die Erfindung eine Wirtszelle, die ein erfindungsgemäßen Vektor enthält, insbesondere eine Wirtszelle, die mit einem solchen Vektor transformiert, transfiziert oder infiziert wurde.
Unter den Begriff "Wirtszelle" fallen erfindungsgemäß sowohl prokaryontische als auch eukaryontische Wirtszellen. Bevorzugte prokaryontische Wirtszellen umfassen z.B. E. coli-Zellen, Streptomyces-, Bacillus- oder Salmonella-Zellen. Bevorzugte eukaryontische Wirtszellen umfassen Pilzzellen, z.B. Hefezellen, insbesondere Saccharomyces cerevisae-Zellen, Insektenzellen wie z.B.
Drosophila- oder SF9-Zellen, tierische Zellen wie z.B. CHO- oder COS-Zellen, Pflanzenzellen oder Säugerzellen.

In einer weiteren Ausführungsform betrifft die Erfindung ein Verfahren zur Herstellung eines erfindungsgemäßen Fusionsproteins, das umfaßt:
(a) Züchten der erfindungsgemäßen Wirtszelle; oder
(b) in vitro Transkription und/oder Translation des erfindungsgemäßen Polynukleotids; und
(c) Isolierung des Fusionsproteins.

Die Züchtung der erfindungsgemäßen Wirtszelle erfolgt unter Bedingungen, die die Expression des Fusionsproteins bewirken.
Die Bedingungen, die eine Expression eines Proteins bewirken, sind dem Fachmann bekannt und können entsprechend der verwendeten Wirtszelle und des eingesetzten Expressionsvektors gewählt werden. Entsprechend gestaltet sich auch die Gewinnung des Expressionsproduktes. Wird z.B. ein Expressionsvektor eingesetzt, der zur Sekretion des synthetisierten (Poly)peptids führt, wird das (Poly)peptid aus dem Kulturüberstand gewonnen. Findet die Expression intrazellulär statt, wird das Expressionsprodukt aus der Wirtszelle isoliert.
Methoden zur in vitro Transkription und/oder Translation sind dem Fachmann ebenfalls bekannt. Beispielsweise lassen sich solche Verfahren mittels kommerzieller Kits entsprechend den Instruktionen des Herstellers durchführen. Vorzugsweise werden bei der Gewinnung des erfindungsgemäßen Fusionsproteins zusätzliche, dem Fachmann bekannte Reinigungsschritte, wie z.B. säulenchromatographische Verfahren, durchgeführt, um Verunreinigungen (z.B. zelluläre Proteine, Nucleinsäuren, Komponenten des in vitro Transkriptions-/Translations-Systems) weitestgehend zu entfernen.

Das erfindungsgemäße Verfahren zur Herstellung umfaßt auch die Expression des Proteins in einem zellfreiem System. Es beinhaltet die Transkription der codierenden DNA in ein RNA-Produkt, posttranskriptionelle Modifikationen und/oder Translation in ein Proteinprodukt sowie mögliche posttranslationale Modifikation. Posttranslationale Modifikation des Polypeptids sind z.B. Glykosylierung, Acetylierung, Phosphorylierung usw. Ein Fachmann weiß, daß es nicht nur möglich ist, das native Protein zu exprimieren, sondern auch daß ein Protein möglicherweise vorteilhaft durch bestimmte Signalsequenzen in bestimmte Kompartimente der Wirtszelle gelenkt wird, um so z.B. die Sezemierung des Proteins in das Kulturmedium zu ermöglichen. Das erfindungsgemäße Fusionsprotein kann auch als Protein mit einem oder mehr zusätzlichen Polypeptiddomänen exprimiert werden, die die Aufreinigung des Proteins erleichtern. Solche Domänen können z.B. Metallchelate bilden, Protein A-Domänen umfassen oder Domänen sein, die in dem FLAGF-Extensions-Affinitätsreinigungssystem verwendet werden (IMMUNEX Corporation, Seattle, WA). Schnittstellen, wie z.B. für den Faktor Xa oder Enterokinase (Invitro-Gen, San Diego, CA) zwischen den Reinigungsdomänen und dem Protein erleichtern die Reinigung und vermindern den Einfluß dieser Sequenzen auf die Faltung des Fusionsproteins.

In einer weiteren Ausführungsform betrifft die vorliegende Erfindung die Verwendung des erfindungsgemäßen Fusionsproteins zur Herstellung von Antikörpern.
Die Herstellung von Antikörpern ist dem Fachmann bekannt und umfaßt beispielsweise die Immunisierung eines Tieres mit einem Antigen, das gegebenenfalls an einen Carrier gekoppelt ist _und/oder in Kombination mit anderen immunstimulierenden Substanzen verabreicht wird (siehe z.B. Harlow und Lane, "Antibodies, a-laboratory manual", Cold Spring Harbor Laboratory Press, Cold Spring Harbor, 1988). Andere bekannte Methoden umfassen das Screening von Antikörperbibliotheken mittels "phage display"-Technologie und die rekombinante Herstellung des gewünschten Antikörpers.

In einer weiteren Ausführungsform betrifft die vorliegende Erfindung ein Verfahren zum Nachweis von Autoantikörpern, das umfaßt:
(a) Inkontaktbringen einer biologischen Probe mit dem erfindungsgemäßen Fusionsprotein, unter Bedingungen, die die Bindung von Antikörpern erlauben; und
(b) Nachweis der in Schritt (a) gebunden Antikörper.

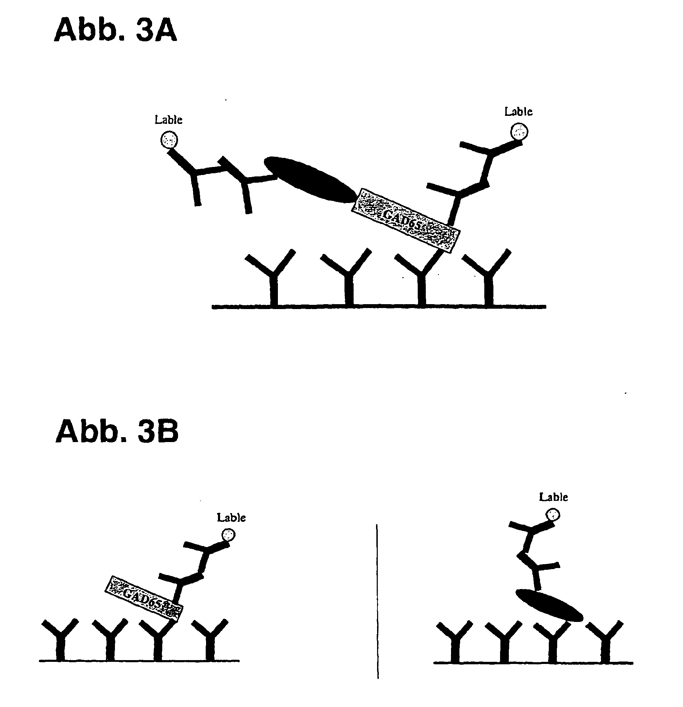

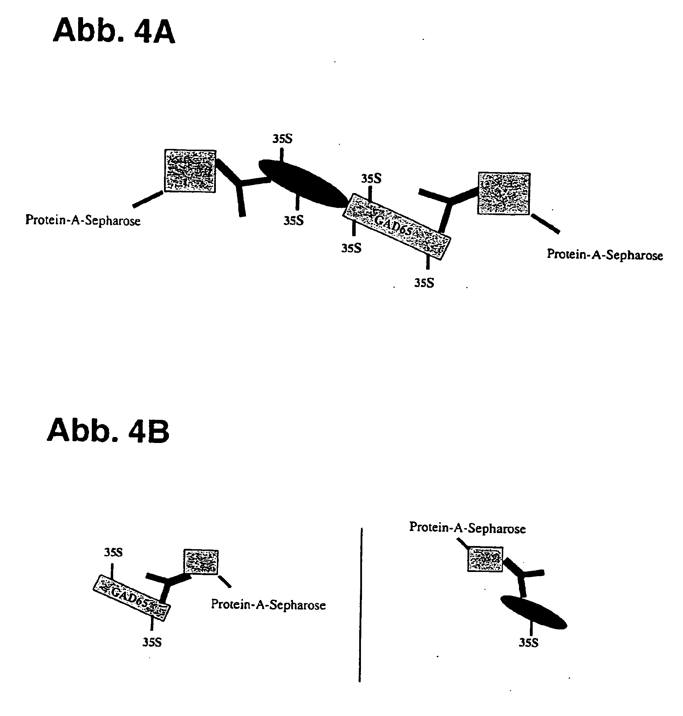

Das erfindungsgemäße Verfahren eignet sich zum Nachweis von Autoantikörpern und somit auch als prognostisches oder diagnostisches Indiz für die mögliche Entwicklung oder das Vorhandensein einer Autoimmunkrankheit wie z.B. Diabetes mellitus Typ I, Stiff Man-Syndrom, Polyglanduläres Autoimmunsyndrom.
Der Begriff "biologische Probe" im Sinne der vorliegenden Erfindung umfaßt eine behandelte oder unbehandelte Form einer Körperflüssigkeit von Zellkulturüberstand, oder Zellysat. Der Begriff "Körperflüssigkeiten" umfaßt im Sinne der vorliegenden Erfindung z.B. Blut, Serum, Lymphe, Gewebeflüssigkeit und Gewebeextrakte z.B. aus Mucosae respiratorischen, urogenitalen oder gastrointestinalen Ursprungs. Desweiteren umfaßt dieser Begriff Körperflüssigkeiten, die derart behandelt bzw. vorbereitet wurden, daß sie z.B. für den Einsatz in den oben erwähnten Methoden geeignet sind. Beispiele hierfür sind in vitro verdünnte Seren, mit Konservierungsstoffen (wie z.B. vor Kälte schützende Substanzen) versetzte Körperflüssigkeiten oder mit gerinnungshemmenden Stoffen (z.B. Heparin) behandelte Körperflüssigkeiten.
Vorzugsweise betrifft die vorliegende Erfindung auf die Verwendung des erfindungsgemäßen Fusionsproteins in Festphasen- und Flüssigphasenassays zum Nachweis von Diabetes mellitus Typ 1-Autoantikörpem in Seren von, z.B., Prädiabetes-Patienten oder Personen, die zunächst als Typ 2 Diabetiker eingestuft wurden. Mit den erfindungsgemäßen Verfahren können Personen aus der Normalbevölkerung selektiert werden, die im Begriff sind einen Diabetes Typ 1 zu entwickeln. Durch die Verwendung der genannten Fusionsproteine, die die autoreaktiven Epitopregionen der beiden bisher wichtigsten Autoantigene miteinander vereinigen, steigt der Informationswert der Tests in Bezug auf Risikoabschätzung an Diabetes mellitus Typ 1 zu erkranken. Erreicht wird dies unter anderem z.B. durch die Gliederung der Tests in zwei Phasen (siehe Abb. 3a/b und Abb. 4a/b). In Phase 1 sollen mit dem erfindungsgemäßen Verfahren alle Patienten diagnostiziert werden, die Autoantikörper gegen GAD65, IA2 oder beide Autoantikörper im Serum besitzen. In Phase 2 werden die aus Phase 1 positiv befundenen Patientenseren noch einmal getestet. Diesmal wird auf die beiden Autoantigene GAD65 und IA2 getrennt getestet. Durch die beiden Einzelmessungen in Phase 2 kann einmal der Titer der detektierten Autoantikörper bestimmt und durch aufeinander folgende Tests kann die Reihenfolge, in der Autoantikörper bei Patienten auftreten, festgestellt werden. Beide Größen sind wichtig für die Risikoabschätzung an Diabetes mellitus Typ 1 zu erkranken.

Vorzugsweise umfaßt das erfindungsgemäße Verfahren daher einen Schritt zur Quantifizierung der Antikörperbindung.

In einer Ausführungsform wird in dem erfindungsgemäßen Verfahren das Fusionsprotein vor oder nach der Inkubation mit der Probe an eine Festphase gekoppelt.
Die Erfindung betrifft somit einen Festphasentest zum Nachweis von Autoantikörpern, z.B. einen ELISA oder andere Schnelltestverfahren (z.B. Flow-Through-, Lateral Flow-Immunoassays usw. z.B. wie in US 4313734, US 4376110, US 4435504, US 4703017, US 4855240, US 4954452, US 5028535, US 5075078, WO 95/16207. US 5654162 oder EP 0810436A1 beschrieben). Ein solcher Festphasentest als ELISA (siehe Abb. 3a/b) umfaßt z.B. folgende Schritte: Streptavidin-beschichtete Mikrotiterplatten (Pierce) werden mit biotinylierten monoklonalen Antikörpern (z.B. 5µg/ml Endkonzentration) beschichtet. Bei den monoklonalen Antikörpern handelt es sich z.B. um GAD65 Autoantikörper MICA 1-10 und IA2 spezifischen Autoantikörper, wie z.B. in der oben genannten Literatur. Für die Beschichtung der streptavidinbeschichtete Mikrotiterplatten werden diese beiden Antikörper einzeln, oder in Kombination verwendet. Der Antikörperbeschichtung schließt sich die Blockierung der Mikrotiter Platte mit z.B. 3% BSA an. Die genannten Fusionsproteine werden mit einer Konzentration von z.B. 1µg/ml an die monoklonalen Antikörper gebunden. Im Anschluß wird die so vorbereitete Mikrotiter Platte mit Aliquots von Patientenseren (z.B. 1:100 verdünnt) inkubiert. Nach diesem Inkubationsschritt gibt man entweder AP-konjugierte oder POD-konjugierte Antikörper (z.B. Dianova, 1:10000 verdünnt) zu der Mikrotiter Platte. Diese konjugierten Antikörper erkennen z.B. den Fc Abschnitt von humanen Antikörpern. Als Substrat für die Alkaline Phosphatase (AP) dient p-Nitrophenyl-Phosphat (pNPP; Sigma) und für die Peroxidase (POD) dient als Substrat ABTS (Sigma). Ausgewertet wird der ELISA mit einem ELISA Platten-Lesegerät. Die 2. Phase des ELISA wird so durchgeführt, daß die zwei Autoantigenbestandteile separat an die monoklonalen Mausantikörper gebunden werden.

In einer Ausführungsform des erfindungsgemäßen Verfahrens erfolgt der Nachweis der Bindung des Autoantikörpers über ein markierbares Protein, das an den Autoantikörper binden kann.
Das Protein kann eine der oben beschriebenen Markierungen aufweisen. Solche Markierungen umfassen z.B. eine Biotinylierung, einen fluoreszierende, chemilumineszierenden oder biolumineszierenden Stoff, ein Kolloidalmetal, ein Enzym, eine radioaktive Markierung und/oder einen Protein-taq.
Vorzugsweise wird das markierte Protein, das das erfindungsgemäße Fusionsprotein bindet, für den Einsatz in dem erfindungsgemäßen Verfahren biotinyliert. Die biotinylierten Komponenten werden dann z.B. über die hochspezifische Biotin-Streptavidin-Interaktion an eine Streptavidin-beschichtete feste Phase gebunden.

In einer Ausführungsform ist das markierbare Protein in dem erfindungsgemäßen Verfahren ein zweiter Antikörper, z.B. ein Antikörper, der die konstante Region eines Antikörpers (Fc) erkennt.
Der Begriff "Antikörper" im Sinne der vorliegenden Erfindung betrifft monoklonale, polyklonale oder synthetische Antikörper, genauso wie Fragmente dieser Antikörper, wie z.B. Fab, Fv oder scFv-Fragmente. Diese Antikörper können z.B. für Immunpräzipitationen, Proteinmarkierungen oder Proteinimmobilisationen verwendet werden.

In einer Ausführungsform umfaßt das erfindungsgemäße Verfahren eine Probe und/oder Autoantikörper, die menschlichen Ursprungs sind.

In einer weiteren Ausführungsform des erfindungsgemäßen Verfahrens erfolgt die Bindung des Fusionsproteins mit dem Autoantikörper in einer Flüssigphase. Flüssigphasentest umfassen z.B. Radioimmunoassays und andere Schnelltestverfahren, z.B. Flow-Through-, Lateral Flow-Immunoassays usw., wie oben beschrieben. Ein Radioimmunoassay (siehe Abb. 4a/b) umfaßt z.B. folgende Schritte: Polynucleotide, die für das erfindungsgemäße Fusionsprotein codieren, werden in RNA transkribiert und danach in das Protein translatiert. Für diese in vitro Transkription und Translation kann z.B. ein SP6-gekoppeltes Retriculozytenlysat-System (Promega) verwendet werden, mit dem es möglich ist, das genannte Fusionsprotein [³⁵S]-Methionin (40µCi, 1000 Ci/mmol; Amersham) zu markieren. Proteingebundene Radioaktivität wird über eine Sephadex G25 Säule gelfiltriert, um freie Radioaktivität davon zu trennen. Über Nacht werden Serenaliquots in Microtiter Rundbodenplatten mit radioaktiv markiertem Fusionsprotein bei 4°C inkubiert. Gebildete Immunkomplexe werden mit Protein-A-Sepharose (Pharmacia) gefällt. Ungebundenes radioaktiv markiertes Protein wird von den Immunkomplexen durch Waschen in Membran-Mikrotiterplatten (Millipore, z.B. 0,6 µm Porengröße) getrennt. Die getrockneten Membranen, an denen die Immunkomplexe gebunden sind, werden in Scintillationsröhrchen überführt. Im Flüssig-Scintillationszähler wird die Menge an Radioaktivität bestimmt. Die Phase 2 des Radioimmunoassays wird genau so durchgeführt, wie die gerade beschriebene Phase 1 mit dem Unterschied, daß in der 2. Phase die radioaktiv markierten Autoantigenbestandteile getrennt für sich eingesetzt werden.

In einer Ausführungsform betrifft daher die vorliegende Erfindung ein Verfahren, wobei die entstehenden Immunkomplexe getrennt, z.B. gefällt und quantifiziert werden.

In einer weiteren Ausführungsform umfaßt das erfindungsgemäße Verfahren ferner
(d) Bestimmung des Titers des anti-GAD65- und anti-IA2-Autoantikörpers in der Probe.

In einer Ausführungsform umfaßt das erfindungsgemäße Verfahren zur Titerbestimmung des Autoantikörpers die Schritte des erfindungsgemäßen Verfahrens mit einem Polypeptid..1, umfassend ein oder mehr Epitope der GAD65 und mit einem Polypeptid 2, umfassend ein oder mehr Epitope des IA2, wobei diese Schritte getrennt wiederholt werden.
Diese Schritte umfassen z.B. die Phasen 2 eines der oben beschriebenen ELISA oder Radioimmunoassays.

Besonders bevorzugt ist das erfindungsgemäße Verfahren, in dem die genannten Schritte mit allen Epitopen wiederholt werden, damit ein möglichst breites Spektrum an Autoantikörpern detektiert und gegebenenfalls quantifiziert werden kann.

Das erfindungsgemäße Verfahren kann z.B. ein Immuno- oder ein Radioimmunoassay sein.
Dem Fachmann sind solche Assays bekannt. Geeignete Immunoassays sind z.B. Enzym-gebundener Immunoabsorptionsassay (ELISA), ein Enzymimmunodotassay, ein lmmunobead-Assay, ein passiver Hämagglutininations-Assay (PHA), ein Peptid-Antikörper-Peptid Sandwich Assay usw. oder wie oben beschrieben.

In einer weiteren bevorzugten Ausführungsform umfaßt das erfindungsgemäße Verfahren einen Nachweis von unspezifischen Bindungen, die von in der biologischen Probe vorhandenen Antikörpern verursacht werden.
Im Falle eines Immuntests, z.B. eines ELISA können zusätzlich vorhandene Antikörper z.B. antigenunabhängig bzw. unspezifisch an die Oberfläche der Vertiefungen einer Mikrotiterplatte binden und ein falsch-positives Signal bewirken.
Falsch-positive Ergebnisse aufgrund unspezifischer Bindung an die Matrix werden durch individuelle Blankwerte sicher erkannt und eliminiert. Dies ist wichtig für den Einsatz im Screening von Blutbanken, um unnötiges Entfernen von Donorblut aufgrund falsch-positiver Ergebnisse zu vermeiden.

In einer weiteren Ausführungsform betrifft die vorliegende Erfindung eine diagnostische Zusammensetzung, die das erfindungsgemäße Fusionsprotein, das erfindungsgemäße Polynucleotid oder das erfindungsgemäße Plasmid umfaßt.
Diese Zusammensetzung kann zur Diagnose oder Prognose von Autoimmunkrankheiten, z.B. Diabetes mellitus Typ I, Stiff Man-Syndrom oder Polyglanduläres Autoimmunsyndrom verwendet werden.

Die Erfindung betrifft weiterhin ein Kit, das das erfindungsgemäße Fusionsprotein, das erfindungsgemäße Polynucleotid oder das erfindungsgemäße Plasmid umfaßt.
Das erfindungsgemäße Kit kann weitere Bestandteile enthalten, die, z.B., für den Nachweis des erfindungsgemäßen Fusionsproteins sinnvoll sind. Weiterhin kann das Kit Puffer und Substrate beinhalten und wird vorteilhafterweise zur Durchführung des erfindungsgemäßen Verfahrens z.B. im diagnostischen oder wissenschaftlichen Bereich verwendet. Teile des Kits können individuell in Behältern oder in einer Kombination von Behältern abgepackt werden. Die Hersteller von Kits folgen vorzugsweise Standardverfahren, die dem Fachmann bekannt sind. Das Kit kann zudem Anleitung und Beschreibung zur Durchführung des erfindungsgemäßen Verfahrens umfassen.

Der erfindungsgemäße Kit kann zum Nachweis von Autoantikörpern und zur Diagnose von Diabetes mellitus Typ I von Säugern vorzugsweise in Körperflüssigkeiten wie, z.B. Blut, Serum, Gewebeextrakten, Gewebeflüssigkeiten, aber auch *in vitro* Zellkulturüberständen und Zellysaten als Testreagentien in einem der genannten Tests verwendet werden. Hierfür kommt jeder geeignete Radio- oder Immunoassay in Frage, insbesondere ein Enzym-gebundener Immunoabsorptionsassay (ELISA), ein Enzymimmunodotassay, ein Immunobead-Assay, ein passiver Hämagglutininations-Assay (PHA), ein Peptid-Antikörper-Peptid Sandwich Assay oder andere dem Fachmann bekannte Methoden.

In einer bevorzugten Ausführungsform umfaßt das Kit daher ein an einen Träger gebundenes erfindungsgemäßes Fusionsprotein.

Die in der vorliegenden Beschreibung zitierten Literaturstellen, inklusive der Beschreibungen und Anleitungen von Herstellern, sind hiermit Teil der Beschreibung, ohne jedoch diese Dokumente als Stand der Technik zu definieren.

Die Abbildungen zeigen:

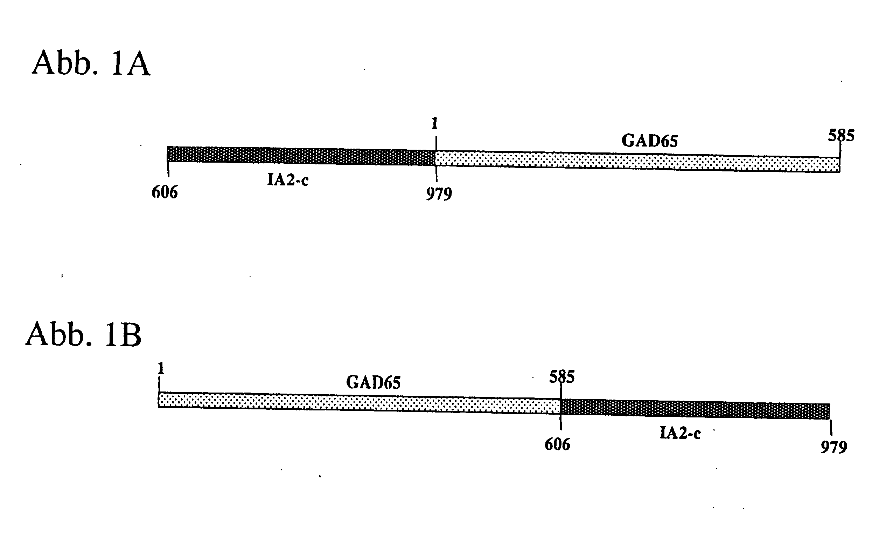

Abb. 1A und 1 B zeigen die cDNA Konstrukte für die Fusionsproteine bezogen auf diese Erfindung.
Abb. 2 zeigt die Aminosäuresequenz vom Autoantigen GAD65 (Datenbank-Code Q05329) (SEQ ID NR:1) (A) und die Aminosäuresequenz vom Autoantigen lA2 (Datenbank-Code Q16849) (SEQ ID NR:2) (B).
Abb.3A und 3B zeigen kurz die Versuchssequenzen des beschriebenen Immunoassays. A: Phase 1, B: Phase 2
Abb. 4A und 4B zeigen kurz die Versuchssequenzen des genannten Radioimmunassays. A: Phase 1, B: Phase 2

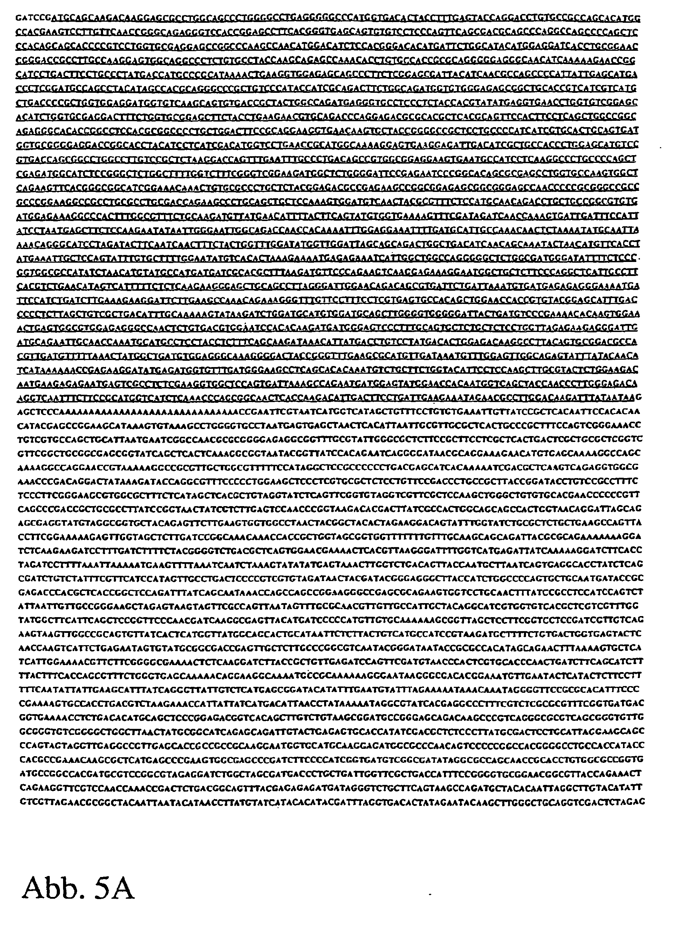

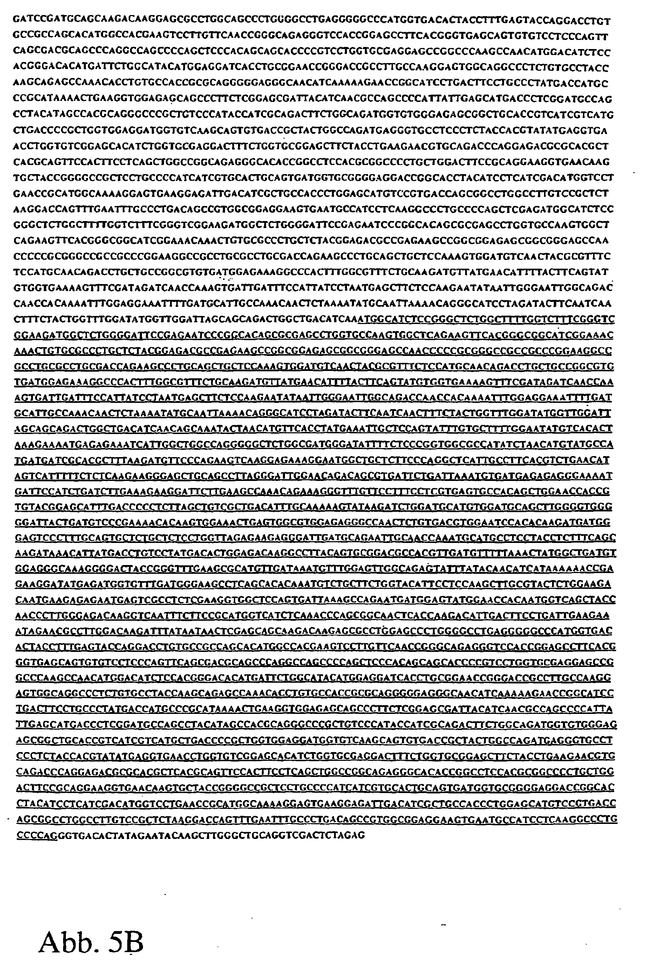

Abb. 5A und 5B zeigen die Nucleotidsequenzen der genannten Plasmidvektoren, mit einklonierter cDNA der Fusionsproteine. A: Nucleotidsequenz des Plasmidvektors pRSET-B. Unterstrichen ist die Nucleotidsequenz, die für die genannte cDNA des Fusionsproteins MR7 (Abb. 1A) codiert. (SEQ ID NR:41) B: Nucleotidsequenz des genannten Plasmidvektors pSP64poly A. Unterstrichen ist die Nucleotidsequenz, die für die zweite Version (Abb. 1B) der genannten cDNA des Fusionsproteins (GAD65₍₁₋₅₈₅₎/IA2c₍₆₀₆₋₉₇₉₎ [MR3]) codiert. (SEQ ID NR:42)

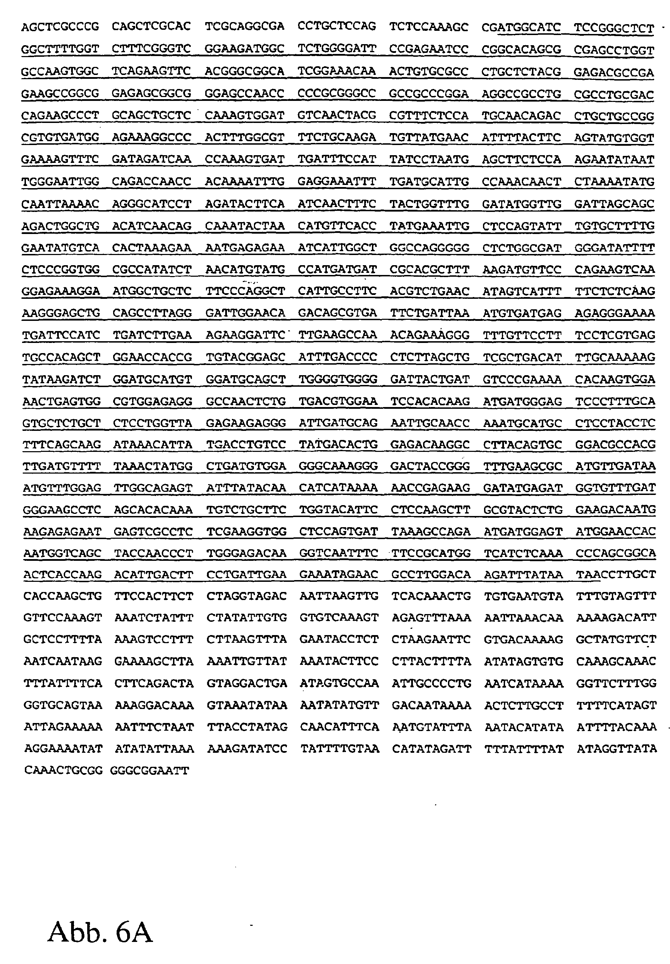

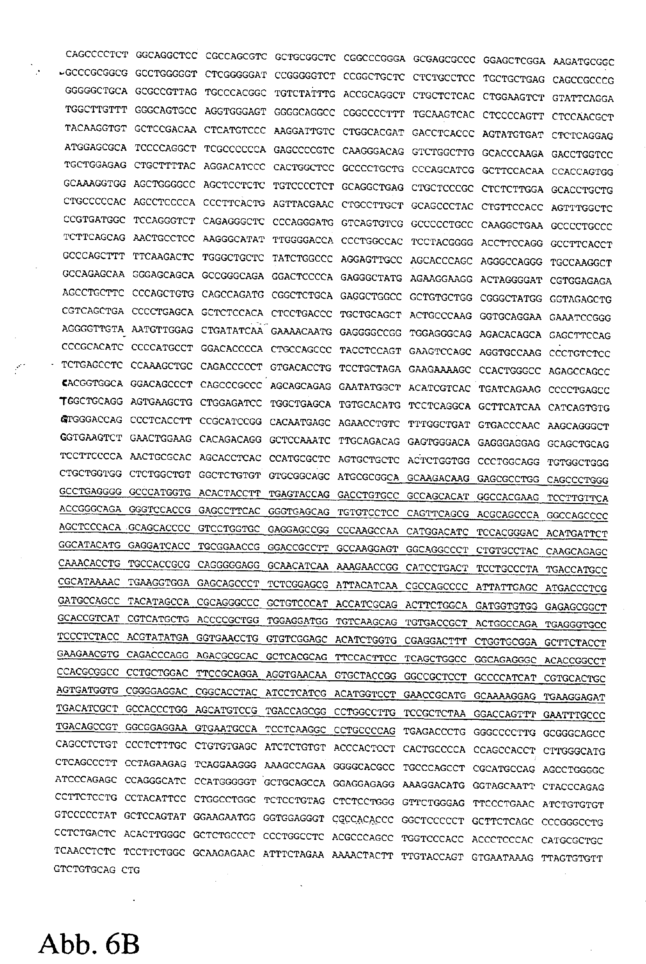

Abb. 6A und 6B zeigen die Nukleotidsequenzen der beiden einzelnen Autoantigenbestandteile GAD65 und IA2. A: Nucleotidsequenz von GAD65 (Datenbank Code M81882). Unterstrichen sind die Nucleotide, die im Fusionsprotein enthalten sind. (SEQ ID NR:3) B: Nucloetidsequenz von IA2 (Datenbank Code L18983). Unterstrichen sind die Nucleotide, die im Fusionsprotein enthalten sind. (SEQ ID NR:4)
Abb. 7A und 7B zeigt die Epitoperkennung der humanen monoklonalen Antikörper MICA 1-10 in GAD65 sowie Epitopstudien der Fusionsproteine im Vergleich mit GAD65. A: Übersicht über die Epitoperkennung von GAD65 durch die humanen monoklonalen GAD Antikörper MICA1-10. B: Epitopstudie mit 9 verschiedenen humanen monoklonalen Antikörpern, MICA 1-10, die verschiedene Epitope von GAD65 erkennen. Der Vergleich des Fusionsproteins IA2₍₆₀₆₋₉₇₉₎/GAD65₍₁₋₅₈₅₎ [MR7] bzw. GAD65₍₁₋₅₈₅₎/IA2₍₆₀₆₋₉₇₉₎ [MR3] mit GAD 65 zeigt, daß nur das Fusionsprotein IA2C₍₆₀₆₋₉₇₉₎/GAD65₍₁₋₅₈₅₎ [MR7] mit allen MICA reagiert, während GAD65₍₁₋₅₈₅₎/IA2c₍₆₀₆₋₉₇₉₎ [MR3] von MICA 7 gar nicht und MICA 2 sehr schlecht erkannt wird. Der direkte Vergleich zwischen diesem Fusionsprotein lA2c₍₆₀₆₋₉₇₉₎/GAD65₍₁₋₅₈₅₎ [MR7] und GAD65 zeigt, daß keine signifikanten Epitop-Veränderungen im besagten Fusionsprotein vorliegen. Die für die verschiedenen GAD65-Epitope spezifischen MICA reagieren sehr ähnlich. Für die Überprüfung der korrekten Faltung des IA2-Bestandteils im Fusionsprotein stand ein Patientenserum und ein monoklonaler Mausantikörper zur Verfügung. Für beide zeigt das besagte Fusionsprotein lA2c₍₆₀₆₋₉₇₉₎/GAD65₍₁₋₅₈₅₎ [MR7] eine hohe Reaktivität.

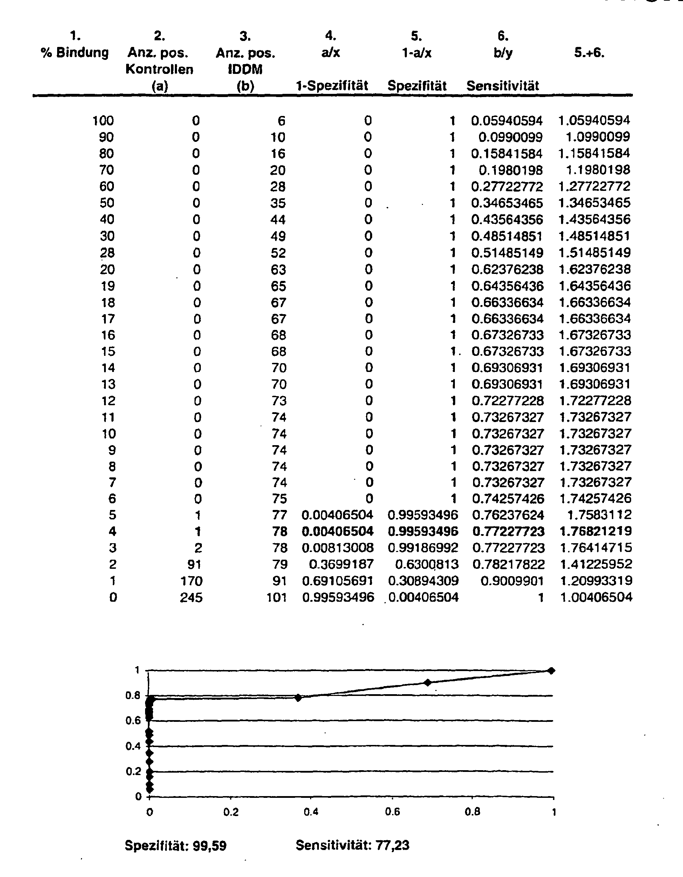

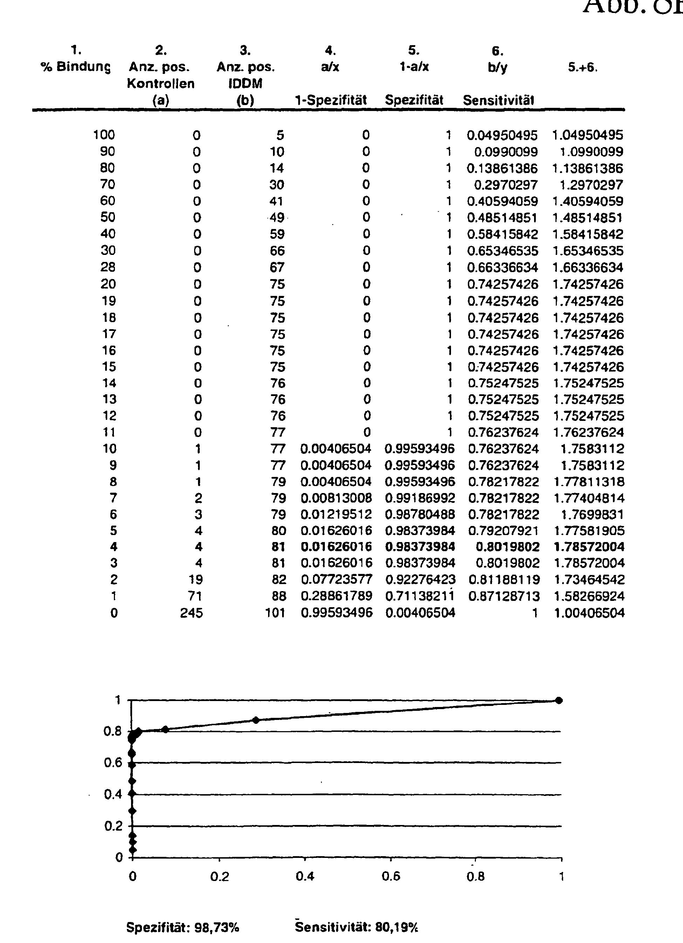

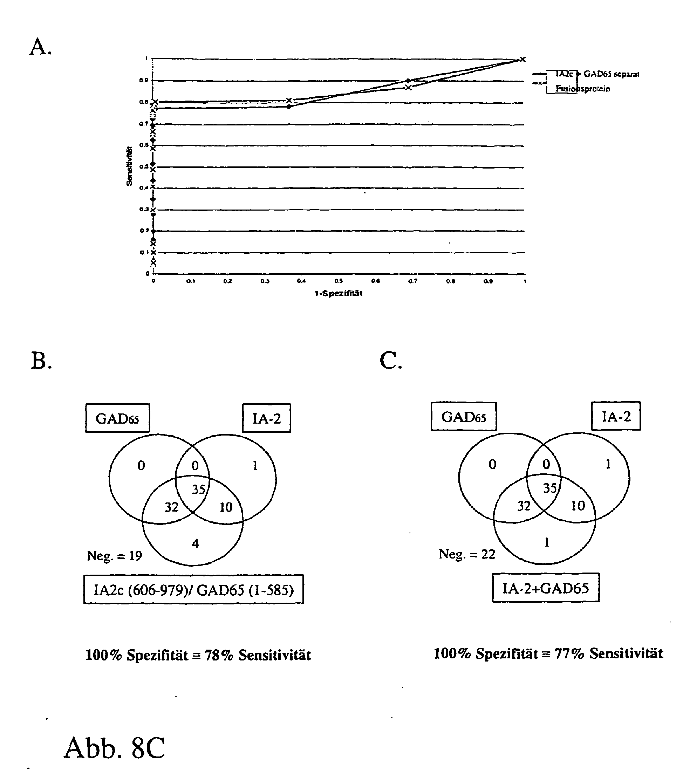

Abb. 8A, B und C zeigen die Ergebnisse einer klinischen Prüfung mit dem Fusionsprotein und mit den einzelnen Autoantigenen IA2 und GAD65. A: Ergebnis der ROC-Plot-Analyse für den RIA zur kombinierten Detektion von GAD65- und IA2-Autoantikörpem basierend auf der Verwendung der separaten Antigene GAD65 und IA2. Hervorgehoben ist der Cut-off ermittelt bei 4% Antikörperbindung. B: Ergebnis der ROC-Plot-Analyse für den RIA zur kombinierten Detektion von GAD65- und IA2-Autoantikörpem basierend auf der Verwendung des Fusionsproteins IA2₍₆₀₆₋₉₇₉₎/ GAD65₍₁₋₅₈₅₎ Hervorgehoben der Cut-off ermittelt bei 4% Antikörperbindung. C: ROC-Plot-Analysenvergleich. Verglichen werden die ROC-Plot-Analysen zwischen dem Testsystem mit dem Fusionsprotein IA2₍₆₀₆₋₉₇₉₎/ GAD65₍₁₋₅₈₅₎ und dem Testsystem IA2 + GAD65 separat. (a) zeigt die graphische Darstellung des ROC-Plotanalysenvergleichs; (b) und (c) zeigen die Antikörperreaktivität von 101 Diabetes Typ 1 Patientenseren, die im RIA ermittelt wurde. In (b) ist das Ergebnis für den RIA basierend auf dem Fusionsprotein IA2₍₆₀₆₋₉₇₉₎/ GAD65₍₁₋₅₈₅₎ gezeigt, in (c) ist das Ergebnis für den RIA abgebildet, bei dem die Antigene GAD65 und IA2 separat eingesetzt wurden.

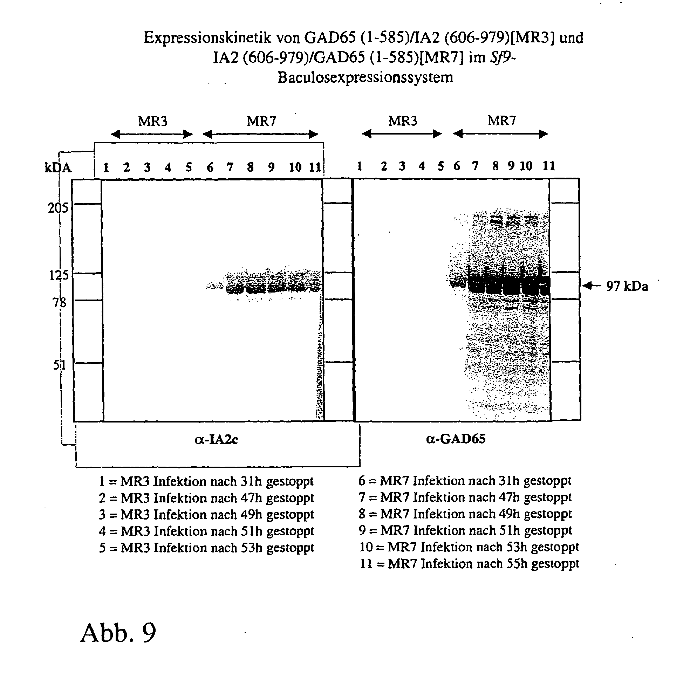

Abb. 9 zeigt einen Westernblot zur Expressionskinetik von GAD65₍₁₋₅₈₅₎/IA2₍₆₀₆₋₉₇₉₎ [MR3] und IA2_{(606-979)/}GAD65₍₁₋₅₈₅₎ [MR7] im Sf9-Baculoexpressionssystem. Man erkennt, daß nur das Fusionsprotein MR8 -in Sf9-Zellen exprimiert wird. Die Expression wurde von 31h bis 55h nach Infektion konstant nachgewiesen. Auf das SDS-PAGE Gel wurde ungereinigtes Zell-Lysat aufgetragen. Auf dem linken Westernblot wurde der 1A2c Anteil, auf dem rechten Westernblot der GAD65 Anteil der Fusionsproteine detektiert. Zusätzliche Banden lassen auf einen schwachen Abbau des Fusionsproteins schließen.

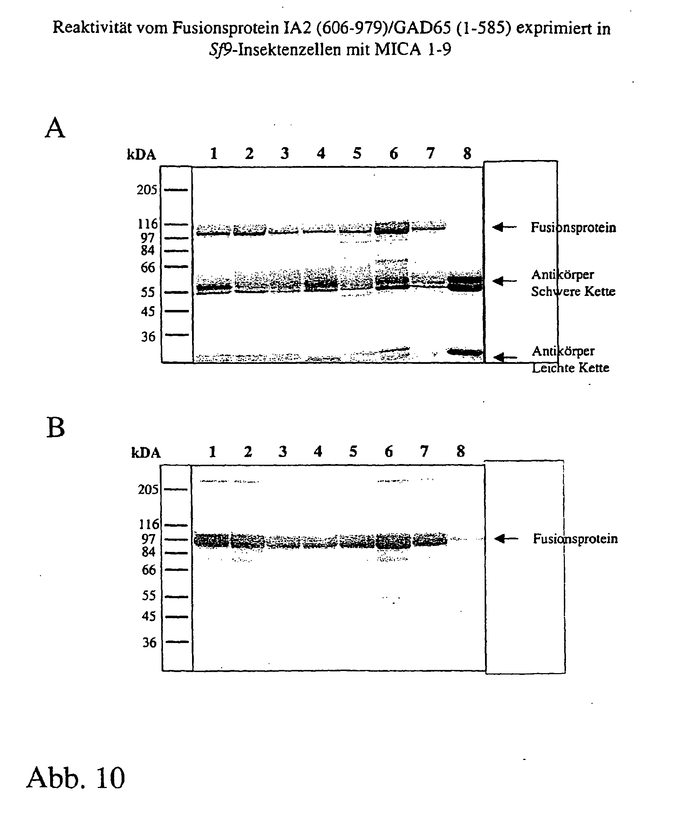

Abb. 10 zeigt die Reaktivität vom Fusionsprotein IA2₍₆₀₆₋₉₇₉₎/GAD65₍₁₋₅₈₅₎ exprimiert in *Sf*9-Insektenzellen mit MICA 1-9. (A) 8% SDS-PAGE Gel das mit Coomassie-Blau gefärbt ist. Das Fusionsprotein wurde aus Sf9-Insektenzell-Lysat mit MICA 1-9 und Protein-A-Sepharose immungefällt. Alle MICA erkennen das Fusionsprotein. Die schwache Präzipitation durch MICA 9 ist sehr wahrscheinlich auf eine schlechte Antikörpercharge zurückzuführen. (B) Westernblot (vom Gel in A), in dem der GAD65-Anteil im Fusionsprotein mit einem α-GAD65 spez. polyklonalen Kaninchenserum detektiert wurde. Zusätzliche Banden lassen auf einen schwachen Abbau des Fusionsproteins während der Immunfällung schließen. MICA 1 (Spur 1); MICA 2 (Spur 2); MICA 3 (Spur 3); MICA 4 (Spur 4); MICA 6 (Spur 5); MICA 7 (Spur 6); MICA 8 (Spur 7); MICA 9 (Spur 9).

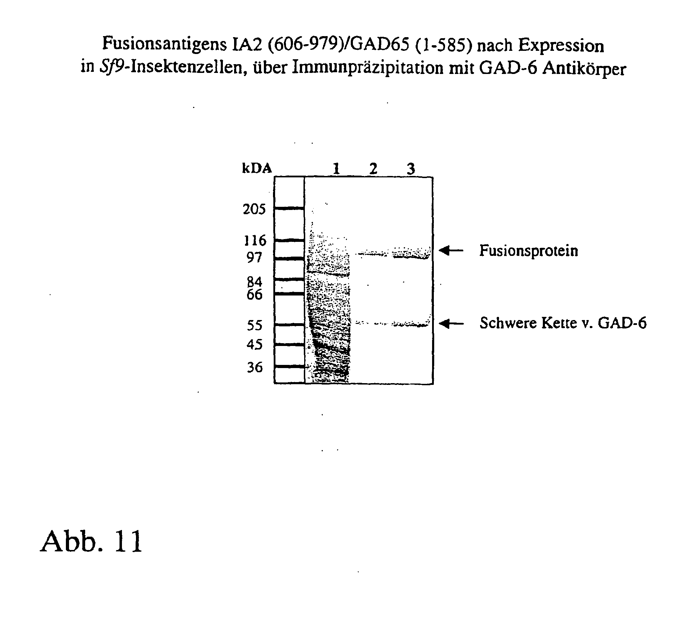

Abb. 11 zeigt die Reinigung des Fusionsantigens IA2₍₆₀₆₋₉₇₉)/GAD65₍₁₋₅₈₅₎ nach Expression in S*f9*-Insektenzellen, über Immunpräzipition mit GAD-6 Antikörper. 8% SDS-PAGE Gel das mit Coomassie gefärbt ist. In kleinem Maßstab wurde das Fusionsprotein aus Sf9-Insektenzellen-Lysat mittels GAD-6 Protein-A-Immunfällung in einem Schritt mit hohem Reinheitsgrad (>95%) aufgereinigt. Proteinkontrolle (Spur 1); zwei verschiedene Aufreinigungsansätze (Spur 2-3).

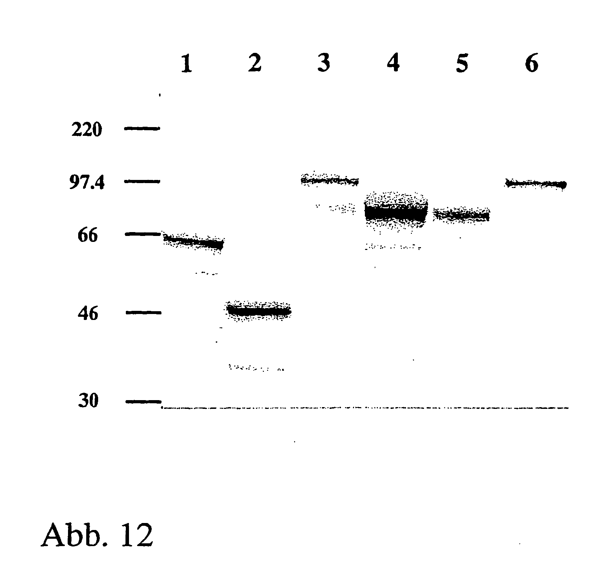

Abb. 12: Errechnetes Molekulargewicht für IA2₍₆₀₆₋₉₇₉₎/GAD65₍₁₋₅₈₅₎ [MR7] und GAD65₍₁₋₅₈₅₎/lA2c₍₆₀₆₋₉₇₉₎ [MR3] ist 108 kDa. Beide Fusionsproteine laufen im 8%-SDS-PAGE Gel auf einer Höhe von 97 kDa. Errechnetes Molekulargewicht für GAD65₍₂₃₄₋₅₈₅₎/IA2c₍₆₀₆₋₉₇₉₎ und IA2₍₆₀₆₋₉₇₉₎/GAD65₍₂₄₄₋₅₈₅₎ ist 72 kDa. Beide Fusionsproteine laufen im 8%-SDS-PAGE Gel auf einer Höhe von 74 kDa.
Abb. 13 zeigt die Epitopkonservierung in GAD65/IA2 Fusionsproteinen. GAD65 (A), IA2 (B) und vier GAD65/IA2 Fusionsproteine (C-F) wurden durch in vitro Translation und Transkription in einem Retikulozyten-Lysatsystem in der Gegenwart von 35-S Methionin exprimiert. Die GAD65-spezifischen Diabetes assoziierten humanen monoklonalen GAD65-Antikörper MIKA 1, 2, 3, 4, 6, 7, 8, 9 und 10, der IA2 reagierende mausmonoklonale Antikörper 76F4B und ein lA2-Antikörper positives (ab⁺)/GAD65-Antikörper negatives (ab⁻) Patientenserum wurden auf ihre Reaktivität mit den Konstrukten in Radioimmunassays getestet. Die mit MlKA3 für GAD65 oder mit IA2-Antikörper positiven(ab⁺)/GAD65-Antikörper negativen (ab⁻) Patientenserum für IA2 gemessene Bindung wurde als 100% gesetzt und hierauf die relative Bindung der anderen Antikörper bezogen. Die Balken repräsentieren den Mittelwert von zwei Messungen.

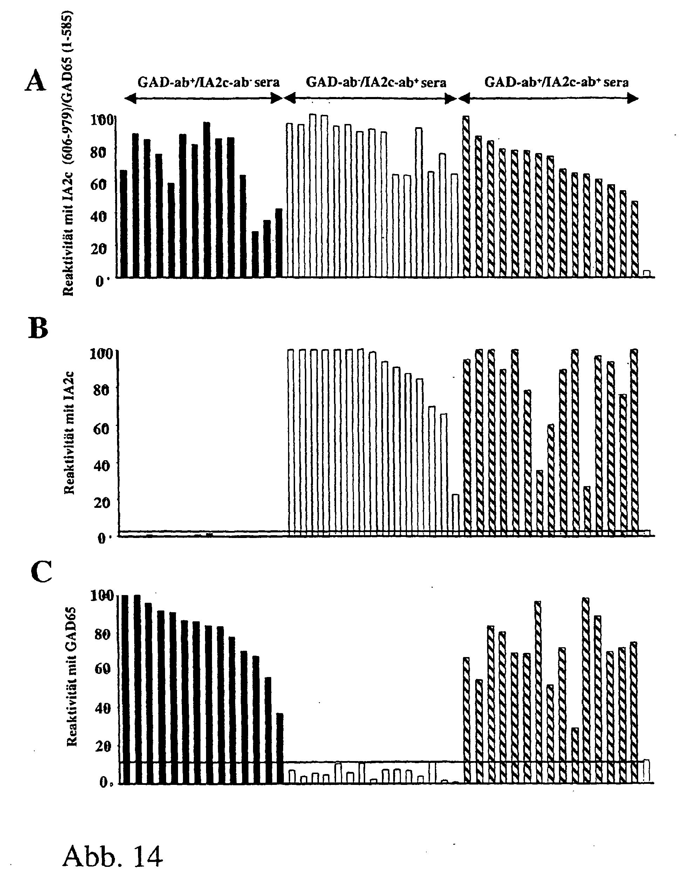

Abb. 14: Reaktivität von GAD65-ab⁺ und/oder IA2-ab⁺ Diabetes Typ 1 Patientenseren mit dem Fusionsprotein IA2₍₆₀₆₋₉₇₉₎/GAD65₍₁₋₅₆₅₎, GAD65 und IA2. Die Seren wurden im Radioimmunoassay analysiert, in dem [35S] Methionin markiertes Fusionsprotein (A), IA2₍₆₀₆₋₉₇₉₎ (B) oder Wildtyp GAD65₍₁₋₅₈₅₎ eingesetzt wurden. Die 14 GAD65-ab⁺/IA2-ab⁻ Seren sind als schwarze Balken, die 15 IA2-ab⁺/GA065-ab⁻ Seren sind als weiße Balken und die 15 GAD65-ab⁺/IA2-ab⁺ Seren sind als gestrichelte Balken abgebildet. Die Ergebnisse werden als Prozentsatz der gemessenen Impulse (cpm) angegeben, die mit dem unverdünnten kombinierten Standard (A), mit dem IA2 Referenzserum (B) und mit dem MICA 3 Standard (C) erzielt wurden. Die Balken stellen einen Durchschnittswert aus einer Doppelmessung dar. Horizontale Linien markieren den Grenzwert des GAD65 und IA2-Assays.

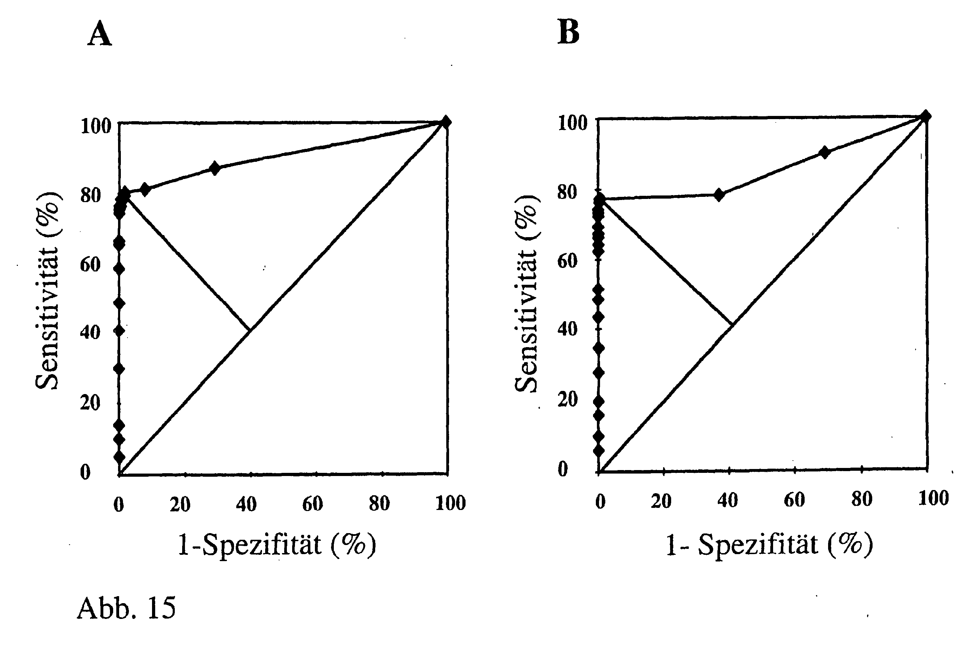

Abb. 15: Receiver-operating characteristic (ROC)-Analyse. Abgebildet ist die ROC-Plot-Analyse des Radioimmunoassays basierend auf dem Fusionsprotein IA2₍₆₀₆₋₉₇₉₎/GAD65₍₁₋₅₈₅₎ (A) und des kombinierten Radioimmunoassays basierend auf dem Assay, welches die beiden Antigene GAD65 und IA2c separat einsetzt (B). Die Sensitivität wurde aus 101 Diabetes Typ 1 Patientenseren, und die Spezifität unabhängig davon aus 245 gesunden Individuen errechnet. Der optimale Schwellenwert für den jeweiligen Test wird dargestellt, als das Paar aus Sensitivität und Spezifität, welches am weitesten entfernt von der Diagonale von links unten nach rechts oben liegt. Für das Radioimmunoassay mit dem Fusionsprotein liegt dieser Schwellenwert bei 4% Antikörperbindung, was einer Sensitivität von 80% und einer Spezifität von 98% entspricht. Für das kombinierte Assay mit den separaten Antigenen (B) liegt dieser Schwellenwert ebenfalls bei 4% Antikörperbindung, was einer Sensitivität von 77% und einer Spezifität von 100% entspricht.

Die vorliegende Erfindung wird durch die folgenden Beispiele beschrieben, ohne dadurch eingeschränkt zu werden.

### Beispiel 1:

### Expression der Fusionsproteine in Sf9-Insektenzellen

Für die Entwicklung von nicht radioaktiven GAD65/IA2 Autoantikörper Testsystemen sollte das neue Fusionsprotein in eukaryontischen Zellen exprimiert werden. Das Baculoexpressionssystem basierend auf infizierten *Sf9-*Zellen wurde ausgewählt. Dafür wurden die beiden Fusionsprotein-Varianten GAD65₍₁₋₅₈₅₎/IA2₍₆₀₆₋₉₇₉₎ [MR3] und IA2₍₆₀₆₋₉₇₉₎/GAD65₍₁₋₅₈₅₎ [MR7] in den Plasmidvektor pVL1393 umkloniert. Mit diesem Vektor ist es möglich rekombinante Baculoviren für die Infektion von Sf9-Zellen, wie vorher beschrieben (Richardson, 1995; Summers, 1988), herzustellen. Im Plaque-Assay werden Einzelklone selektiert, um sicher zu stellen, daß mit funktionstüchtigen Viren gearbeitet wird. Ein auf diesem Weg hergestelltes Baculovirus-Fusionsprotein IA2 ₍₆₀₆₋₉₇₉₎/GAD65₍₁₋₅₈₅₎ wurde am 25. Februar 2000 unter der Nummer 00022237 bei der European Collection of Cell Cultures, Centre of Applied Microbiology & Research, Salisbury, Wiltshire SP4 OJG, UK hinterlegt.
Zunächst wurde getestet, ob die Fusionsproteine in Sf9-Zellen exprimiert werden. Es wurde eine Expressionskinetik zum Vergleich der beiden Fusionsproteine GAD65₍₁₋₅₈₅₎/IA2c₍₆₀₆₋₉₇₉₎ [MR3] und IA2c₍₆₀₆₋₉₇₉₎/GAD65₍₁₋₅₈₅₎ [MR7] aufgenommen. Zusätzlich wurde untersucht, ob die Proteinbestandteile in ihre native Konformation gefaltet werden. Die Ergebnisse zeigen, daß nur das Fusionsprotein IA2c₍₆₀₆₋₉₇₉₎/GAD65₍₁₋₅₈₅₎ [MR7] in Sf9-Zellen stabil in seiner korrekten Konformation exprimiert wurde

### Beispiel 2:

### Expressionskinetik von GAD65₍₁₋₅₈₅₎/IA2c₍₆₀₆₋₉₇₉₎ [MR3] und IA2c₍₆₀₆₋₉₇₉₎/GAD65₍₁₋₅₈₅₎ [MR7]

Für jeden Kontrollansatz wurden 10 Mio. Sf9-Zellen in einer 80 cm²-Kulturflasche (NUNC) ausgesät. Mit einer MOI = 3 wurde 1h auf einem Horizontalschüttler (Heidolph) bei RT infiziert. Nach der Infektion wurde die Virensuspension abgenommen und durch 15 ml Grace's Insektenmedium (Invitrogen) ersetzt. Die Inkubation der Zellen erfolgte bei 27°C im Zellinkubator für 31-55h nach Infektion. Für den Aufschluß wurden je 10 Mio. Zellen in 1 ml PBS-Puffer und Proteaselnhibitor-Mix (Boehringer Mannheim) aufgenommen. Mit Ultraschall (BRANSON Sonifier 250) wurden die Zellen auf Eis lysiert. Das Lysat wurde 30 min bei 100.000 g zentrifugiert. 16 µl Überstand wurden für die folgende SDS-PAGE Analyse auf ein 8% Polyacrylamidgel aufgetragen. Je ein Gel wurde mit Coomassie-Farbstoff gefärbt und ein zweites Gel für eine Westernblot-Analyse verwendet. Der Proteinübertrag erfolgte auf eine PVDF-Membran (Immobilon, Millipore). Für die Expressionskinetik wurde auf einer Membran der GAD65-Anteil im Fusionsprotein mit einem α-GAD65 spezifischen polyklonalen Kaninchenserum und auf einer zweiten Membran der IA2 Anteil mit einem α-IA2 spezifischen polyklonalem Kaninchenserum detektiert. Gefärbt wurde jeweils mit einem Alkaline-Phosphatase konjugierten Antikörper (Ziege α-Kaninchen IgG, Fc Fragment spezifisch, Dianova). Die Ergebnisse sind in Fig. 9 gezeigt. Abb. 9 zeigt eine Expressionskinetik der beiden Fusionsprotein-Varianten GAD65₍₁₋₅₈₅₎/IA2c₍₆₀₆₋₉₇₉₎ [MR3] IA2c₍₆₀₆₋₉₇₉₎/GAD65₍₁₋₅₈₅₎ [MR7] in Sf9-Insektzellen. Man erkennt, daß nur das Fusionsprotein IA2c₍₆₀₆₋₉₇₉₎/GAD65₍₁₋₅₈₅₎ [MR7] in Sf9-Zellen exprimiert wird. Die Expression wurde von 31h bis 55h nach Infektion nachgewiesen. Auf das SDS-PAGE Gel wurde ungereinigtes Zell-Lysat aufgetragen. Auf dem linken Westernblot wurde der IA2c Anteil, auf dem rechten Westernblot der GAD65 Anteil der Fusionsproteine detektiert. Zusätzliche Banden lassen auf einen schwachen Abbau des Fusionsproteins schließen. 1 = GAD65₍₁₋₅₈₅₎/IA2c₍₆₀₆₋₉₇₉₎ [MR3] Infektion nach 31h gestoppt, 2 = GAD65₍₁₋₅₈₅₎/IA2C₍₆₀₆₋₉₇₉₎ [MR3] Infektion nach 47h gestoppt, 3 = GAD65₍₁₋₅₈₅₎/IA2c₍₆₀₆₋₉₇₉₎ [MR3] Infektion nach 49h gestoppt, 4 = GAD65₍₁₋₅₈₅₎/IA2c₍₆₀₆₋₉₇₉₎ [MR3] Infektion nach 51h gestoppt, 5 = GAD65₍₁₋₅₈₅₎/IA2c₍₆₀₆₋₉₇₉₎ [MR3] Infektion nach 53h gestoppt, 6 = und IA2c₍₆₀₆₋₉₇₉₎/GAD65₍₁₋₅₈₅₎ [MR7] Infektion nach 31h gestoppt, 7 = und IA2c₍₆₀₆₋₉₇₉₎/GAD65₍₁₋₅₈₅₎ [MR7] Infektion nach 47h gestoppt, 8 = und IA2c₍₆₀₆₋₉₇₉₎/GAD65₍₁₋₅₈₅₎ [MR7] Infektion nach 49h gestoppt, 9 = und IA2c₍₆₀₆₋₉₇₉₎/GAD65₍₁₋₅₈₅₎ [MR7] Infektion nach 51 h gestoppt, 10 = und IA2_{C(606-979)/}GAD65₍₁₋₅₈₅₎ [MR7] Infektion nach 53h gestoppt, 11 = und IA2c₍₆₀₆₋₉₇₉₎/GAD65₍₁₋₅₈₅₎ [MR7] Infektion nach 55h gestoppt

Im Zeitraum von 31-53h nach Infektion wurde kein GAD65₍₁₋₅₈₅₎/IA2_{C(606-979})-Fusionsprotein [MR3] exprimiert. Die Expression des MR7-Fusionsprotein startete dagegen bei 31h nach Infektion und blieb über einen Zeitraum von 55h auf hohem Niveau konstant. Bei nachfolgenden Versuchen die Expression des GAD65(_{1- 585)}IA2c(₆₀₆₋₉₇₉₎-Fusionsproteins [MR3] zu optimieren, stellte sich heraus, daß dieses Protein nicht in voller Länge exprimiert wurde. Die Expression war zudem instabil und die unvollständig exprimierten Proteinfragmente wurden schnell in Sf9-Zellen abgebaut (Ergebnisse nicht abgebildet). Da alle Plasmid Konstrukte durch Sequenzierung auf korrekte Leserahmen hin überprüft worden sind, konnte dieser Fehler ausgeschlossen werden.

### Beispiel 3:

### Prüfung der Faltung von Proteinuntereinheiten im Fusionsprotein IA2c₍₆₀₆₋₉₇₉₎/GAD65₍₁₋₅₈₅₎

Dafür wurde das IA2c₍₆₀₆₋₉₇₉)/ GAD65₍₁₋₅₈₅₎ -Fusionsprotein [MR7] in Sf9-Zellen exprimiert und auf die Reaktivität mit humanen Diabetes Typ 1 assoziierten monoklonalen Inselzellantikörpern MICA 1-9 hin getestet. Die Ergebnisse dieser acht Immunpräzipitationen (Fig. 10) zeigte, daß alle MICA das Fusionsantigen erkannten. Die schwache Präzipitation durch MICA 9 ist sehr wahrscheinlich auf eine schlechte Antikörpercharge zurückzuführen, da MICA 9 nach Affinitätsaufreinigung oft keine Reaktivität mehr zeigt.
Die Produktion des Fusionsproteins in *Sf*9-Zellen und dessen Aufbereitung war analog zum ersten Versuch.
Jeweils 1 ml Lysat Überstand wird nach der Ultrazentrifugation, 30 min bei 100.000 g, mit 1-2 µg Affinitäts-gereinigten MICA bei 4°C ÜN auf einem Drehschüttler (Heidolph) inkubiert. Die Immunkomplexe werden dann mit 30 µl gequollener Protein-A-Sepharose (Pharmacia) 1 h bei 4°C auf einem Drehschüttler präzipitiert. Das Präzipitat wird in 2 ml Eppendorf Gefäßen 3x mit Schwyzer Puffer (100 mM Tris-HCl pH = 9; 500 mM LiCl; 1 % Triton X-100; 1 % 2-Mercaptoethanol), 2x mit PBS Puffer und 2x mit H₂O bidest gewaschen. Danach wird das Protein-A-Sepharose-Pellet in 40 µl Probenpuffer (10% 2-Mercaptoethanol; 6% SDS; 20% Glycerol; Bromphenol-Blau; 300 mM Tris-HCl pH = 6,8) aufgenommen und 6 min bei 97 °C gekocht. Je 20 µl des so aufbereiteten Präzipitats werden für die folgende SDS-PAGE-Analyse auf ein 8% Polyacrylamid-Gel aufgetragen. Die anschließende Coomassie-Färbung (Fig. 10A) und Westernblot Analyse (Fig. 10B) wurde analog zu Beispiel 1 durchgeführt.

### Beispiel 4:

### Reinigung von Sf9-exprimiertem MR1-Fusionsprotein

Das Fusionsprotein IA2₍₆₀₆₋₉₇₉₎/GAD65₍₁₋₅₈₅₎ wurde wie oben beschrieben in *Sf9-*lnsektenzellen produziert und mit dem mausmonoklonalen Antikörper GAD-6 (Developmental Studies Hybridoma Bank, lowa) aus dem Zell-Lysat mittels Immunpräzipitation aufgereinigt. Wie die Coomassie-Färbung zeigt, konnte das Fusionsprotein aus dem Zell-Lysat mit einem geschätzten Reinheitsgrad von >95% gereinigt werden (Fig. 11). Über dieses Prinzip soll daher das Fusionsprotein in größerem Maßstab gereinigt werden. Der GAD-6 Antikörper wird dafür kovalent an CH4B- oder CL4B-Sepharose (Pharmacia) gekoppelt und ist somit bis zu 10 mal wiederverwendbar. Auf diesem Weg können größere Mengen Protein für nichtradioaktive Tests zur Verfügung gestellt werden.

### Beispiel 5:

### Konstruktion der Expressionsplasmide

Für die Konstruktion der cDNA, die für das genannte Fusionsprotein (Abb. 1a) codiert, werden zwei PCR-Fragmente hergestellt. Das eine PCR-Fragment umfaßt den IA2-Proteinanteil von Aminosäure 606-979. Am N-Terminus des genannten PCR-Fragments wird eine Ncol- und an den C-Terminus ein Xhol-Schnittstelle angehängt. Das andere PCR-Fragment umfaßt den GAD65-Proteinanteil von Aminosäure 1 bis 585. An den N-Terminus dieses GAD65-PCR-Fragments wird eine Xhol- und an den C-Terminus wird eine Sacl-Schnittstelle angehängt.
Kloniert werden die PCR-Fragmente in den Vektor pSP64(A)n, in dem schon der IA2-Proteinanteil von Aminosäure 606-979 einkloniert ist. Der genannte Vektor wird mit den Restriktionsenzymen Ncol und Sacl verdaut.
Die genannten IA2- und GAD65-PCR-Fragmente werden mit den aufgeführten Restriktionsenzymen verdaut und aufgereinigt.
In einem Ligationsansatz werden die beiden genannten PCR-Fragmente und das genannte Vektorfragment miteinander ligiert.
Für die Konstruktion der cDNA, die für das genannte Fusionsprotein (Abb. 1 b) codiert, werden zwei PCR-Fragmente hergestellt. Das eine PCR-Fragment umfaßt den IA2-Proteinanteil von Aminosäure 606-979. Am N-Terminus des genannten PCR Fragments wird eine Notl- und an den C-Terminus eine EcoRI-Schnittstelle angehängt. Das andere PCR-Fragment umfaßt den GAD65-Proteinanteil von Aminosäure 1 bis 585. An den N-Terminus dieses GAD65 PCR-Fragments wird eine Stul- und an den C-Terminus wird eine Notl-Schnittstelle angehängt.
Kloniert werden die PCR Fragmente in den Vektor pVL1393, in dem schon der GAD65-Proteinanteil von Aminosäure 1-585 einkloniert ist. Der genannte Vektor wird mit den Restriktionsenzymen Stul und EcoRl verdaut.
Die genannten IA2- und GAD65-PCR Fragmente werden mit den aufgeführten Restriktionsenzymen verdaut und aufgereinigt.
In einem Ligationsansatz werden die beiden genannten PCR-Fragmente und das genannte Vektorfragment miteinander ligiert.
Für die In vitro Transcription/Translation wird die cDNA der des beschriebenen Fusionsproteins über die Restriktionsenzyme BamHl und Ncol in den Vektor pSP64(A)n umkloniert, in dem schon der IA2-Proteinanteil von Aminosäure 606-979 einkloniert ist.
Mit dem Vektor pSP64(A)n sollen die genannten Fusionsprotein mittels in vitro Transkription und Translation [³⁵S]-Methionin markiert werden. So radioaktiv markiert, können die Fusionsproteine im genannten Radioimmunoassay eingesetzt werden (Petersen, 1994).

### Beispiel 6:

### Expression der Fusionsproteine

Für die Produktion der genannten Fusionsproteine in Sf9 Zellen werden die aufgeführten cDNAs, die für die Fusionsproteine codieren, in den Vektor pVL1393 umkloniert. Rekombinante Sf9-Viruspartikel werden nach Standardmethoden produziert (Richardson 1995).
Virus produzierende Sf9-Zellen werden kultiviert und Virenstocks werden angelegt. Im großen Maßstab werden sowohl die Einzelproteinbestandteile, als auch die Fusionsproteine produziert und aufgereinigt.
Für die Produktion der genannten Fusionsproteine in E. coli (Stamm BL21, Stratagene) werden die genannten cDNAs in den Vektor pRSET umkloniert. Für die Proteinproduktion werden die *E*. *coli* Zellen mit IPTG induziert. Ein E.coli Stamm, der die Sequenz für das Fusionsprotein IA2₍₆₀₆₋₉₇₉₎/GAD65₍₁₋₅₈₅₎ in diesem Expressionsvektor enthält, wurde am 23. Februar 2000 unter der Nummer DSM 13334 bei der Deutschen Sammlung von Mikroorganismen und Zellkulturen GmbH, Mascheroder Weg 1 b, D-38124 Braunschweig hinterlegt.
Proteinexpressionskontrollen werden mit SDS-PAGE und Westernblot durchgeführt. Für die Kontrolle der korrekten Immunreaktivität werden lmmunfällungen mit verschiedenen monoklonalen Antikörpern spezifisch für beide Proteinanteile im Fusionsprotein angesetzt.
Die Aufreinigung der Fusionsproteine aus Zellextrakten geschieht über CH4B-oder CL4B-Sepharose-Säulen mit immobilisiertem monoklonalen GAD6-Antikörper (Chang et al., 1988). Die Elution des Proteins von der CH4B-Sepharose-Säule geschieht bei pH11. Das Eluat wird nach dem Elutionsvorgang sofort auf pH7 eingestellt. Die Reinigung über ein CL4B-Sepharose-Säule umfaßt folgende Schritte: Der zur Reinigung vorbereitete Zellkulturüberstand von MAK GAD6 wird unter Hochsalz Bedingungen an eine Protein A Säule (Hi Trap Protein A, Amersham Pharmacia 5ml) gebunden. Der IgG-Antikörper bindet mit dem Fc-Teil an Protein A. Mit Disuccinimidyl oder Bis(Salfosuccinimidyl) wird die Bindung GAD6-Protein A stabilisiert. Anschließend wird die Säule mit den Puffern zur Proteinaufreinigung equilibriert. Der Auftrag des vorbereiteten Zelllysat erfolgt über Nacht. Mit geeigneten Elutionsbedingungen wird das gebundene Protein von der Säule getrennt.

### Beispiel 7:

### Epitopkonservierung in den Fusionsproteinen

Für die Überprüfung der korrekten Proteinfaltung innerhalb der Fusionsprotein werden diese in vitro transkribiert und dabei [³⁵S]-Methionin radioaktiv markiert. Es werden neun GAD65 spezifische Diabetes Typ 1 assoziierte humane monoklonale Antikörper (MICA 1-10), ein IA2 spezifischer Maus monoklonaler Antikörper und ein IA2-ab⁺/GAD65-ab⁻ Patientenserum auf ihre Reaktivität mit den Fusionsproteinen im Rahmen eines Radioimmunoassays hin getestet. Es konnte gezeigt werden, daß im Fusionsprotein GAD65(₁₋₅₈₅)/IA2₍₆₀₆₋₉₇₉₎ [MR1] die Reaktivitäten für die zwei Antikörper MICA 2 und MICA 7 verloren gehen. Praktisch ihre komplette Reaktivität verlieren alle getesteten MICA Antikörper in den Konstrukten MR4 und MR6, in denen der GAD65 Proteinanteil verkürzt vorliegt. Mit der Fusionsprotein-Variante IA2₍₆₀₆₋₉₇₉₎/GAD65₍₁₋₅₈₅₎ [MR7] können die zwei Reaktivitäten von MICA 2 und MICA 7, die im GAD65₍₁₋₅₈₅₎/IA2c₍₆₀₆₋₉₇₉₎-Konstrukt [MR1] verloren gegangen waren, wieder hergestellt werden.

In einem zweiten Versuchsabschnitt wurde die lmmunreaktivität von GAD65-ab⁺ und/oder IA2-ab⁺ polyklonalen Seren von Patienten mit Diabetes Typ 1 zum Fusionsprotein IA2₍₆₀₆₋₉₇₉₎/GAD65₍₁₋₅₈₅₎ mit der Reaktivität der separaten GAD65 und IA2 Proteine verglichen. Es wurden drei Seren-Gruppen ausgesucht und im Radioimmunoassay analysiert. Alle 14 GAD65-ab⁺/IA2-ab⁻ Seren, alle 15 GAD65-ab⁻/IA2-ab⁺ Seren und alle 15 GAD65-ab⁺/IA2-ab⁺ Seren zeigten positive Ergebnisse mit dem Fusionsprotein IA2c₍₆₀₆₋₉₇₉₎/GAD65₍₁₋₅₈₅₎.[MR7] (s. Abb. 14 und 15)

### Literaturverzeichnis:

Borg et al., Clin Chem 43 (1997); 2358-63
Bosi et al., J Pediatr Endocrinol Metab 11 (1998); 293-305
Christie et al., Diabetes Care 20 (1997); 965-70
Dittler et al., Diabetes 47 (1998); 592-7
Ellis et al., Diabetes, 48 (1999); 299-303
Harrop et al., Diabetes Res Clin Pract 18 (1992); 107-12
Krueger et al., EMBO J 9 (1990); 3241-52
Lampasona et al., J Immunol 15 (1996); 2707-11
Leslie et al., Diabetologia 42 (1999); 3-14
Papouchado et al., J Immunol Methods 24 (1997); 169-78
Petersen et al., Diabetes 43 (1994); 459-67
Pietropaolo et al., J Autoimmun 11 (1998); 1-10
Richardson, C. D., ed. (1995). Baculovirus Expression Protocolls. In Methods in Molecular Biology, Vol. 39 (J.M. Walker, ed.) Humana Press, Totowa, N. J.
Sabbah et al., J Clin Endocrinol Metab 81 (1996); 2455-9
Schranz et al., A Diabetes Metab Rev 14 (1998); 3-29
Schwartz et al., J Mol Biol 287 (1999); 983-99
Semana et al., J Autoimmun 12 (1999); 259-67
Snorgaard et al., Diabetes Care 19 (1996); 146-50
Söhnlein et al., Diabetologia 43 (2000); 210-7
Syren et al., J lmmunol 157 (1996); 5208-14
Summers et al., Texas Agricultural Expreriment Station Bulletin No. 1555.
Verge et al., Diabetes 45 (1996); 926-33
Wiest-Ladenburger et al., Diabetes 46 (1997); 565-71
Xie et al., J Immunol 159 (1997); 3662-7

## Patentansprüche

1. Fusionsprotein, das am N-Terminus ein oder mehr. Epitope umfasst, die einen Autoantikörper gegen das Inselzellantigen IA2 spezifisch binden, und am C-Terminus Epitope umfasst, die einen Antikörper gegen die Glutamatdecarboxylase GAD65 spezifisch binden, wobei
(i) das Fusionsprotein spezifisch an die nachfolgend unter (a), (b), (c) und (d) definierten Antikörper bindet:
(a) MICA 2 (SEQ ID NR: 9 und 11) oder MICA 7 (SBQ ID NR: 29 und 31);
(b) MICA 4 (SEQ ID NR: 17 und 19), oder MICA 6 (SEQ ID NR: 25 und 27);
(c) MICA 1 (SEQ ID NR: 5 und 7) oder MICA 3 (SEQ ID NR: 13 und 15); und
(d) MICA 9 (SEQ 11) NP- 33 und 35); wobei
(ii) Epitope des Inselzellantigens IA2 Epitope aus der Aminosäuresequenz der Aminosäuren 606 bis 979 von SEQ ID NO: 2 oder einer Allelvariation davon sind und Epitope der Glutamatdecarboxylase GAD65 Epitope aus der Aminosäuresequenz der Aminosäuren 1 bis 585 von SEQ ID NO: 1 oder einer Allelvariation davon sind.

2. Fusionsprotein nach Anspruch 1, wobei das Fusionsprotein die monoklonalen Antikörper MICA 1, 2, 3, 4, 6, 7 und 9 spezifisch bindet.

3. Fusionsprotein nach einem der Ansprüche 1 oder 2, in dem ein oder mehr Epitope der GAD65 und ein oder mehr Epitope des IA2 ohne Verbindungspeptid miteinander verbunden sind.

4. Fusionsprotein nach einem der Ansprüche 1 bis 3, wobei das Fusionsprotein an eine Festphase gekoppelt ist

5. Fusionsprotein nach einem der Ansprüche 1 bis 4, wobei das Fusionsprotein markiert ist.

6. Polynucleotid, welches das Fusionsprotein nach einem der Ansprüche 1 bis 5 codiert

7. Polynucleotid nach Anspruch 6, das DNA oder RNA ist

8. Vektor, der das Polynucleotid nach Anspruch 6 oder 7 umfasst.

9. Wirtszelle, die mit dem Polynucleotid nach Anspruch 6 oder 7 oder dem Vektor nach Anspruch 8 transformiert oder transfiziert ist

10. Wirtszelle nach Anspruch 9, die eine eukaryotische Wirtszelle ist.

11. Wirtszelle nach Anspruch 10, die eine CHO-Zelle ist.

12. Wirtszelle nach Anspruch 9, die eine prokaryotische Wirtszelle ist

13. Wirtszelle nach Anspruch 12, die *E*. *coli* ist

14. Verfahren zur Herstellung eines Fusionsproteins nach einem der Ansprüche 1 bis 5, umfassend:
(a) Züchten der Wirtszelle nach einem der Ansprüche 12 oder 13; oder
(b) in vitro Transkription und/oder Translation des Polynucleotids nach Anspruch 6 oder 7; und
(c) Isolierung des Fusionsproteins.

15. Verfahren zur Herstellung von Antikörpern, umfassend die Immunisierung eines nichtmenschlichen Tieres mit dem Fusionsprotein nach einem der Ansprüche 1 bis 5.

16. Verfahren zum Nachweis von Autoantikörpern, umfassend
(a) Inkontaktbringen einer biologischen Probe mit dem Fusionsprotein nach einem der Ansprüche 1 bis 5, unter Bedingungen, die die Bindung von Antikörpern erlauben; und
(b) Nachweis der in Schritt (a) gebundenen Antikörper.

17. Verfahren nach Anspruch 16, ferner umfassend
(c) Quantifizierung der Antikörperbindung.

18. Verehren nach Anspruch 16 oder 17, wobei das Fusionsprotein vor oder nach der Inkubation mit der Probe an eine Festphase gekoppelt wird.

19. Verfahren nach einem der Ansprüche 16 bis 18, wobei der Nachweis der Bindung des Autoantikörpers über ein markierbares Protein erfolgt, das an den Autoantikörper binden kann.

20. Verfahren nach Anspruch 19, wobei das markierbare Protein ein zweiter Antikörper ist.

21. Verfahren nach Anspruch 20, wobei der zweite Antikörper spezifisch die konstante Region eines Antikörpers (Fc) erkennt.

22. Verfahren nach einem der Ansprüche 16 bis 21, wobei die Probe und/oder die Autoantikörper menschlichen Ursprungs sind.

23. Verfahren nach einem der Ansprüche 16 bis 22, wobei die Bindung des Fusionsproteins mit dem Autoantikörper in einer Flussigphase erfolgt.

24. Verfahren nach Anspruch 23, wobei die entstehenden Immunkomplexe getrennt und quantifiziert werden.

25. Verfahren nach einem der Anspruche 16 bis 24, ferner umfassend
(d) Bestimmung des Titers des anti-CAD65- und anti-IA2-Atitoantikörpers in der Probe.

26. Verfahren nach Anspruch 25, wobei zur Titerbestimmung des Autoantikörpers die Schritte des Verfahrens nach einem der Ansprüche 16 bis 24 mit einem Polypeptid 1, umfassend ein oder mehr Epitope der GAD65 und mit einem Polypeptid 2, umfassend ein oder mehr Epitope des IA2, getrennt wiederholt wird.

27. Verfahren nach Anspruch 26, wobei die Schritte mit allen Epitopen wiederholt werden.

28. Verfahren nach einem der Ansprüche 16 bis 27, das ein Immuno- oder ein Radioimmunoassay ist.

29. Verfahren nach einem der Ansprüche 16 bis 28, umfassend den Nachweis von unspezifischen Bindungen, die von in der biologischen Probe vorhandenen Antikörpern verursacht werden.

30. Verwendung des Fusionsproteins nach einem der Ansprüche 1 bis 5, des Polynucleotids nach Anspruch 6 oder 7, des Vektors nach Anspruch 8 oder der Wirtszelle nach einem der Ansprüche 9 bis 13 für die Herstellung einer Zusammensetzung zur Diagnose oder Prognose von Diabetes mellitus Typ I, Stiff-Man-Syndrom, Polyglanduläres Autoimmunsyndrom und anderen Autoimmunkrankheiten mit Autoantikörper gegen GAD65 oder IA2.

31. Fusionsprotein nach einem der Ansprüche 1 bis 5, Polynucleotid nach Anspruch 6 oder 7, Vektor nach Anspruch 8 oder Wirtszelle nach einem der Ansprüche 9 bis 13 zur Diagnose oder Prognose von Diabetes mellitus Typ I, Stiff-Man-Syndrom, Polyglanduläres Autoimmunsyndrom und anderen Autoimmunkrankheiten mit Autoantikörper gegen GAD65 oder IA2.

32. Diagnostische Zusammensetzung, die das Fusionsprotein nach einem der Ansprüche 1 bis 5, das Polynucleotid nach Anspruch 6 oder 7 oder den Vektor nach Anspruch 8 umfasst.

33. Kit, das das Fusionsprotein nach einem der Ansprüche 1 bis 5, das Polynucleotid nach Anspruch 6 oder 7 oder den Vektor nach Anspruch 8 umfasst.

34. Kit nach Anspruch 33, wobei das Fusionsprotein an einen Träger gebunden ist.

## Claims

1. A fusion protein comprising one or more epitopes at the N-terminus which specifically bind an autoantibody against islet cell antigen IA2 and comprising epitopes at the C-terminus which specifically bind an antibody against glutamate decarboxylase GAD65, wherein
(i) the fusion protein specifically binds to the antibodies defined hereinafter under (a), (b), (c) and (d):
(a) MICA 2 (SEQ ID NO:9 and 11) or MICA 7 (SEQ ID NO:29 and 31);
(b) MICA 4 (SEQ ID N0:17 and 19) or MICA 6 (SEQ ID NO:25 and 27);
(c) MICA 1 (SEQ ID NO:5 and 7) or MICA 3 (SEQ ID N0:13 and 15); and
(d) MICA 9 (SEQ ID NO:33 and 35); wherein
(ii) epitopes of the islet cell antigen IA2 are epitopes from the amino acid sequence of amino acids 606 to 979 of SEQ ID NO:2 or an allele variation thereof and epitopes of the glutamate decarboxylase GAD65 are epitopes from the amino acid sequence of amino acids 1 to 585 of SEQ ID NO:1 or an allele variation thereof.

2. The fusion protein of claim 1, wherein the fusion protein specifically binds the monoclonal antibodies MICA 1, 2, 3, 4, 6, 7 and 9.

3. The fusion protein of any one of claim 1 or 2, in which one or more epitopes of GAD65 and one or more epitopes of IA2 are linked to each other without a linker peptide.

4. The fusion protein of any one of claims 1 to 3, wherein the fusion protein is linked to a solid phase.

5. The fusion protein of any one of claims 1 to 4, wherein the fusion protein is labeled.

6. A polynucleotide which encodes the fusion protein of any one of claims 1 to 5.

7. The polynucleotide of claim 6 which is DNA or RNA.

8. A vector comprising the polynucleotide of claim 6 or 7.

9. A host cell which is transformed or transfected with the polynucleotide of claim 6 or 7 or the vector of claim 8.

10. The host cell of claim 9 which is a eukaryotic host cell.

11. The host cell of claim 10 which is a CHO cell.

12. The host cell of claim 9 which is a prokaryotic host cell.

13. The host cell of claim 12 which is *E. coli.*

14. A method for the production of a fusion protein of any one of claims 1 to 5, comprising:
(a) culturing the host cell of any one of claims 12 or 13; or
(b) in vitro transcription and/or translation of the polynucleotide of claim 6 or 7; and
(c) isolating the fusion protein.

15. A method for the production of antibodies, comprising the immunisation of a non-human animal with the fusion protein of any one of claims 1 to 5.

16. A method for the detection of autoantibodies, comprising
(a) contacting a biological sample with the fusion protein of any one of claims 1 to 5 under conditions allowing the binding of antibodies; and
(b) detecting the antibodies bound in step (a).

17. The method of claim 16, further comprising
(c) quantifying the binding of antibodies.

18. The method of any one of claim 16 or 17, wherein the fusion protein is linked to a solid phase prior or subsequent to incubation with the sample.

19. The method of any one of claims 16 to 18, wherein the binding of the autoantibody is detected using a protein which can be labeled and which is capable of binding to the autoantibody.

20. The method of claim 19, wherein the protein which can be labeled is a second antibody.

21. The method of claim 20, wherein the second antibody specifically recognizes the constant region of an antibody (Fc).

22. The method of any one of claims 16 to 21, wherein the sample and/or the autoantibodies are/is of human origin.

23. The method of any one of claims 16 to 22, wherein the binding of the fusion protein to the autoantibody takes place in a fluid phase.

24. The method of claim 23, wherein the resulting immune complexes are separated and quantified.

25. The method of any one of claims 16 to 24, further comprising
(d) determining the titer of the anti-GAD65 autoantibody and the anti-lA2 autoantibody in the sample.

26. The method of claim 25, wherein, for the determination of the autoantibody titer, the steps of the method of any one of claims 16 to 24 with a polypeptide 1 comprising one or more epitopes of GAD65 and with a polypeptide 2 comprising one or more epitopes of lA2 are repeated separately.

27. The method of claim 26, wherein the steps are repeated with all epitopes.

28. The method of any one of claims 16 to 27, which is an immunoassay or a radioimmunoassay.

29. The method of any one of claims 16 to 28, comprising the detection of unspecific binding caused by antibodies which are present in the biological sample.

30. Use of the fusion protein of any one of claims 1 to 5, the polynucleotide of claim 6 or 7, the vector of claim 8 or the host cell of any one of claims 9 to 13 for the preparation of a composition for the diagnosis or prognosis of diabetes mellitus type I, stiff-man syndrome, polyglandular autoimmune syndrome and other autoimmune diseases with an autoantibody against GAD65 or IA2.

31. Fusion protein of any one of claims 1 to 5, the polynucleotide of claim 6 or 7, the vector of claim 8 or the host cell of any one of claims 9 to 13 for the diagnosis or prognosis of diabetes mellitus type I, stiff-man syndrome, polyglandular autoimmune syndrome and other autoimmune diseases with an autoantibody against GAD65 or IA2.

32. Diagnostic composition comprising the fusion protein of any one of claims 1 to 5, the polynucleotide of claim 6 or 7 or the vector of claim 8.

33. Kit comprising the fusion protein of any one of claims 1 to 5, the polynucleotide of claim 6 or 7 or the vector of claim 8.

34. The kit of claim 33, wherein the fusion protein is bound to a carrier.

## Revendications

1. Protéine de fusion, qui comprend en N-Terminal un ou plusieurs épitopes qui lient spécifiquement un autoanticorps contre l'antigène îlot IA2, et en C-Terminal des épitopes qui lient spécifiquement un anticorps contre la glutamate décarboxylase GAD65, où
(i) la protéine de fusion se lie spécifiquement aux anticorps définis dans ce qui suit au (a), (b), (c) et (d) :
(a) MICA 2 (SEQ ID N° 9 et 11) ou MICA 7 (SEQ ID N° 29 et 31) ;
(b) MICA 4 (SEQ ID N° 17 et 19) ou MICA 6 (SEQ ID N° 25 et 27) ;
(c) MICA 1 (SEQ ID N° 5 et 7) ou MICA 3 (SEQ ID N° 13 et 15) ; et
(d) MICA 9 (SEQ ID N°33 et 35) ; où
(ii) les épitopes de l'antigène îlot IA2 sont des épitopes de la séquence d'acides aminés 606 à 979 de la SEQ ID N°2 ou d'une variation allélique de celle-ci et les épitopes de la glutamate décarboxylase GAD65 sont des épitopes de la séquence d'acides aminés 1 à 585 de la SEQ ID N°1 ou d'une variation allélique de celle-ci.

2. Protéine de fusion selon la revendication 1, où la protéine de fusion lie spécifiquement les anticorps monoclonaux MICA 1, 2, 3, 4, 6, 7 et 9.

3. Protéine de fusion selon l'une des revendications 1 ou 2, dans laquelle un ou plusieurs épitopes de GAD65 ou un ou plusieurs épitopes de IA2 sont liés ensemble sans peptide de liaison.

4. Protéine de fusion selon l'une des revendications 1 à 3, où la protéine de fusion est couplée à une phase solide.

5. Protéine de fusion selon l'une des revendications 1 à 4, où la protéine de fusion est marquée.

6. Polynucléotide, qui code la protéine de fusion selon l'une des revendications 1 à 5.

7. Polynucléotide selon la revendication 6, qui est de l'ADN ou de l'ARN.

8. Vecteur, qui comprend le polynucléotide selon la revendication 6 ou 7.

9. Cellule hôte, qui est transformée ou transfectée avec le polynucléotide selon la revendication 6 ou 7 ou le vecteur selon la revendication 8.

10. Cellule hôte selon la revendication 9, qui est une cellule hôte eucaryote.

11. Cellule hôte selon la revendication 10, qui est une cellule CHO.

12. Cellule hôte selon la revendication 9, qui est une cellule hôte procaryote.

13. Cellule hôte selon la revendication 12, qui est *E*. *coli.*

14. Procédé de fabrication d'une protéine de fusion selon l'une des revendications 1 à 5, comprenant :
(a) la culture de la cellule hôte selon l'une des revendications 12 ou 13 ; ou
(b) la transcription et/ou traduction *in vitro* du polynucléotide selon la revendication 6 ou 7 ; et
(c) l'isolement de la protéine de fusion.

15. Procédé de fabrication d'anticorps, comprenant l'immunisation d'un animal non humain avec la protéine de fusion selon l'une des revendications 1 à 5.

16. Procédé pour la détection de l'autoanticorps, comprenant :
(a) la mise en contact d'un échantillon biologique avec la protéine de fusion selon l'une des revendications 1 à 5, dans des conditions permettant la liaison des anticorps ; et
(b) la détection des anticorps liés à l'étape (a).

17. Procédé selon la revendication 16, comprenant :
(c) la quantification de la liaison des anticorps.

18. Procédé selon la revendication 16 ou 17, où la protéine de fusion est couplée à une phase solide avant ou après l'incubation avec l'échantillon.

19. Procédé selon l'une des revendications 16 à 18, où la détection de la liaison de l'autoanticorps s'effectue par le biais d'une protéine marquable, qui peut se lier aux autoanticorps.

20. Procédé selon la revendication 19, où la protéine marquable est un deuxième anticorps.

21. Procédé selon la revendication 20, où le deuxième anticorps reconnaît spécifiquement la région constante d'un anticorps (Fc).

22. Procédé selon l'une des revendications 16 à 21, où l'échantillon et/ou les autoanticorps sont d'origine humaine.

23. Procédé selon l'une des revendications 16 à 22, où la liaison de la protéine de fusion avec l'autoanticorps s'effectue dans une phase liquide.

24. Procédé selon la revendication 23, où les complexes immuns générés sont séparés et quantifiés.

25. Procédé selon l'une des revendications 16 à 24, comprenant en outre :
(d) la détermination du titre de l'autoanticorps anti-GAD65 et anti-IA2 dans l'échantillon.

26. Procédé selon la revendication 25, où, pour déterminer le titre de l'autoanticorps, on répète séparément les étapes du procédé selon l'une des revendications 16 à 24 avec un polypeptide 1, comprenant un ou plusieurs épitopes de GAD65, et avec un polypeptide 2, comprenant un ou plusieurs épitopes de IA2.

27. Procédé selon la revendication 26, où les étapes sont répétées avec tous les épitopes.

28. Procédé selon l'une des revendications 16 à 27, qui est un immunodosage ou un dosage radioimmunologique.

29. Procédé selon l'une des revendications 16 à 28, comprenant la détection des liaisons non spécifiques, qui sont provoquées par les anticorps présents dans l'échantillon biologique.

30. Utilisation de la protéine de fusion selon l'une des revendications 1 à 5, du polynucléotide selon la revendication 6 ou 7, du vecteur selon la revendication 8 ou de la cellule hôte selon l'une des revendications 9 à 13 pour la fabrication d'une composition pour le diagnostic ou le pronostic du diabète sucré de type I, du syndrome de l'homme raide, du syndrome auto-immun polyglandulaire et d'autres maladies auto-immunes avec des autoanticorps dirigés contre GAD65 ou IA2.

31. Protéine de fusion selon l'une des revendications 1 à 5, du polynucléotide selon la revendication 6 ou 7, du vecteur selon la revendication 8 ou de la cellule hôte selon l'une des revendications 9 à 13 pour le diagnostic ou le pronostic du diabète sucré de type I, du syndrome de l'homme raide, du syndrome auto-immun polyglandulaire et d'autres maladies auto-immunes avec des autoanticorps dirigés contre GAD65 ou IA2.

32. Composition diagnostique qui comprend la protéine de fusion selon l'une des revendications 1 à 5, le polynucléotide selon la revendication 6 ou 7 ou le vecteur selon la revendication 8.

33. Kit qui comprend la protéine de fusion selon l'une des revendications 1 à 5, qui comprend le polynucléotide selon la revendication 6 ou 7 ou le vecteur selon la revendication 8.

34. Kit selon la revendication 33, où la protéine de fusion est liée à un support.


### SEQUENZPROTOKOLL

<110> Labor Dr. Koch - Dr. Merk
<120> Fusionsprotein zur gleichzeitigen Erkennung von humanen Autoantikörpern
<130> E 1207 DE
<140>
   <141>
<160> 42
<170> Patentln Ver. 2.1
<210> 1
   <211> 585
   <212> PRT
   <213> Homo sapiens
<400> 1
   Met Ala Ser Pro Gly Ser Gly Phe Trp Ser Phe Gly Ser Glu Asp Gly
    1               5                  10                  15
   Ser Gly Asp Ser Glu Asn Pro Gly Thr Ala Arg Ala Trp Cys Gln Val
               20                  25                  30
   Ala Gln Lys Phe Thr Gly Gly Ile Gly Asn Lys Leu Cys Ala Leu Leu
           35                  40                  45
   Tyr Gly Asp Ala Glu Lys Pro Ala Glu Ser Gly Gly Ser Gln Pro Pro
       50                  55                  60
   Arg Ala Ala Ala Arg Lys Ala Ala Cys Ala Cys Asp Gln Lys Pro Cys
   65                  70                  75                  80
   Ser Cys Ser Lys Val Asp Val Asn Tyr Ala Phe Leu His Ala Thr Asp
                   85                  90                  95
   Leu Leu Pro Ala Cys Asp Gly Glu Arg Pro Thr Leu Ala Phe Leu Gln
               100                 105                 110
   Asp Val Met Asn Ile Leu Leu Gln Tyr Val Val Lys Ser Phe Asp Arg
           115                 120                 125
   Ser Thr Lys Val Ile Asp Phe His Tyr Pro Asn Glu Leu Leu Gln Glu
       130                 135                 140
   Tyr Asn Trp Glu Leu Ala Asp Gln Pro Gln Asn Leu Glu Glu Ile Leu
   145                 150                 155                 160
   Met His Cys Gln Thr Thr Leu Lys Tyr Ala Ile Lys Thr Gly His Pro
                   165                 170                 175
   Arg Tyr Phe Asn Gln Leu Ser Thr Gly Leu Asp Met Val Gly Leu Ala
               180                 185                 190
   Ala Asp Trp Leu Thr Ser Thr Ala Asn Thr Asn Met Phe Thr Tyr Glu
           195                 200                 205
   Ile Ala Pro Val Phe Val Leu Leu Glu Tyr Val Thr Leu Lys Lys Met
       210                 215                 220
   Arg Glu Ile Ile Gly Trp Pro Gly Gly Ser Gly Asp Gly Ile Phe Ser
   225 230 235 240
   Pro Gly Gly Ala Ile Ser Asn Met Tyr Ala Met Met Ile Ala Arg Phe
   245 250 255
   Lys Met Phe Pro Glu Val Lys Glu Lys Gly Met Ala Ala Leu Pro Arg
   260 265 270
   Leu Ile Ala Phe Thr Ser Glu His Ser His Phe Ser Leu Lys Lys Gly
   275 280 285
   Ala Ala Ala Leu Gly Ile Gly Thr Asp Ser Val Ile Leu Ile Lys Cys
   290 295 300
   Asp Glu Arg Gly Lys Met Ile Pro Ser Asp Leu Glu Arg Arg Ile Leu
   305 310 315 320
   Glu Ala Lys Gln Lys Gly Phe Val Pro Phe Leu Val Ser Ala Thr Ala
   325 330 335
   Gly Thr Thr Val Tyr Gly Ala Phe Asp Pro Leu Leu Ala Val Ala Asp
   340 345 350
   Ile Cys Lys Lys Tyr Lys Ile Trp Met His Val Asp Ala Ala Trp Gly
   355 360 365
   Gly Gly Leu Leu Met Ser Arg Lys His Lys Trp Lys Leu Ser Gly Val
   370 375 380
   Glu Arg Ala Asn Ser Val Thr Trp Asn Pro His Lys Met Met Gly Val
   385 390 395 400
   Pro Leu Gln Cys Ser Ala Leu Leu Val Arg Glu Glu Gly Leu Met Gln
   405 410 415
   Asn Cys Asn Gln Met His Ala Ser Tyr Leu Phe Gln Gln Asp Lys His
   420 425 430
   Tyr Asp Leu Ser Tyr Asp Thr Gly Asp Lys Ala Leu Gln Cys Gly Arg
   435 440 445
   His Val Asp Val Phe Lys Leu Trp Leu Met Trp Arg Ala Lys Gly Thr
   450 455 460
   Thr Gly Phe Glu Ala His Val Asp Lys Cys Leu Glu Leu Ala Glu Tyr
   465 470 475 480
   Leu Tyr Asn Ile Ile Lys Asn Arg Glu Gly Tyr Glu Met Val Phe Asp
   485 490 495
   Gly Lys Pro Gln His Thr Asn Val Cys Phe Trp Tyr Ile Pro Pro Ser
   500 505 510
   Leu Arg Thr Leu Glu Asp Asn Glu Glu Arg Met Ser Arg Leu Ser Lys
   515 520 525
   Val Ala Pro Val Ile Lys Ala Arg Met Met Glu Tyr Gly Thr Thr Met
   530 535 540
   Val Ser Tyr Gln Pro Leu Gly Asp Lys Val Asn Phe Phe Arg Met Val
   545 550 555 560
   Ile Ser Asn Pro Ala Ala Thr His Gln Asp Ile Asp Phe Leu Ile Glu
   565 570 575
   Glu Ile Glu Arg Leu Gly Gln Asp Leu
   580 585
<210> 2
   <211> 979
   <212> PRT
   <213> Homo sapiens
<400> 2
   Met Arg Arg Pro Arg Arg Pro Gly Gly Leu Gly Gly Ser Gly Gly Leu
   1 5 10 15
   Arg Leu Leu Leu Cys Leu Leu Leu Leu Ser Ser Arg Pro Gly Gly Cys
   20 25 30
   Ser Ala Val Ser Ala His Gly Cys Leu Phe Asp Arg Arg Leu Cys Ser
   35 40 45
   His Leu Glu Val Cys Ile Gln Asp Gly Leu Phe Gly Gln Cys Gln Val
   50 55 60
   Gly Val Gly Gln Ala Arg Pro Leu Leu Gln Val Thr Ser Pro Val Leu
   65 70 75 80
   Gln Arg Leu Gln Gly Val Leu Arg Gln Leu Met Ser Gln Gly Leu Ser
   85 90 95
   Trp His Asp Asp Leu Thr Gln Tyr Val Ile Ser Gln Glu Met Glu Arg
   100 105 110
   Ile Pro Arg Leu Arg Pro Pro Glu Pro Arg Pro Arg Asp Arg Ser Gly
   115 120 125
   Leu Ala Pro Lys Arg Pro Gly Pro Ala Gly Glu Leu Leu Leu Gln Asp
   130 135 140
   Ile Pro Thr Gly Ser Ala Pro Ala Ala Gln His Arg Leu Pro Gln Pro
   145 150 155 160
   Pro Val Gly Lys Gly Gly Ala Gly Ala Ser Ser Ser Leu Ser Pro Leu
   165 170 175
   Gln Ala Glu Leu Leu Pro Pro Leu Leu Glu His Leu Leu Leu Pro Pro
   180 185 190
   Gln Pro Pro His Pro Ser Leu Ser Tyr Glu Pro Ala Leu Leu Gln Pro
   195 200 205
   Tyr Leu Phe His Gln Phe Gly Ser Arg Asp Gly Ser Arg Val Ser Glu
   210 215 220
   Gly Ser Pro Gly Met Val Ser Val Gly Pro Leu Pro Lys Ala Glu Ala
   225 230 235 240
   Pro Ala Leu Phe Ser Arg Thr Ala Ser Lys Gly Ile Phe Gly Asp His
   245 250 255
   Pro Gly His Ser Tyr Gly Asp Leu Pro Gly Pro Ser Pro Ala Gln Leu
   260 265 270
   Phe Gln Asp Ser Gly Leu Leu Tyr Leu Ala Gln Glu Leu Pro Ala Pro
   275 280 285
   Ser Arg Ala Arg Val Pro Arg Leu Pro Glu Gln Gly Ser Ser Ser Arg
   290 295 300
   Ala Glu Asp Ser Pro Glu Gly Tyr Glu Lys Glu Gly Leu Gly Asp Arg
   305 310 315 320
   Gly Glu Lys Pro Ala Ser Pro Ala Val Gln Pro Asp Ala Ala Leu Gln
   325 330 335
   Arg Leu Ala Ala Val Leu Ala Gly Tyr Gly Val Glu Leu Arg Gln Leu
   340 345 350
   Thr Pro Glu Gln Leu Ser Thr Leu Leu Thr Leu Leu Gln Leu Leu Pro
   355 360 365
   Lys Gly Ala Gly Arg Asn Pro Gly Gly Val Val Asn Val Gly Ala Asp
   370 375 380
   Ile Lys Lys Thr Met Glu Gly Pro Val Glu Gly Arg Asp Thr Ala Glu
   385 390 395 400
   Leu Pro Ala Arg Thr Ser Pro Met Pro Gly His Pro Thr Ala Ser Pro
   405 410 415
   Thr Ser Ser Glu Val Gln Gln Val Pro Ser Pro Val Ser Ser Glu Pro
   420 425 430
   Pro Lys Ala Ala Arg Pro Pro Val Thr Pro Val Leu Leu Glu Lys Lys
   435 440 445
   Ser Pro Leu Gly Gln Ser Gln Pro Thr Val Ala Gly Gln Pro Ser Ala
   450 455 460
   Arg Pro Ala Ala Glu Glu Tyr Gly Tyr Ile Val Thr Asp Gln Lys Pro
   465 470 475 480
   Leu Ser Leu Ala Ala Gly Val Lys Leu Leu Glu Ile Leu Ala Glu His
   485 490 495
   Val His Met Ser Ser Gly Ser Phe Ile Asn Ile Ser Val Val Gly Pro
   500 505 510
   Ala Leu Thr Phe Arg Ile Arg His Asn Glu Gln Asn Leu Ser Leu Ala
   515 520 525
   Asp Val Thr Gln Gln Ala Gly Leu Val Lys Ser Glu Leu Glu Ala Gln
   530 535 540
   Thr Gly Leu Gln Ile Leu Gln Thr Gly Val Gly Gln Arg Glu Glu Ala
   545 550 555 560
   Ala Ala Val Leu Pro Gln Thr Ala His Ser Thr Ser Pro Met Arg Ser
   565 570 575
   Val Leu Leu Thr Leu Val Ala Leu Ala Gly Val Ala Gly Leu Leu Val
   580 585 590
   Ala Leu Ala Val Ala Leu Cys Val Arg Gln His Ala Arg Gln Gln Asp
   595 600 605
   Lys Glu Arg Leu Ala Ala Leu Gly Pro Glu Gly Ala His Gly Asp Thr
   610 615 620
   Thr Phe Glu Tyr Gln Asp Leu Cys Arg Gln His Met Ala Thr Lys Ser
   625 630 635 640
   Leu Phe Asn Arg Ala Glu Gly Pro Pro Glu Pro Ser Arg Val Ser Ser
   645 650 655
   Val Ser Ser Gln Phe Ser Asp Ala Ala Gln Ala Ser Pro Ser Ser His
   660 665 670
   Ser Ser Thr Pro Ser Trp Cys Glu Glu Pro Ala Gln Ala Asn Met Asp
   675 680 685
   Ile Ser Thr Gly His Met Ile Leu Ala Tyr Met Glu Asp His Leu Arg
   690 695 700
   Asn Arg Asp Arg Leu Ala Lys Glu Trp Gln Ala Leu Cys Ala Tyr Gln
   705 710 715 720
   Ala Glu Pro Asn Thr Cys Ala Thr Ala Gln Gly Glu Gly Asn Ile Lys
   725 730 735
   Lys Asn Arg His Pro Asp Phe Leu Pro Tyr Asp His Ala Arg Ile Lys
   740 745 750
   Leu Lys Val Glu Ser Ser Pro Ser Arg Ser Asp Tyr Ile Asn Ala Ser
   755 760 765
   Pro Ile Ile Glu His Asp Pro Arg Met Pro Ala Tyr Ile Ala Thr Gln
   770 775 780
   Gly Pro Leu Ser His Thr Ile Ala Asp Phe Trp Gln Met Val Trp Glu
   785 790 795 800
   Ser Gly Cys Thr Val Ile Val Met Leu Thr Pro Leu Val Glu Asp Gly
   805 810 815
   Val Lys Gln Cys Asp Arg Tyr Trp Pro Asp Glu Gly Ala Ser Leu Tyr
   820 825 830
   His Val Tyr Glu Val Asn Leu Val Ser Glu His Ile Trp Cys Glu Asp
   835 840 845
   Phe Leu Val Arg Ser Phe Tyr Leu Lys Asn Val Gln Thr Gln Glu Thr
   850 855 860
   Arg Thr Leu Thr Gln Phe His Phe Leu Ser Trp Pro Ala Glu Gly Thr
   865 870 875 880
   Pro Ala Ser Thr Arg Pro Leu Leu Asp Phe Arg Arg Lys Val Asn Lys
   885 890 895
   Cys Tyr Arg Gly Arg Ser Cys Pro Ile Ile Val His Cys Ser Asp Gly
   900 905 910
   Ala Gly Arg Thr Gly Thr Tyr Ile Leu Ile Asp Met Val Leu Asn Arg
   915 920 925
   Met Ala Lys Gly Val Lys Glu Ile Asp Ile Ala Ala Thr Leu Glu His
   930 935 940
   Val Arg Asp Gln Arg Pro Gly Leu Val Arg Ser Lys Asp Gln Phe Glu
   945 950 955 960
   Phe Ala Leu Thr Ala Val Ala Glu Glu Val Asn Ala Ile Leu Lys Ala
   965 970 975
   Leu Pro Gln
<210> 3
   <211> 2400
   <212> DNA
   <213> Homo sapiens
<400> 3
   agctcgcccg cagctcgcac tcgcaggcga cctgctccag tctccaaagc cgatggcatc 60
   tccgggctct ggcttttggt ctttcgggtc ggaagatggc tctggggatt ccgagaatcc 120
   cggcacagcg cgagcctggt gccaagtggc tcagaagttc acgggcggca tcggaaacaa 180
   actgtgcgcc ctgctctacg gagacgccga gaagccggcg gagagcggcg ggagccaacc 240
   cccgcgggcc gccgcccgga aggccgcctg cgcctgcgac cagaagccct gcagctgctc 300
   caaagtggat gtcaactacg cgtttctcca tgcaacagac ctgctgccgg cgtgtgatgg 360
   agaaaggccc actttggcgt ttctgcaaga tgttatgaac attttacttc agtatgtggt 420
   gaaaagtttc gatagatcaa ccaaagtgat tgatttccat tatcctaatg agcttctcca 480
   agaatataat tgggaattgg cagaccaacc acaaaatttg gaggaaattt tgatgcattg 540
   ccaaacaact ctaaaatatg caattaaaac agggcatcct agatacttca atcaactttc 600
   tactggtttg gatatggttg gattagcagc agactggctg acatcaacag caaatactaa 660
   catgttcacc tatgaaattg ctccagtatt tgtgcttttg gaatatgtca cactaaagaa 720
   aatgagagaa atcattggct ggccaggggg ctctggcgat gggatatttt ctcccggtgg 780
   cgccatatct aacatgtatg ccatgatgat cgcacgcttt aagatgttcc cagaagtcaa 840
   ggagaaagga atggctgctc ttcccaggct cattgccttc acgtctgaac atagtcattt 900
   ttctctcaag aagggagctg cagccttagg gattggaaca gacagcgtga ttctgattaa 960
   atgtgatgag agagggaaaa tgattccatc tgatcttgaa agaaggattc ttgaagccaa 1020
   acagaaaggg tttgttcctt tcctcgtgag tgccacagct ggaaccaccg tgtacggagc 1080
   atttgacccc ctcttagctg tcgctgacat ttgcaaaaag tataagatct ggatgcatgt 1140
   ggatgcagct tggggtgggg gattactgat gtcccgaaaa cacaagtgga aactgagtgg 1200
   cgtggagagg gccaactctg tgacgtggaa tccacacaag atgatgggag tccctttgca 1260
   gtgctctgct ctcctggtta gagaagaggg attgatgcag aattgcaacc aaatgcatgc 1320
   ctcctacctc tttcagcaag ataaacatta tgacctgtcc tatgacactg gagacaaggc 1380
   cttacagtgc ggacgccacg ttgatgtttt taaactatgg ctgatgtgga gggcaaaggg 1440
   gactaccggg tttgaagcgc atgttgataa atgtttggag ttggcagagt atttatacaa 1500
   catcataaaa aaccgagaag gatatgagat ggtgtttgat gggaagcctc agcacacaaa 1560
   tgtctgcttc tggtacattc ctccaagctt gcgtactctg gaagacaatg aagagagaat 1620
   gagtcgcctc tcgaaggtgg ctccagtgat taaagccaga atgatggagt atggaaccac 1680
   aatggtcagc taccaaccct tgggagacaa ggtcaatttc ttccgcatgg tcatctcaaa 1740
   cccagcggca actcaccaag acattgactt cctgattgaa gaaatagaac gccttggaca 1800
   agatttataa taaccttgct caccaagctg ttccacttct ctaggtagac aattaagttg 1860
   tcacaaactg tgtgaatgta tttgtagttt gttccaaagt aaatctattt ctatattgtg 1920
   gtgtcaaagt agagtttaaa aattaaacaa aaaagacatt gctcctttta aaagtccttt 1980
   cttaagttta gaatacctct ctaagaattc gtgacaaaag gctatgttct aatcaataag 2040
   gaaaagctta aaattgttat aaatacttcc cttactttta atatagtgtg caaagcaaac 2100
   tttattttca cttcagacta gtaggactga atagtgccaa attgcccctg aatcataaaa 2160
   ggttctttgg ggtgcagtaa aaaggacaaa gtaaatataa aatatatgtt gacaataaaa 2220
   actcttgcct ttttcatagt attagaaaaa aatttctaat ttacctatag caacatttca 2280
   aatgtattta aatacatata attttacaaa aggaaaatat atatattaaa aaagatatcc 2340
   tattttgtaa catatagatt tttattttat ataggttata caaactgcgg gggcggaatt 2400
<210> 4
   <211> 3613
   <212> DNA
   <213> Homo sapiens
<400> 4
   cagcccctct ggcaggctcc cgccagcgtc gctgcggctc cggcccggga gcgagcgccc 60
   ggagctcgga aagatgcggc gcccgcggcg gcctgggggt ctcgggggat ccgggggtct 120
   ccggctgctc ctctgcctcc tgctgctgag cagccgcccg gggggctgca gcgccgttag 180
   tgcccacggc tgtctatttg accgcaggct ctgctctcac ctggaagtct gtattcagga 240
   tggcttgttt gggcagtgcc aggtgggagt ggggcaggcc cggccccttt tgcaagtcac 300
   ctccccagtt ctccaacgct tacaaggtgt gctccgacaa ctcatgtccc aaggattgtc 360
   ctggcacgat gacctcaccc agtatgtgat ctctcaggag atggagcgca tccccaggct 420
   tcgcccccca gagccccgtc caagggacag gtctggcttg gcacccaaga gacctggtcc 480
   tgctggagag ctgcttttac aggacatccc cactggctcc gcccctgctg cccagcatcg 540
   gcttccacaa ccaccagtgg gcaaaggtgg agctggggcc agctcctctc tgtcccctct 600
   gcaggctgag ctgctcccgc ctctcttgga gcacctgctg ctgcccccac agcctcccca 660
   cccttcactg agttacgaac ctgccttgct gcagccctac ctgttccacc agtttggctc 720
   ccgtgatggc tccagggtct cagagggctc cccagggatg gtcagtgtcg gccccctgcc 780
   caaggctgaa gcccctgccc tcttcagcag aactgcctcc aagggcatat ttggggacca 840
   ccctggccac tcctacgggg accttccagg gccttcacct gcccagcttt ttcaagactc 900
   tgggctgctc tatctggccc aggagttgcc agcacccagc agggccaggg tgccaaggct 960
   gccagagcaa gggagcagca gccgggcaga ggactcccca gagggctatg agaaggaagg 1020
   actaggggat cgtggagaga agcctgcttc cccagctgtg cagccagatg cggctctgca 1080
   gaggctggcc gctgtgctgg cgggctatgg ggtagagctg cgtcagctga cccctgagca 1140
   gctctccaca ctcctgaccc tgctgcagct actgcccaag ggtgcaggaa gaaatccggg 1200
   aggggttgta aatgttggag ctgatatcaa gaaaacaatg gaggggccgg tggagggcag 1260
   agacacagca gagcttccag cccgcacatc ccccatgcct ggacacccca ctgccagccc 1320
   tacctccagt gaagtccagc aggtgccaag ccctgtctcc tctgagcctc ccaaagctgc 1380
   cagaccccct gtgacacctg tcctgctaga gaagaaaagc ccactgggcc agagccagcc 1440
   cacggtggca ggacagccct cagcccgccc agcagcagag gaatatggct acatcgtcac 1500
   tgatcagaag cccctgagcc tggctgcagg agtgaagctg ctggagatcc tggctgagca 1560
   tgtgcacatg tcctcaggca gcttcatcaa catcagtgtg gtgggaccag ccctcacctt 1620
   ccgcatccgg cacaatgagc agaacctgtc tttggctgat gtgacccaac aagcagggct 1680
   ggtgaagtct gaactggaag cacagacagg gctccaaatc ttgcagacag gagtgggaca 1740
   gagggaggag gcagctgcag tccttcccca aactgcgcac agcacctcac ccatgcgctc 1800
   agtgctgctc actctggtgg ccctggcagg tgtggctggg ctgctggtgg ctctggctgt 1860
   ggctctgtgt gtgcggcagc atgcgcggca gcaagacaag gagcgcctgg cagccctggg 1920
   gcctgagggg gcccatggtg acactacctt tgagtaccag gacctgtgcc gccagcacat 1980
   ggccacgaag tccttgttca accgggcaga gggtccaccg gagccttcac gggtgagcag 2040
   tgtgtcctcc cagttcagcg acgcagccca ggccagcccc agctcccaca gcagcacccc 2100
   gtcctggtgc gaggagccgg cccaagccaa catggacatc tccacgggac acatgattct 2160
   ggcatacatg gaggatcacc tgcggaaccg ggaccgcctt gccaaggagt ggcaggccct 2220
   ctgtgcctac caagcagagc caaacacctg tgccaccgcg cagggggagg gcaacatcaa 2280
   aaagaaccgg catcctgact tcctgcccta tgaccatgcc cgcataaaac tgaaggtgga 2340
   gagcagccct tctcggagcg attacatcaa cgccagcccc attattgagc atgaccctcg 2400
   gatgccagcc tacatagcca cgcagggccc gctgtcccat accatcgcag acttctggca 2460
   gatggtgtgg gagagcggct gcaccgtcat cgtcatgctg accccgctgg tggaggatgg 2520
   tgtcaagcag tgtgaccgct actggccaga tgagggtgcc tccctctacc acgtatatga 2580
   ggtgaacctg gtgtcggagc acatctggtg cgaggacttt ctggtgcgga gcttctacct 2640
   gaagaacgtg cagacccagg agacgcgcac gctcacgcag ttccacttcc tcagctggcc 2700
   ggcagagggc acaccggcct ccacgcggcc cctgctggac ttccgcagga aggtgaacaa 2760
   gtgctaccgg ggccgctcct gccccatcat cgtgcactgc agtgatggtg cggggaggac 2820
   cggcacctac atcctcatcg acatggtcct gaaccgcatg gcaaaaggag tgaaggagat 2880
   tgacatcgct gccaccctgg agcatgtccg tgaccagcgg cctggccttg tccgctctaa 2940
   ggaccagttt gaatttgccc tgacagccgt ggcggaggaa gtgaatgcca tcctcaaggc 3000
   cctgccccag tgagaccctg gggccccttg gcgggcagcc cagcctctgt ccctctttgc 3060
   ctgtgtgagc atctctgtgt acccactcct cactgcccca ccagccacct cttgggcatg 3120
   ctcagccctt cctagaagag tcaggaaggg aaagccagaa ggggcacgcc tgcccagcct 3180
   cgcatgccag agcctggggc atcccagagc ccagggcatc ccatgggggt gctgcagcca 3240
   ggaggagagg aaaggacatg ggtagcaatt ctacccagag ccttctcctg cctacattcc 3300
   ctggcctggc tctcctgtag ctctcctggg gttctgggag ttccctgaac atctgtgtgt 3360
   gtccccctat gctccagtat ggaagaatgg ggtggagggt cgccacaccc ggctccccct 3420
   gcttctcagc cccgggcctg cctctgactc acacttgggc gctctgccct ccctggcctc 3480
   acgcccagcc tggtcccacc accctcccac catgcgctgc tcaacctctc tccttctggc 3540
   gcaagagaac atttctagaa aaaactactt ttgtaccagt gtgaataaag ttagtgtgtt 3600
   gtctgtgcag ctg 3613
<210> 5
   <211> 351
   <212> DNA
   <213> Homo sapiens
<220>
   <221> CDS
   <222> (1)..(351)
<400> 5
   tgc aca ggg tcc tgg gcc gag ctc gtg gtg act cag cct gcc tcc gtg    48
   Cys Thr Gly Ser Trp Ala Glu Leu Val Val Thr Gln Pro Ala Ser Val
   1               5                   10                  15
   tct ggg tct cct gga cag tcg atc acc atc tcc tgc act gga acc agc    96
   Ser Gly Ser Pro Gly Gln Ser Ile Thr Ile Ser Cys Thr Gly Thr Ser
               20                  25                  30
   agt gac gtt ggt ggt tat aac tat gtc tcc tgg tac cga cat cac cca    144
   Ser Asp Val Gly Gly Tyr Asn Tyr Val Ser Trp Tyr Arg His His Pro
           35                  40                  45
   gcc aaa gcc ccc aaa ctc atg att tat gat gtc agt aat cgg ccc tca    192
   Ala Lys Ala Pro Lys Leu Met Ile Tyr Asp Val Ser Asn Arg Pro Ser
       50                  55                  60
   ggg gtt tct aat cgc ttc tct ggc tcc aag tct ggc aac acg gcc tcc    240
   Gly Val Ser Asn Arg Phe Ser Gly Ser Lys Ser Gly Asn Thr Ala Ser
   65                  70                  75                  80
   ctg acc atc tct ggg ctc cag gct gag gac gag gct gat tat tac tgc    288
   Leu Thr Ile Ser Gly Leu Gln Ala Glu Asp Glu Ala Asp Tyr Tyr Cys
                   85                  90                  95
   agc tca tat aca agc agc agc act ctg gta ttc ggc gga ggg acc aag    336
   Ser Ser Tyr Thr Ser Ser Ser Thr Leu Val Phe Gly Gly Gly Thr Lys
               100                 105                 110
   ctg acc gtc cta ggt                                                351
   Leu Thr Val Leu Gly
           115
<210> 6
   <211> 117
   <212> PRT
   <213> Homo sapiens
<400> 6
   Cys Thr Gly Ser Trp Ala Glu Leu Val Val Thr Gln Pro Ala Ser Val
    1               5                  10                  15
   Ser Gly Ser Pro Gly Gln Ser Ile Thr Ile Ser Cys Thr Gly Thr Ser
               20                  25                  30
   Ser Asp Val Gly Gly Tyr Asn Tyr Val Ser Trp Tyr Arg His His Pro
           35                  40                  45
   Ala Lys Ala Pro Lys Leu Met Ile Tyr Asp Val Ser Asn Arg Pro Ser
       50                  55                  60
   Gly Val Ser Asn Arg Phe Ser Gly Ser Lys Ser Gly Asn Thr Ala Ser
   65                  70                  75                  80
   Leu Thr Ile Ser Gly Leu Gln Ala Glu Asp Glu Ala Asp Tyr Tyr Cys
                   85                  90                  95
   Ser Ser Tyr Thr Ser Ser Ser Thr Leu Val Phe Gly Gly Gly Thr Lys
               100                 105                 110
   Leu Thr Val Leu Gly
           115
<210> 7
   <211> 369
   <212> DNA
   <213> Homo sapiens
<220>
   <221> CDS
   <222> (1)..(369)
<400> 7
   nag gtg cag ctg ctc gag tcg ggc cca gga ctg gtg aag cct tcg gag   48
   Xaa Val Gln Leu Leu Glu Ser Gly Pro Gly Leu Val Lys Pro Ser Glu
    1               5                  10                  15
   acc ctg tcc ctc acc tgc act gtc tct ggt ggg tcc atc aat agt tac   96
   Thr Leu Ser Leu Thr Cys Thr Val Ser Gly Gly Ser Ile Asn Ser Tyr
               20                  25                  30
   tac tgg agc tgg atc cgg cag ccc cca ggg aaa gga ctg gag tgg att   144
   Tyr Trp Ser Trp Ile Arg Gln Pro Pro Gly Lys Gly Leu Glu Trp Ile
           35                  40                  45
   ggg ttc atc tct cac agt ggg atc acc aac tac aac ccg tcc cgc aag   192
   Gly Phe Ile Ser His Ser Gly Ile Thr Asn Tyr Asn Pro Ser Arg Lys
       50                  55                  60
   agt cga gtc acc ata tta gta gac acg tcc aag aac cag atc tcc ctg   240
   Ser Arg Val Thr Ile Leu Val Asp Thr Ser Lys Asn Gln Ile Ser Leu
   65                  70                  75                  80
   aag ctg agc tct gtg acc gct ggg gac acg gcc gtg tat tac tgt gcg   288
   Lys Leu Ser Ser Val Thr Ala Gly Asp Thr Ala Val Tyr Tyr Cys Ala
                   85                  90                  95
   aga gag ggg aaa gta tcc agt ggc tgg cga ctc tgg tac ttc gat ctc   336
   Arg Glu Gly Lys Val Ser Ser Gly Trp Arg Leu Trp Tyr Phe Asp Leu
               100                 105                 110
   tgg ggc cgt ggc acc ctg gtc act gtc tcc tca                       369
   Trp Gly Arg Gly Thr Leu Val Thr Val Ser Ser
           115                 120
<210> 8
   <211> 123
   <212> PRT
   <213> Homo sapiens
<400> 8
   Xaa Val Gln Leu Leu Glu Ser Gly Pro Gly Leu Val Lys Pro Ser Glu
   1               5                   10                  15
   Thr Leu Ser Leu Thr Cys Thr Val Ser Gly Gly Ser Ile Asn Ser Tyr
               20                  25                  30
   Tyr Trp Ser Trp Ile Arg Gln Pro Pro Gly Lys Gly Leu Glu Trp Ile
           35                  40                  45
   Gly Phe Ile Ser His Ser Gly Ile Thr Asn Tyr Asn Pro Ser Arg Lys
       50                  55                  60
   Ser Arg Val Thr Ile Leu Val Asp Thr Ser Lys Asn Gln Ile Ser Leu
   65                  70                  75                  80
   Lys Leu Ser Ser Val Thr Ala Gly Asp Thr Ala Val Tyr Tyr Cys Ala
                   85                  90                  95
   Arg Glu Gly Lys Val Ser Ser Gly Trp Arg Leu Trp Tyr Phe Asp Leu
               100                 105                 110
   Trp Gly Arg Gly Thr Leu Val Thr Val Ser Ser
           115                 120
<210> 9
   <211> 354
   <212> DNA
   <213> Homo sapiens
<220>
   <221> CDS
   <222> (1)..(354)
<400> 9
   tgc aca ggg tcc tgg gcc gag ctc gtg gtg act cag cca ccc tca gcg    48
   Cys Thr Gly Ser Trp Ala Glu Leu Val Val Thr Gln Pro Pro Ser Ala
    1               5                  10                  15
   tct ggg acc ccc ggg cag agg gtc acc atc tct tgt tct gga ggc aac    96
   Ser Gly Thr Pro Gly Gln Arg Val Thr Ile Ser Cys Ser Gly Gly Asn
               20                  25                  30
   tcc aac atc gga agt aat act gta aac tgg tac cag cag ctc cca gga    144
   Ser Asn Ile Gly Ser Asn Thr Val Asn Trp Tyr Gln Gln Leu Pro Gly
           35                  40                  45
   acg gcc ccc aag ctc ctc atc tat agt aat aat cag cgg ccc tca ggg    192
   Thr Ala Pro Lys Leu Leu Ile Tyr Ser Asn Asn Gln Arg Pro Ser Gly
       50                  55                  60
   gtc cct gac cga ttc tct ggc tcc aag tct ggc acc tca gcc tcc ctg    240
   Val Pro Asp Arg Phe Ser Gly Ser Lys Ser Gly Thr Ser Ala Ser Leu
   65                  70                  75                  80
   gcc atc agt ggg ctc cag tct gag gat gag gct gat tat tac tgt gca    288
   Ala Ile Ser Gly Leu Gln Ser Glu Asp Glu Ala Asp Tyr Tyr Cys Ala
                   85                  90                  95
   aca tgg gat gac agc ctg aat gcc tat gtg gta ttc ggc gga ggg acc    336
   Thr Trp Asp Asp Ser Leu Asn Ala Tyr Val Val Phe Gly Gly Gly Thr
               100                 105                 110
   aaa ctg acc gtc cta ggt                                            354
   Lys Leu Thr Val Leu Gly
           115
<210> 10
   <211> 118
   <212> PRT
   <213> Homo sapiens
<400> 10
   Cys Thr Gly Ser Trp Ala Glu Leu Val Val Thr Gln Pro Pro Ser Ala
   1               5                   10                  15
   Ser Gly Thr Pro Gly Gln Arg Val Thr Ile Ser Cys Ser Gly Gly Asn
               20                  25                  30
   Ser Asn Ile Gly Ser Asn Thr Val Asn Trp Tyr Gln Gln Leu Pro Gly
           35                  40                  45
   Thr Ala Pro Lys Leu Leu Ile Tyr Ser Asn Asn Gln Arg Pro Ser Gly
       50                  55                  60
   Val Pro Asp Arg Phe Ser Gly Ser Lys Ser Gly Thr Ser Ala Ser Leu
   65                  70                  75                  80
   Ala Ile Ser Gly Leu Gln Ser Glu Asp Glu Ala Asp Tyr Tyr Cys Ala
                   85                  90                  95
   Thr Trp Asp Asp Ser Leu Asn Ala Tyr Val Val Phe Gly Gly Gly Thr
               100                 105                 110
   Lys Leu Thr Val Leu Gly
           115
<210> 11
   <211> 351
   <212> DNA
   <213> Homo sapiens
<220>
   <221> CDS
   <222> (1)..(351)
<400> 11
   gtg caa ctg ctc gag tcg ggc cca gga ctg gtg aag cct tcg gag acc      48
   Val Gln Leu Leu Glu Ser Gly Pro Gly Leu Val Lys Pro Ser Glu Thr
   1               5                   10                  15
   ctg tcc ctc act tgc act gtc tct ggt ggc tcc atc act tct tac tac      96
   Leu Ser Leu Thr Cys Thr Val Ser Gly Gly Ser Ile Thr Ser Tyr Tyr
               20                  25                  30
   tgg agc tgg atc cgg cag ccc gcc ggg aag ggg ctg gac ttt att ggg      144
   Trp Ser Trp Ile Arg Gln Pro Ala Gly Lys Gly Leu Asp Phe Ile Gly
           35                  40                  45
   cgt gtc aac atc gct ggg agc acc aac tac aac ccc tcc ctc acg agt      192
   Arg Val Asn Ile Ala Gly Ser Thr Asn Tyr Asn Pro Ser Leu Thr Ser
        50                  55                  60
   cga gtc tcc atg tca gtg gac acg gcc aag aag cac ttc tcc ctg aga    240
   Arg Val Ser Met Ser Val Asp Thr Ala Lys Lys His Phe Ser Leu Arg
    65                  70                  75                  80
   ctg act tct gtg acc gcc gcg gac acg gcc gtt tac tac tgt gcg att    288
   Leu Thr Ser Val Thr Ala Ala Asp Thr Ala Val Tyr Tyr Cys Ala Ile
                   85                  90                  95
   tct ggt cgc tcc act ctg tta cct caa tac tgg ggc cag gga acc ctg    336
   Ser Gly Arg Ser Thr Leu Leu Pro Gln Tyr Trp Gly Gln Gly Thr Leu
               100                 105                 110
   gtc acc gtc tct tca                                                351
   Val Thr Val Ser Ser
           115
<210> 12
   <211> 117
   <212> PRT
   <213> Homo sapiens
<400> 12
   Val Gln Leu Leu Glu Ser Gly Pro Gly Leu Val Lys Pro Ser Glu Thr
    1               5                   10                  15
   Leu Ser Leu Thr Cys Thr Val Ser Gly Gly Ser Ile Thr Ser Tyr Tyr
                   20                  25                  30
   Trp Ser Trp Ile Arg Gln Pro Ala Gly Lys Gly Leu Asp Phe Ile Gly
               35                  40                  45
   Arg Val Asn Ile Ala Gly Ser Thr Asn Tyr Asn Pro Ser Leu Thr Ser
           50                  55                  60
   Arg Val Ser Met Ser Val Asp Thr Ala Lys Lys His Phe Ser Leu Arg
    65                  70                  75                  80
   Leu Thr Ser Val Thr Ala Ala Asp Thr Ala Val Tyr Tyr Cys Ala Ile
                   85                  90                  95
   Ser Gly Arg Ser Thr Leu Leu Pro Gln Tyr Trp Gly Gln Gly Thr Leu
               100                 105                 110
   Val Thr Val Ser Ser
           115
<210> 13
   <211> 351
   <212> DNA
   <213> Homo sapiens
<220>
   <221> CDS
   <222> (1)..(351)
<400> 13
   tgc aca ggg tcc tgg gcc gag ctc gtg gtg act cag cct ccc tcc gtg    48
   Cys Thr Gly Ser Trp Ala Glu Leu Val Val Thr Gln Pro Pro Ser Val
   1               5                   10                  15
   tcc ggg tct cct gga cag tca gtc acc atc tcc tgc act gga acc agc    96
   Ser Gly Ser Pro Gly Gln Ser Val Thr Ile Ser Cys Thr Gly Thr Ser
               20                  25                  30
   agt gac gtt ggt ggt tat aac cgt gtc tcc tgg tac cag cag ccc cca    144
   Ser Asp Val Gly Gly Tyr Asn Arg Val Ser Trp Tyr Gln Gln Pro Pro
           35                  40                  45
   ggc aca gcc ccc aaa ctc atg att tat gat gtc agt tat cgg ccc tca    192
   Gly Thr Ala Pro Lys Leu Met Ile Tyr Asp Val Ser Tyr Arg Pro Ser
       50                  55                  60
   ggg gtc cct gat cgc ttc tct ggg tcc aag tct ggc aac acg gcc tcc    240
   Gly Val Pro Asp Arg Phe Ser Gly Ser Lys Ser Gly Asn Thr Ala Ser
   65                  70                  75                  80
   ctg acc atc tct ggg ctc cag act gag gac gag gct gat tat tac tgc    288
   Leu Thr Ile Ser Gly Leu Gln Thr Glu Asp Glu Ala Asp Tyr Tyr Cys
                   85                  90                  95
   agc tca tat aca agc agc agt ctt tat gtc ttc gga act ggg acc aag    336
   Ser Ser Tyr Thr Ser Ser Ser Leu Tyr Val Phe Gly Thr Gly Thr Lys
               100                 105                 110
   gtc acc gtc cta ggt                                                351
   Val Thr Val Leu Gly
           115
<210> 14
   <211> 117
   <212> PRT
   <213> Homo sapiens
<400> 14
   Cys Thr Gly Ser Trp Ala Glu Leu Val Val Thr Gln Pro Pro Ser Val
    1               5                  10                  15
   Ser Gly Ser Pro Gly Gln Ser Val Thr Ile Ser Cys Thr Gly Thr Ser
               20                  25                  30
   Ser Asp Val Gly Gly Tyr Asn Arg Val Ser Trp Tyr Gln Gln Pro Pro
           35                  40                  45
   Gly Thr Ala Pro Lys Leu Met Ile Tyr Asp Val Ser Tyr Arg Pro Ser
       50                  55                  60
   Gly Val Pro Asp Arg Phe Ser Gly Ser Lys Ser Gly Asn Thr Ala Ser
   65                  70                  75                  80
   Leu Thr Ile Ser Gly Leu Gln Thr Glu Asp Glu Ala Asp Tyr Tyr Cys
                   85                  90                  95
   Ser Ser Tyr Thr Ser Ser Ser Leu Tyr Val Phe Gly Thr Gly Thr Lys
               100                 105                 110
   Val Thr Val Leu Gly
           115
<210> 15
   <211> 366
   <212> DNA
   <213> Homo sapiens
<220>
   <221> CDS
   <222> (1)..(366)
<400> 15
   nag gtg cag ctg ctc gag tct ggg gct gag gtg aag aag cct ggg gcc     48
   Xaa Val Gln Leu Leu Glu Ser Gly Ala Glu Val Lys Lys Pro Gly Ala
    1               5                  10                  15
   tca gtg aag gtt tcc tgc aag gcc tct gga tac acc ttc acc aac tac     96
   Ser Val Lys Val Ser Cys Lys Ala Ser Gly Tyr Thr Phe Thr Asn Tyr
               20                  25                  30
   tat ata tac tgg gtg cga cag gcc cct gga caa ggg ctt gag tgg atg     144
   Tyr Ile Tyr Trp Val Arg Gln Ala Pro Gly Gln Gly Leu Glu Trp Met
           35                  40                  45
   gga ata atc aat cct agt ggt ggg agc aca agc tac gca gag gag ttc     192
   Gly Ile Ile Asn Pro Ser Gly Gly Ser Thr Ser Tyr Ala Glu Glu Phe
       50                  55                  60
   cag ggc aga gtc acc ttg acc agg gac acg tcc acg agc aca gtc tac     240
   Gln Gly Arg Val Thr Leu Thr Arg Asp Thr Ser Thr Ser Thr Val Tyr
   65                  70                  75                  80
   atg gaa ctg agc agc ctg aga tct gag gac acg gcc ctc tat tac tgt     288
   Met Glu Leu Ser Ser Leu Arg Ser Glu Asp Thr Ala Leu Tyr Tyr Cys
                   85                  90                  95
   gcg aga gac ttt caa gcg tac tac agg tat ggt tat ctc tac gcc tgg     336
   Ala Arg Asp Phe Gln Ala Tyr Tyr Arg Tyr Gly Tyr Leu Tyr Ala Trp
               100                 105                 110
   ggc cag gga acc ttg tcc tcc gtc tcc tca                             366
   Gly Gln Gly Thr Leu Ser Ser Val Ser Ser
           115                 120
<210> 16
   <211> 122
   <212> PRT
   <213> Homo sapiens
<400> 16
   Xaa Val Gln Leu Leu Glu Ser Gly Ala Glu Val Lys Lys Pro Gly Ala
    1               5                  10                  15
   Ser Val Lys Val Ser Cys Lys Ala Ser Gly Tyr Thr Phe Thr Asn Tyr
               20                  25                  30
   Tyr Ile Tyr Trp Val Arg Gln Ala Pro Gly Gln Gly Leu Glu Trp Met
           35                  40                  45
   Gly Ile Ile Asn Pro Ser Gly Gly Ser Thr Ser Tyr Ala Glu Glu Phe
       50                  55                  60
   Gln Gly Arg Val Thr Leu Thr Arg Asp Thr Ser Thr Ser Thr Val Tyr
   65                  70                  75                  80
   Met Glu Leu Ser Ser Leu Arg Ser Glu Asp Thr Ala Leu Tyr Tyr Cys
                   85                  90                  95
   Ala Arg Asp Phe Gln Ala Tyr Tyr Arg Tyr Gly Tyr Leu Tyr Ala Trp
               100                 105                 110
   Gly Gln Gly Thr Leu Ser Ser Val Ser Ser
           115                 120
<210> 17
   <211> 336
   <212> DNA
   <213> Homo sapiens
<220>
   <221> CDS
   <222> (1)..(336)
<400> 17
   gcc aga tgt gag ctc gtg atg acc cag tct cca gcc acc ctg tct gtg    48
   Ala Arg Cys Glu Leu Val Met Thr Gln Ser Pro Ala Thr Leu Ser Val
   1               5                   10                  15
   tct cca ggg gaa aga gcc acc ctc tcc tgc agg gcc agt cag agt gtt    96
   Ser Pro Gly Glu Arg Ala Thr Leu Ser Cys Arg Ala Ser Gln Ser Val
               20                  25                  30
   aac agc aac tta gcc tgg tac cag cag aaa cct ggc cag gct ccc agg    144
   Asn Ser Asn Leu Ala Trp Tyr Gln Gln Lys Pro Gly Gln Ala Pro Arg
           35                  40                  45
   ctc ctc atc tat aat aca tcc acc agg gcc act ggt atc cca gcc agg    192
   Leu Leu Ile Tyr Asn Thr Ser Thr Arg Ala Thr Gly Ile Pro Ala Arg
       50                  55                  60
   ttc agt ggc agt ggg tct ggg aca gag ttc act ctc acc atc agc agc    240
   Phe Ser Gly Ser Gly Ser Gly Thr Glu Phe Thr Leu Thr Ile Ser Ser
   65                  70                  75                  80
   ctg cag tct gaa gat ttt gca ctt tat tac tgt cag cag tat aat aac    288
   Leu Gln Ser Glu Asp Phe Ala Leu Tyr Tyr Cys Gln Gln Tyr Asn Asn
                   85                  90                  95
   tgg cct ccg agg gcc act ttc ggc gga ggg acc aag gtg gag atc aaa    336
   Trp Pro Pro Arg Ala Thr Phe Gly Gly Gly Thr Lys Val Glu Ile Lys
               100                 105                 110
<210> 18
   <211> 112
   <212> PRT
   <213> Homo sapiens
<400> 18
   Ala Arg Cys Glu Leu Val Met Thr Gln Ser Pro Ala Thr Leu Ser Val
   1               5                   10                  15
   Ser Pro Gly Glu Arg Ala Thr Leu Ser Cys Arg Ala Ser Gln Ser Val
               20                  25                  30
   Asn Ser Asn Leu Ala Trp Tyr Gln Gln Lys Pro Gly Gln Ala Pro Arg
           35                  40                  45
   Leu Leu Ile Tyr Asn Thr Ser Thr Arg Ala Thr Gly Ile Pro Ala Arg
       50                  55                  60
   Phe Ser Gly Ser Gly Ser Gly Thr Glu Phe Thr Leu Thr Ile Ser Ser
   65                  70                  75                  80
   Leu Gln Ser Glu Asp Phe Ala Leu Tyr Tyr Cys Gln Gln Tyr Asn Asn
                   85                  90                  95
   Trp Pro Pro Arg Ala Thr Phe Gly Gly Gly Thr Lys Val Glu Ile Lys
               100                 105                 110
<210> 19
   <211> 354
   <212> DNA
   <213> Homo sapiens
<220>
   <221> CDS
   <222> (1)..(359)
<400> 19
   nag gtg cag ctg ctc gag tcg gga gca gag gtg aaa aag ccc ggg gag    48
   Xaa Val Gln Leu Leu Glu Ser Gly Ala Glu Val Lys Lys Pro Gly Glu
    1               5                  10                  15
   tct ctg aag atc tcc tgt aag gct tct gga tac agg ctt gcc agc tat    96
   Ser Leu Lys Ile Ser Cys Lys Ala Ser Gly Tyr Arg Leu Ala Ser Tyr
               20                  25                  30
   tgg atc gcc tgg gtg cgc cag atg ccc ggg aaa ggc ctg gag tgg atg    144
   Trp Ile Ala Trp Val Arg Gln Met Pro Gly Lys Gly Leu Glu Trp Met
           35                  40                  45
   ggg atc atc tat cct gat gac tct gat acc aca tat agc ccg tcc ttc    192
   Gly Ile Ile Tyr Pro Asp Asp Ser Asp Thr Thr Tyr Ser Pro Ser Phe
       50                  55                  60
   caa ggc cat gtc acc atc tca gcc gac aag tcc atc aac acc gcc tac    240
   Gln Gly His Val Thr Ile Ser Ala Asp Lys Ser Ile Asn Thr Ala Tyr
   65                  70                  75                  80
   ctg cag tgg agc agc ctg agg gcc tcg gac acc gcc ata tat tac tgt    288
   Leu Gln Trp Ser Ser Leu Arg Ala Ser Asp Thr Ala Ile Tyr Tyr Cys
                   85                  90                  95
   gcg aga ctc gag ggt tta ggg tgg ttc gac ccc tgg ggc cag gga acc    336
   Ala Arg Leu Glu Gly Leu Gly Trp Phe Asp Pro Trp Gly Gln Gly Thr
               100                 105                 110
   ctg gtc acc gtc tcc tca                                            354
   Leu Val Thr Val Ser Ser
           115
<210> 20
   <211> 118
   <212> PRT
   <213> Homo sapiens
<400> 20
   Xaa Val Gln Leu Leu Glu Ser Gly Ala Glu Val Lys Lys Pro Gly Glu
   1               5                   10                  15
   Ser Leu Lys Ile Ser Cys Lys Ala Ser Gly Tyr Arg Leu Ala Ser Tyr
               20                  25                  30
   Trp Ile Ala Trp Val Arg Gln Met Pro Gly Lys Gly Leu Glu Trp Met
           35                  40                  45
   Gly Ile Ile Tyr Pro Asp Asp Ser Asp Thr Thr Tyr Ser Pro Ser Phe
       50                  55                  60
   Gln Gly His Val Thr Ile Ser Ala Asp Lys Ser Ile Asn Thr Ala Tyr
   65                  70                  75                  80
   Leu Gln Trp Ser Ser Leu Arg Ala Ser Asp Thr Ala Ile Tyr Tyr Cys
                   85                  90                  95
   Ala Arg Leu Glu Gly Leu Gly Trp Phe Asp Pro Trp Gly Gln Gly Thr
               100                 105                 110
   Leu Val Thr Val Ser Ser
           115
<210> 21
   <211> 345
   <212> DNA
   <213> Homo sapiens
<220>
   <221> CDS
   <222> (1)..(345)
<400> 21
   gcc aga tgt gag ctc gtg atg acc cag tct cca ctc tcc ctg ccc gtc    48
   Ala Arg Cys Glu Leu Val Met Thr Gln Ser Pro Leu Ser Leu Pro Val
     1               5                  10                  15
   acc ctt gga cag ccg gcc tcc atc tcc tgc agg tct agt caa ggc ctc    96
   Thr Leu Gly Gln Pro Ala Ser Ile Ser Cys Arg Ser Ser Gln Gly Leu
                20                  25                  30
   gta tac agt gat gga aaa tct tac ttg agt tgg ttc cag cag agg cca   144
   Val Tyr Ser Asp Gly Lys Ser Tyr Leu Ser Trp Phe Gln Gln Arg Pro
            35                  40                  45
   ggc caa tct cca agg cgc cta att tat aaa gtt tct agc cgg gac tct   192
   Gly Gln Ser Pro Arg Arg Leu Ile Tyr Lys Val Ser Ser Arg Asp Ser
        50                  55                  60
   ggg gtc cca gac aga ttc agc ggc agt gga tca ggc act gat ttc aca   240
   Gly Val Pro Asp Arg Phe Ser Gly Ser Gly Ser Gly Thr Asp Phe Thr
    65                  70                  75                  80
   ctg aaa atc agc agg gtg gag gct gag gat gtt ggg gtt tat tac tgc   288
   Leu Lys Ile Ser Arg Val Glu Ala Glu Asp Val Gly Val Tyr Tyr Cys
                    85                  90                  95
   atg caa ggt aca cac tgg cct ggc acg ttc ggc caa ggg acc agg gtg   336
   Met Gln Gly Thr His Trp Pro Gly Thr Phe Gly Gln Gly Thr Arg Val
               100                 105                 110
   gaa atc aaa                                                       345
   Glu Ile Lys
           115
<210> 22
   <211> 115
   <212> PRT
   <213> Homo sapiens
<400> 22
   Ala Arg Cys Glu Leu Val Met Thr Gln Ser Pro Leu Ser Leu Pro Val
   1 5 10 15
   Thr Leu Gly Gln Pro Ala Ser Ile Ser Cys Arg Ser Ser Gln Gly Leu
   20 25 30
   Val Tyr Ser Asp Gly Lys Ser Tyr Leu Ser Trp Phe Gln Gln Arg Pro
   35 40 45
   Gly Gln Ser Pro Arg Arg Leu Ile Tyr Lys Val Ser Ser Arg Asp Ser
   50 55 60
   Gly Val Pro Asp Arg Phe Ser Gly Ser Gly Ser Gly Thr Asp Phe Thr
   65 70 75 80
   Leu Lys Ile Ser Arg Val Glu Ala Glu Asp Val Gly Val Tyr Tyr Cys
   85 90 95
   Met Gln Gly Thr His Trp Pro Gly Thr Phe Gly Gln Gly Thr Arg Val
   100 105 110
   Glu Ile Lys
   115
<210> 23
   <211> 366
   <212> DNA
   <213> Homo sapiens
<220>
   <221> CDS
   <222> (1)..(366)
<400> 23
   cag gtt cac ctg ggg caa tct gga gct gag gtg aag aag cct ggg gcc 48
   Gln Val His Leu Gly Gln Ser Gly Ala Glu Val Lys Lys Pro Gly Ala
   1 5 10 15
   tca gtg aag gtc tcc tgc aag gct tct ggt tac aag ttt tac aat tat 96
   Ser Val Lys Val Ser Cys Lys Ala Ser Gly Tyr Lys Phe Tyr Asn Tyr
   20 25 30
   ggt atc agt tgg gtg cga cag gcc cct gga caa ggg ctt gag tgg atg 144
   Gly Ile Ser Trp Val Arg Gln Ala Pro Gly Gln Gly Leu Glu Trp Met
   35 40 45
   gga tgg atc agc ggt tac aat ggt aac aca aac tat gca cac aag ttc    192
   Gly Trp Ile Ser Gly Tyr Asn Gly Asn Thr Asn Tyr Ala His Lys Phe
       50                  55                  60
   ctg ggc aga gtc acc atg acc gca gac aca tcc acg agc aca gcc tac    240
   Leu Gly Arg Val Thr Met Thr Ala Asp Thr Ser Thr Ser Thr Ala Tyr
   65                  70                  75                  80
   atg gag ttg agg agc ctg aga tcg gac gac acg gcc gta tat tac tgt    288
   Met Glu Leu Arg Ser Leu Arg Ser Asp Asp Thr Ala Val Tyr Tyr Cys
                   85                  90                  95
   gcg aga gac act aca cta atg gcg tat gct agc ctc ttt gac cac tgg    336
   Ala Arg Asp Thr Thr Leu Met Ala Tyr Ala Ser Leu Phe Asp His Trp
               100                 105                 110
   ggc cag gga acc ctg gtc acc gtc tcc tca                            366
   Gly Gln Gly Thr Leu Val Thr Val Ser Ser
           115                 120
<210> 24
   <211> 122
   <212> PRT
   <213> Homo sapiens
<400> 24
   Gln Val His Leu Gly Gln Ser Gly Ala Glu Val Lys Lys Pro Gly Ala
   1               5                   10                  15
   Ser Val Lys Val Ser Cys Lys Ala Ser Gly Tyr Lys Phe Tyr Asn Tyr
               20                  25                  30
   Gly Ile Ser Trp Val Arg Gln Ala Pro Gly Gln Gly Leu Glu Trp Met
           35                  40                  45
   Gly Trp Ile Ser Gly Tyr Asn Gly Asn Thr Asn Tyr Ala His Lys Phe
       50                  55                  60
   Leu Gly Arg Val Thr Met Thr Ala Asp Thr Ser Thr Ser Thr Ala Tyr
   65                  70                  75                  80
   Met Glu Leu Arg Ser Leu Arg Ser Asp Asp Thr Ala Val Tyr Tyr Cys
                   85                  90                  95
   Ala Arg Asp Thr Thr Leu Met Ala Tyr Ala Ser Leu Phe Asp His Trp
               100                 105                 110
   Gly Gln Gly Thr Leu Val Thr Val Ser Ser
           115                 120
<210> 25
   <211> 351
   <212> DNA
   <213> Homo sapiens
<220>
   <221> CDS
   <222> (1)..(351)
<400> 25
   tgc aca ggg tcc tgg gcc gag ctc gtg gtg act cag cct ccc tcc gcg    48
   Cys Thr Gly Ser Trp Ala Glu Leu Val Val Thr Gln Pro Pro Ser Ala
    1               5                  10                  15
   tcc ggg tct cct gga cag tca gtc acc atc tcc tgc act gga acc agc    96
   Ser Gly Ser Pro Gly Gln Ser Val Thr Ile Ser Cys Thr Gly Thr Ser
               20                  25                  30
   agt gac gtt ggt ggt tat aac tat gtc tcc tgg tac caa cag cac cca    144
   Ser Asp Val Gly Gly Tyr Asn Tyr Val Ser Trp Tyr Gln Gln His Pro
           35                  40                  45
   ggc aaa gcc ccc aaa ctc atg att ttt gag gtc act aag cgg ccc tca    192
   Gly Lys Ala Pro Lys Leu Met Ile Phe Glu Val Thr Lys Arg Pro Ser
       50                  55                  60
   ggg gtc cct gat cgc ttc tct ggc tcc aag tct ggc aac acg gcc tcc    240
   Gly Val Pro Asp Arg Phe Ser Gly Ser Lys Ser Gly Asn Thr Ala Ser
   65                  70                  75                  80
   ctg acc gtc tct ggg ctc cag gct gag gat gag gct gat tat tac tgc    288
   Leu Thr Val Ser Gly Leu Gln Ala Glu Asp Glu Ala Asp Tyr Tyr Cys
                   85                  90                  95
   acc tca tat gca ggc agc aac aat ttg gtg ttc ggc gga ggg acc aag    336
   Thr Ser Tyr Ala Gly Ser Asn Asn Leu Val Phe Gly Gly Gly Thr Lys
               100                 105                 110
   ctg acc gtc cta ggt                                                351
   Leu Thr Val Leu Gly
           115
<210> 26
   <211> 117
   <212> PRT
   <213> Homo sapiens
<400> 26
   Cys Thr Gly Ser Trp Ala Glu Leu Val Val Thr Gln Pro Pro Ser Ala
   1 5 10 15
   Ser Gly Ser Pro Gly Gln Ser Val Thr Ile Ser Cys Thr Gly Thr Ser
   20 25 30
   Ser Asp Val Gly Gly Tyr Asn Tyr Val Ser Trp Tyr Gln Gln His Pro
   35 40 45
   Gly Lys Ala Pro Lys Leu Met Ile Phe Glu Val Thr Lys Arg Pro Ser
   50 55 60
   Gly Val Pro Asp Arg Phe Ser Gly Ser Lys Ser Gly Asn Thr Ala Ser
   65 70 75 80
   Leu Thr Val Ser Gly Leu Gln Ala Glu Asp Glu Ala Asp Tyr Tyr Cys
   85 90 95
   Thr Ser Tyr Ala Gly Ser Asn Asn Leu Val Phe Gly Gly Gly Thr Lys
   100 105 110
   Leu Thr Val Leu Gly
   115
<210> 27
   <211> 381
   <212> DNA
   <213> Homo sapiens
<220>
   <221> CDS
   <222> (1)..(381)
<400> 27
   cag gtg caa ctg ctc gag tcg ggc cca gga ctg gtg aag cct tcg gag   48
   Gln Val Gln Leu Leu Glu Ser Gly Pro Gly Leu Val Lys Pro Ser Glu
    1               5                  10                  15
   acc ctg tcc ctc acc tgc act gtc tct ggt ggc tcc atc agc agt agg   96
   Thr Leu Ser Leu Thr Cys Thr Val Ser Gly Gly Ser Ile Ser Ser Arg
               20                  25                  30
   aat tac tac tgg ggc tgg atc cgc cag ccc cca ggg aag ggg ctg gag   144
   Asn Tyr Tyr Trp Gly Trp Ile Arg Gln Pro Pro Gly Lys Gly Leu Glu
           35                  40                  45
   tgg att gga agt att tat cat agt ggg aga acc tac tat aac ttg tcc   192
   Trp Ile Gly Ser Ile Tyr His Ser Gly Arg Thr Tyr Tyr Asn Leu Ser
       50                  55                  60
   ctc aag agt cga gtc acc ata tcc gta gac acg tcc aag aac cag ttc   240
   Leu Lys Ser Arg Val Thr Ile Ser Val Asp Thr Ser Lys Asn Gln Phe
   65                  70                  75                  80
   tcc ctg aag ctg agc tct gtg acc gcc gca gac acg gct gtg tat tac   288
   Ser Leu Lys Leu Ser Ser Val Thr Ala Ala Asp Thr Ala Val Tyr Tyr
                   85                  90                  95
   tgt gcg agg tca tat tgt agt gct gtt acc tgc tac cca gac tac tac   336
   Cys Ala Arg Ser Tyr Cys Ser Ala Val Thr Cys Tyr Pro Asp Tyr Tyr
               100                 105                 110
   ggt ctg gac atc tgg ggc caa ggg acc acg gtc acc gtc tcc tca       381
   Gly Leu Asp Ile Trp Gly Gln Gly Thr Thr Val Thr Val Ser Ser
           115                 120                 125
<210> 28
   <211> 127
   <212> PRT
   <213> Homo sapiens
<400> 28
   Gln Val Gln Leu Leu Glu Ser Gly Pro Gly Leu Val Lys Pro Ser Glu
    1               5                  10                  15
   Thr Leu Ser Leu Thr Cys Thr Val Ser Gly Gly Ser Ile Ser Ser Arg
               20                  25                  30
   Asn Tyr Tyr Trp Gly Trp Ile Arg Gln Pro Pro Gly Lys Gly Leu Glu
           35                  40                  45
   Trp Ile Gly Ser Ile Tyr His Ser Gly Arg Thr Tyr Tyr Asn Leu Ser
       50                  55                  60
   Leu Lys Ser Arg Val Thr Ile Ser Val Asp Thr Ser Lys Asn Gln Phe
   65                  70                  75                  80
   Ser Leu Lys Leu Ser Ser Val Thr Ala Ala Asp Thr Ala Val Tyr Tyr
                   85                  90                  95
   Cys Ala Arg Ser Tyr Cys Ser Ala Val Thr Cys Tyr Pro Asp Tyr Tyr
               100                 105                 110
   Gly Leu Asp Ile Trp Gly Gln Gly Thr Thr Val Thr Val Ser Ser
           115                 120                 125
<210> 29
   <211> 294
   <212> DNA
   <213> Homo sapiens
<220>
   <221> CDS
   <222> (1)..(294)
<400> 29
   gcc aga tgt gag ctc gtg atg acc cag tct cca tcc acc ctg tct gca      48
   Ala Arg Cys Glu Leu Val Met Thr Gln Ser Pro Ser Thr Leu Ser Ala
   1               5                   10                  15
   tct gta gga gac aga gtc acc atc act tgc cgg gcc agt cag aat att      96
   Ser Val Gly Asp Arg Val Thr Ile Thr Cys Arg Ala Ser Gln Asn Ile
               20                  25                  30
   aat agt tgg ttg gcc tgg tat cag cag aaa cca ggg aaa gcc cct aac      144
   Asn Ser Trp Leu Ala Trp Tyr Gln Gln Lys Pro Gly Lys Ala Pro Asn
           35                  40                  45
   ctc ctg atc tct aag gcg tct act tta gaa agt ggg gtc cca tca agg      192
   Leu Leu Ile Ser Lys Ala Ser Thr Leu Glu Ser Gly Val Pro Ser Arg
       50                  55                  60
   ttc agc ggc agt gga tct ggg aca gaa ttc act ctc acc atc agc agc      240
   Phe Ser Gly Ser Gly Ser Gly Thr Glu Phe Thr Leu Thr Ile Ser Ser
   65                  70                  75                  80
   ctg cag cct gat gat ttt gca agt tat tac tgc caa cag tat aag aat      288
   Leu Gln Pro Asp Asp Phe Ala Ser Tyr Tyr Cys Gln Gln Tyr Lys Asn
                   85                  90                  95
   tac tcg                                                              294
   Tyr Ser
<210> 30
   <211> 98
   <212> PRT
   <213> Homo sapiens
<400> 30
   Ala Arg Cys Glu Leu Val Met Thr Gln Ser Pro Ser Thr Leu Ser Ala
   1 5 10 15
   Ser Val Gly Asp Arg Val Thr Ile Thr Cys Arg Ala Ser Gln Asn Ile
   20 25 30
   Asn Ser Trp Leu Ala Trp Tyr Gln Gln Lys Pro Gly Lys Ala Pro Asn
   35 40 45
   Leu Leu Ile Ser Lys Ala Ser Thr Leu Glu Ser Gly Val Pro Ser Arg
   50 55 60
   Phe Ser Gly Ser Gly Ser Gly Thr Glu Phe Thr Leu Thr Ile Ser Ser
   65 70 75 80
   Leu Gln Pro Asp Asp Phe Ala Ser Tyr Tyr Cys Gln Gln Tyr Lys Asn
   85 90 95
   Tyr Ser
<210> 31
   <211> 348
   <212> DNA
   <213> Homo sapiens
<220>
   <221> CDS
   <222> (1)..(348)
<400> 31
   gtg cag ctg ctc gag tct ggg gct gag gtg aag aag cct ggg tcc tcg    48
   Val Gln Leu Leu Glu Ser Gly Ala Glu Val Lys Lys Pro Gly Ser Ser
    1               5                  10                  15
   gtg aag gtc tcc tgc aag gct tct gga ggc acg ttc aac att tat tct    96
   Val Lys Val Ser Cys Lys Ala Ser Gly Gly Thr Phe Asn Ile Tyr Ser
               20                  25                  30
   ttc agt tgg gtg cga cag gcc cct gga caa ggg ctt gaa tgg atg gga    144
   Phe Ser Trp Val Arg Gln Ala Pro Gly Gln Gly Leu Glu Trp Met Gly
           35                  40                  45
   gga atc atc cct atc tat cgt cca gca aat tac gca cag aat ttc cag    192
   Gly Ile Ile Pro Ile Tyr Arg Pro Ala Asn Tyr Ala Gln Asn Phe Gln
       50                  55                  60
   ggc aga gtc acg att acc gcg gac gaa tcc acg agc aca gtc tac atg    240
   Gly Arg Val Thr Ile Thr Ala Asp Glu Ser Thr Ser Thr Val Tyr Met
   65                  70                  75                  80
   gac ctg agc agc ctg agg tct gac gac acg gcc atg tat tac tgt gcg    288
   Asp Leu Ser Ser Leu Arg Ser Asp Asp Thr Ala Met Tyr Tyr Cys Ala
                   85                  90                  95
   aga ggg aca ggg ggt tat tac aac tcc tgg ggc cag gga acc ctg gtc    336
   Arg Gly Thr Gly Gly Tyr Tyr Asn Ser Trp Gly Gln Gly Thr Leu Val
               100                 105                 110
   acc gtc tcc tca                                                    348
   Thr Val Ser Ser
           115
<210> 32
   <211> 116
   <212> PRT
   <213> Homo sapiens
<400> 32
   Val Gln Leu Leu Glu Ser Gly Ala Glu Val Lys Lys Pro Gly Ser Ser
   1               5                   10                  15
   Val Lys Val Ser Cys Lys Ala Ser Gly Gly Thr Phe Asn Ile Tyr Ser
               20                  25                  30
   Phe Ser Trp Val Arg Gln Ala Pro Gly Gln Gly Leu Glu Trp Met Gly
           35                  40                  45
   Gly Ile Ile Pro Ile Tyr Arg Pro Ala Asn Tyr Ala Gln Asn Phe Gln
       50                  55                  60
   Gly Arg Val Thr Ile Thr Ala Asp Glu Ser Thr Ser Thr Val Tyr Met
   65                  70                  75                  80
   Asp Leu Ser Ser Leu Arg Ser Asp Asp Thr Ala Met Tyr Tyr Cys Ala
                   85                  90                  95
   Arg Gly Thr Gly Gly Tyr Tyr Asn Ser Trp Gly Gln Gly Thr Leu Val
               100                 105                 110
   Thr Val Ser Ser
           115
<210> 33
   <211> 331
   <212> DNA
   <213> Homo sapiens
<220>
   <221> CDS
   <222> (1)..(330)
<400> 33
   cag tct gtg ctg act cag cca ccc tca gcg tct ggg acc ccc ggg cag    48
   Gln Ser Val Leu Thr Gln Pro Pro Ser Ala Ser Gly Thr Pro Gly Gln
    1               5                  10                  15
   agg gtc acc atc tct tgt tct gga agc agc tcc aac atc gga agt gat    96
   Arg Val Thr Ile Ser Cys Ser Gly Ser Ser Ser Asn Ile Gly Ser Asp
               20                  25                  30
   agt gtg tgc tgg tac cag cac ctc cca gga acg gcc ccc aaa ctc ctc    144
   Ser Val Cys Trp Tyr Gln His Leu Pro Gly Thr Ala Pro Lys Leu Leu
           35                  40                  45
   atc tat ggt aat aat ctg cgg ccc tca tgg gtc tct gac cga ttc tct    192
   Ile Tyr Gly Asn Asn Leu Arg Pro Ser Trp Val Ser Asp Arg Phe Ser
       50                  55                  60
   ggc tcc gag tct ggc acc tca gcc tcc ctg gcc atc agt ggg ctc cag    240
   Gly Ser Glu Ser Gly Thr Ser Ala Ser Leu Ala Ile Ser Gly Leu Gln
   65                  70                  75                  80
   tct gag gat gag gct gat tat tac tgt tca gca tgg gat gac tac ctg    288
   Ser Glu Asp Glu Ala Asp Tyr Tyr Cys Ser Ala Trp Asp Asp Tyr Leu
                   85                  90                  95
   aat gct gtg gta ttc ggc gga ggg acc aag ctg acc gtc cta g          331
   Asn Ala Val Val Phe Gly Gly Gly Thr Lys Leu Thr Val Leu
               100                 105                 110
<210> 34
   <211> 110
   <212> PRT
   <213> Homo sapiens
<400> 34
   Gln Ser Val Leu Thr Gln Pro Pro Ser Ala Ser Gly Thr Pro Gly Gln
   1               5                   10                  15
   Arg Val Thr Ile Ser Cys Ser Gly Ser Ser Ser Asn Ile Gly Ser Asp
               20                  25                  30
   Ser Val Cys Trp Tyr Gln His Leu Pro Gly Thr Ala Pro Lys Leu Leu
           35                  40                  45
   Ile Tyr Gly Asn Asn Leu Arg Pro Ser Trp Val Ser Asp Arg Phe Ser
       50                  55                  60
   Gly Ser Glu Ser Gly Thr Ser Ala Ser Leu Ala Ile Ser Gly Leu Gln
   65                  70                  75                  80
   Ser Glu Asp Glu Ala Asp Tyr Tyr Cys Ser Ala Trp Asp Asp Tyr Leu
                   85                  90                  95
   Asn Ala Val Val Phe Gly Gly Gly Thr Lys Leu Thr Val Leu
               100                 105                 110
<210> 35
   <211> 378
   <212> DNA
   <213> Homo sapiens
<220>
   <221> CDS
   <222> (1)..(378)
<400> 35
   cag ctg cag ctg cag gag tcg ggc cca gga ctg gtg aag cct tcg gag    48
   Gln Leu Gln Leu Gln Glu Ser Gly Pro Gly Leu Val Lys Pro Ser Glu
   1               5                   10                  15
   acc ctg tcc ctc acc tgc act gtc tct ggt ggc gcc gtc agc agt ggt    96
   Thr Leu Ser Leu Thr Cys Thr Val Ser Gly Gly Ala Val Ser Ser Gly
               20                  25                  30
   agt tac tac tgg ggc tgg atc cgc cag tcc cca ggg aag ggg ctg gag    144
   Ser Tyr Tyr Trp Gly Trp Ile Arg Gln Ser Pro Gly Lys Gly Leu Glu
           35                  40                  45
   tgg att ggg aat atc ttt tat agt ggg acc acc tac aaa aac ccg tcc    192
   Trp Ile Gly Asn Ile Phe Tyr Ser Gly Thr Thr Tyr Lys Asn Pro Ser
       50                  55                  60
   ctc agg agt cga gtc acc ata tcc gta gac acg tcc aag aac cag ttc    240
   Leu Arg Ser Arg Val Thr Ile Ser Val Asp Thr Ser Lys Asn Gln Phe
   65                  70                  75                  80
   tcc ctg aag gcg aac tct gtg acc gcc gca gac acg gct gtg tat ttc     288
   Ser Leu Lys Ala Asn Ser Val Thr Ala Ala Asp Thr Ala Val Tyr Phe
                   85                  90                  95
   tgt gcg aga caa cgg gtc gct ggg tgg tat agt ccg gcg acg aac tgg     336
   Cys Ala Arg Gln Arg Val Ala Gly Trp Tyr Ser Pro Ala Thr Asn Trp
               100                 105                 110
   ttc gac ccc tgg ggc cag gga acc ctg gtc atc gtc tcc tca             378
   Phe Asp Pro Trp Gly Gln Gly Thr Leu Val Ile Val Ser Ser
           115                 120                 125
<210> 36
   <211> 126
   <212> PRT
   <213> Homo sapiens
<400> 36
   Gln Leu Gln Leu Gln Glu Ser Gly Pro Gly Leu Val Lys Pro Ser Glu
   1               5                   10                  15
   Thr Leu Ser Leu Thr Cys Thr Val Ser Gly Gly Ala Val Ser Ser Gly
               20                  25                  30
   Ser Tyr Tyr Trp Gly Trp Ile Arg Gln Ser Pro Gly Lys Gly Leu Glu
           35                  40                  45
   Trp Ile Gly Asn Ile Phe Tyr Ser Gly Thr Thr Tyr Lys Asn Pro Ser
       50                  55                  60
   Leu Arg Ser Arg Val Thr Ile Ser Val Asp Thr Ser Lys Asn Gln Phe
   65                  70                  75                  80
   Ser Leu Lys Ala Asn Ser Val Thr Ala Ala Asp Thr Ala Val Tyr Phe
                   85                  90                  95
   Cys Ala Arg Gln Arg Val Ala Gly Trp Tyr Ser Pro Ala Thr Asn Trp
               100                 105                 110
   Phe Asp Pro Trp Gly Gln Gly Thr Leu Val Ile Val Ser Ser
           115                 120                 125
<210> 37
   <211> 331
   <212> DNA
   <213> Homo sapiens
<220>
   <221> CDS
   <222> (1)..(330)
<400> 37
   cag tct gtg gtg act cag ccg ccc tca gtg tct ggg gcc cca ggg cag    48
   Gln Ser Val Val Thr Gln Pro Pro Ser Val Ser Gly Ala Pro Gly Gln
     1               5                  10                  15
   agg gtc acc atc tcc tgc act ggg agc agc tcc aac atc ggg gca ggt    96
   Arg Val Thr Ile Ser Cys Thr Gly Ser Ser Ser Asn Ile Gly Ala Gly
                    20                  25                  30
   tat gat gta cac tgg tac cag cag ctt cca gga aca gcc ccc aaa ctc   144
   Tyr Asp Val His Trp Tyr Gln Gln Leu Pro Gly Thr Ala Pro Lys Leu
                35                  40                  45
   ctc atc tat ggt aac agc aat cgg ccc tca ggg gtc cct gac cga ttc   192
   Leu Ile Tyr Gly Asn Ser Asn Arg Pro Ser Gly Val Pro Asp Arg Phe
        50                  55                  60
   tct ggc tcc aag tct ggc acc tca gcc tcc ctg gcc atc act ggg ctc   240
   Ser Gly Ser Lys Ser Gly Thr Ser Ala Ser Leu Ala Ile Thr Gly Leu
    65                  70                  75                  80
   cag gct gag gat gag gct gat tat tac tgc cag tcc tat gac agc agc   288
   Gln Ala Glu Asp Glu Ala Asp Tyr Tyr Cys Gln Ser Tyr Asp Ser Ser
                    85                  90                  95
   ctg agt ggt tca ttc ggc gga cgg acc aag ctg acc gtc cta g         331
   Leu Ser Gly Ser Phe Gly Gly Arg Thr Lys Leu Thr Val Leu
               100                 105                 110
<210> 38
   <211> 110
   <212> PRT
   <213> Homo sapiens
<400> 38
   Gln Ser Val Val Thr Gln Pro Pro Ser Val Ser Gly Ala Pro Gly Gln
     1               5                  10                  15
   Arg Val Thr Ile Ser Cys Thr Gly Ser Ser Ser Asn Ile Gly Ala Gly
                    20                  25                  30
   Tyr Asp Val His Trp Tyr Gln Gln Leu Pro Gly Thr Ala Pro Lys Leu
                35                  40                  45
   Leu Ile Tyr Gly Asn Ser Asn Arg Pro Ser Gly Val Pro Asp Arg Phe
        50                  55                  60
   Ser Gly Ser Lys Ser Gly Thr Ser Ala Ser Leu Ala Ile Thr Gly Leu
    65                  70                  75                  80
   Gln Ala Glu Asp Glu Ala Asp Tyr Tyr Cys Gln Ser Tyr Asp Ser Ser
                    85                  90                  95
   Leu Ser Gly Ser Phe Gly Gly Arg Thr Lys Leu Thr Val Leu
               100                 105                 110
<210> 39
   <211> 379
   <212> DNA
   <213> Homo sapiens
<220>
   <221> CDS
   <222> (1).. (378)
<400> 39
   gag gtg cag ctg gtg gag tct ggg gga ggc ttg gta aag cct ggg ggg     48
   Glu Val Gln Leu Val Glu Ser Gly Gly Gly Leu Val Lys Pro Gly Gly
     1               5                  10                  15
   tcc ctt aga ctc tcc tgt gca gcc tct gga ttc act ttc att aac gcc     96
   Ser Leu Arg Leu Ser Cys Ala Ala Ser Gly Phe Thr Phe Ile Asn Ala
                20                  25                  30
   tgg atg aac tgg gtc cgc cag gct cca ggg aag ggg ctg gag tgg gtt    144
   Trp Met Asn Trp Val Arg Gln Ala Pro Gly Lys Gly Leu Glu Trp Val
            35                  40                  45
   ggc cgt att aga agc aac att gat ggt ggg aca aca gac tac gct gca    192
   Gly Arg Ile Arg Ser Asn Ile Asp Gly Gly Thr Thr Asp Tyr Ala Ala
        50                  55                  60
   ccc gtg aaa ggc aga ttc acc atc tca aga gat gat tca aaa aac acg    240
   Pro Val Lys Gly Arg Phe Thr Ile Ser Arg Asp Asp Ser Lys Asn Thr
    65                  70                  75                  80
   ctg ttt ctg caa atg aac acc ctg aaa acc gag gac aca gcc gtg tat    288
   Leu Phe Leu Gln Met Asn Thr Leu Lys Thr Glu Asp Thr Ala Val Tyr
                    85                  90                  95
   tac tgt gtc aca gat ggg tat tac gag tct tgg agt ggt cct tat cct    336
   Tyr Cys Val Thr Asp Gly Tyr Tyr Glu Ser Trp Ser Gly Pro Tyr Pro
               100                 105                 110
   ttt gat gtc tgg ggc caa ggg aca atg gtc acc gtc tct tca g          379
   Phe Asp Val Trp Gly Gln Gly Thr Met Val Thr Val Ser Ser
           115                 120                 125
<210> 40
   <211> 126
   <212> PRT
   <213> Homo sapiens
<400> 40
   Glu Val Gln Leu Val Glu Ser Gly Gly Gly Leu Val Lys Pro Gly Gly
    1               5                  10                  15
   Ser Leu Arg Leu Ser Cys Ala Ala Ser Gly Phe Thr Phe Ile Asn Ala
               20                  25                  30
   Trp Met Asn Trp Val Arg Gln Ala Pro Gly Lys Gly Leu Glu Trp Val
           35                  40                  45
   Gly Arg Ile Arg Ser Asn Ile Asp Gly Gly Thr Thr Asp Tyr Ala Ala
       50                  55                  60
   Pro Val Lys Gly Arg Phe Thr Ile Ser Arg Asp Asp Ser Lys Asn Thr
   65                  70                  75                  80
   Leu Phe Leu Gln Met Asn Thr Leu Lys Thr Glu Asp Thr Ala Val Tyr
                   85                  90                  95
   Tyr Cys Val Thr Asp Gly Tyr Tyr Glu Ser Trp Ser Gly Pro Tyr Pro
               100                 105                 110
   Phe Asp Val Trp Gly Gln Gly Thr Met Val Thr Val Ser Ser
           115                 120                 125
<210> 41
   <211> 5920
   <212> DNA
   <213> Homo sapiens
<400> 41
   gatccgatgc agcaagacaa ggagcgcctg gcagccctgg ggcctgaggg ggcccatggt 60
   gacactacct ttgagtacca ggacctgtgc cgccagcaca tggccacgaa gtccttgttc 120
   aaccgggcag agggtccacc ggagccttca cgggtgagca gtgtgtcctc ccagttcagc 180
   gacgcagccc aggccagccc cagctcccac agcagcaccc cgtcctggtg cgaggagccg 240
   gcccaagcca acatggacat ctccacggga cacatgattc tggcatacat ggaggatcac 300
   ctgcggaacc gggaccgcct tgccaaggag tggcaggccc tctgtgccta ccaagcagag 360
   ccaaacacct gtgccaccgc gcagggggag ggcaacatca aaaagaaccg gcatcctgac 420
   ttcctgccct atgaccatgc ccgcataaaa ctgaaggtgg agagcagccc ttctcggagc 480
   gattacatca acgccagccc cattattgag catgaccctc ggatgccagc ctacatagcc 540
   acgcagggcc cgctgtccca taccatcgca gacttctggc agatggtgtg ggagagcggc 600
   tgcaccgtca tcgtcatgct gaccccgctg gtggaggatg gtgtcaagca gtgtgaccgc 660
   tactggccag atgagggtgc ctccctctac cacgtatatg aggtgaacct ggtgtcggag 720
   cacatctggt gcgaggactt tctggtgcgg agcttctacc tgaagaacgt gcagacccag 780
   gagacgcgca cgctcacgca gttccacttc ctcagctggc cggcagaggg cacaccggcc 840
   tccacgcggc ccctgctgga cttccgcagg aaggtgaaca agtgctaccg gggccgctcc 900
   tgccccatca tcgtgcactg cagtgatggt gcggggagga ccggcaccta catcctcatc 960
   gacatggtcc tgaaccgcat ggcaaaagga gtgaaggaga ttgacatcgc tgccaccctg 1020
   gagcatgtcc gtgaccagcg gcctggcctt gtccgctcta aggaccagtt tgaatttgcc 1080
   ctgacagccg tggcggagga agtgaatgcc atcctcaagg ccctgcccca gctcgagatg 1140
   gcatctccgg gctctggctt ttggtctttc gggtcggaag atggctctgg ggattccgag 1200
   aatcccggca cagcgcgagc ctggtgccaa gtggctcaga agttcacggg cggcatcgga 1260
   aacaaactgt gcgccctgct ctacggagac gccgagaagc cggcggagag cggcgggagc 1320
   caacccccgc gggccgccgc ccggaaggcc gcctgcgcct gcgaccagaa gccctgcagc 1380
   tgctccaaag tggatgtcaa ctacgcgttt ctccatgcaa cagacctgct gccggcgtgt 1440
   gatggagaaa ggcccacttt ggcgtttctg caagatgtta tgaacatttt acttcagtat 1500
   gtggtgaaaa gtttcgatag atcaaccaaa gtgattgatt tccattatcc taatgagctt 1560
   ctccaagaat ataattggga attggcagac caaccacaaa atttggagga aattttgatg 1620
   cattgccaaa caactctaaa atatgcaatt aaaacagggc atcctagata cttcaatcaa 1680
   ctttctactg gtttggatat ggttggatta gcagcagact ggctgacatc aacagcaaat 1740
   actaacatgt tcacctatga aattgctcca gtatttgtgc ttttggaata tgtcacacta 1800
   aagaaaatga gagaaatcat tggctggcca gggggctctg gcgatgggat attttctccc 1860
   ggtggcgcca tatctaacat gtatgccatg atgatcgcac gctttaagat gttcccagaa 1920
   gtcaaggaga aaggaatggc tgctcttccc aggctcattg ccttcacgtc tgaacatagt 1980
   catttttctc tcaagaaggg agctgcagcc ttagggattg gaacagacag cgtgattctg 2040
   attaaatgtg atgagagagg gaaaatgatt ccatctgatc ttgaaagaag gattcttgaa 2100
   gccaaacaga aagggtttgt tcctttcctc gtgagtgcca cagctggaac caccgtgtac 2160
   ggagcatttg accccctctt agctgtcgct gacatttgca aaaagtataa gatctggatg 2220
   catgtggatg cagcttgggg tgggggatta ctgatgtccc gaaaacacaa gtggaaactg 2280
   agtggcgtgg agagggccaa ctctgtgacg tggaatccac acaagatgat gggagtccct 2340
   ttgcagtgct ctgctctcct ggttagagaa gagggattga tgcagaattg caaccaaatg 2400
   catgcctcct acctctttca gcaagataaa cattatgacc tgtcctatga cactggagac 2460
   aaggccttac agtgcggacg ccacgttgat gtttttaaac tatggctgat gtggagggca 2520
   aaggggacta ccgggtttga agcgcatgtt gataaatgtt tggagttggc agagtattta 2580
   tacaacatca taaaaaaccg agaaggatat gagatggtgt ttgatgggaa gcctcagcac 2640
   acaaatgtct gcttctggta cattcctcca agcttgcgta ctctggaaga caatgaagag 2700
   agaatgagtc gcctctcgaa ggtggctcca gtgattaaag ccagaatgat ggagtatgga 2760
   accacaatgg tcagctacca acccttggga gacaaggtca atttcttccg catggtcatc 2820
   tcaaacccag cggcaactca ccaagacatt gacttcctga ttgaagaaat agaacgcctt 2880
   ggacaagatt tataataaga gctcccaaaa aaaaaaaaaa aaaaaaaaaa aaaaaaccga 2940
   attcgtaatc atggtcatag ctgtttcctg tgtgaaattg ttatccgctc acaattccac 3000
   acaacatacg agccggaagc ataaagtgta aagcctgggg tgcctaatga gtgagctaac 3060
   tcacattaat tgcgttgcgc tcactgcccg ctttccagtc gggaaacctg tcgtgccagc 3120
   tgcattaatg aatcggccaa cgcgcgggga gaggcggttt gcgtattggg cgctcttccg 3180
   cttcctcgct cactgactcg ctgcgctcgg tcgttcggct gcggcgagcg gtatcagctc 3240
   actcaaaggc ggtaatacgg ttatccacag aatcagggga taacgcagga aagaacatgt 3300
   gagcaaaagg ccagcaaaag gccaggaacc gtaaaaaggc cgcgttgctg gcgtttttcc 3360
   ataggctccg cccccctgac gagcatcaca aaaatcgacg ctcaagtcag aggtggcgaa 3420
   acccgacagg actataaaga taccaggcgt ttccccctgg aagctccctc gtgcgctctc 3480
   ctgttccgac cctgccgctt accggatacc tgtccgcctt tctcccttcg ggaagcgtgg 3540
   cgctttctca tagctcacgc tgtaggtatc tcagttcggt gtaggtcgtt cgctccaagc 3600
   tgggctgtgt gcacgaaccc cccgttcagc ccgaccgctg cgccttatcc ggtaactatc 3660
   gtcttgagtc caacccggta agacacgact tatcgccact ggcagcagcc actggtaaca 3720
   ggattagcag agcgaggtat gtaggcggtg ctacagagtt cttgaagtgg tggcctaact 3780
   acggctacac tagaaggaca gtatttggta tctgcgctct gctgaagcca gttaccttcg 3840
   gaaaaagagt tggtagctct tgatccggca aacaaaccac cgctggtagc ggtggttttt 3900
   ttgtttgcaa gcagcagatt acgcgcagaa aaaaaggatc tcaagaagat cctttgatct 3960
   tttctacggg gtctgacgct cagtggaacg aaaactcacg ttaagggatt ttggtcatga 4020
   gattatcaaa aaggatcttc acctagatcc ttttaaatta aaaatgaagt tttaaatcaa 4080
   tctaaagtat atatgagtaa acttggtctg acagttacca atgcttaatc agtgaggcac 4140
   ctatctcagc gatctgtcta tttcgttcat ccatagttgc ctgactcccc gtcgtgtaga 4200
   taactacgat acgggagggc ttaccatctg gccccagtgc tgcaatgata ccgcgagacc 4260
   cacgctcacc ggctccagat ttatcagcaa taaaccagcc agccggaagg gccgagcgca 4320
   gaagtggtcc tgcaacttta tccgcctcca tccagtctat taattgttgc cgggaagcta 4380
   gagtaagtag ttcgccagtt aatagtttgc gcaacgttgt tgccattgct acaggcatcg 4440
   tggtgtcacg ctcgtcgttt ggtatggctt cattcagctc cggttcccaa cgatcaaggc 4500
   gagttacatg atcccccatg ttgtgcaaaa aagcggttag ctccttcggt cctccgatcg 4560
   ttgtcagaag taagttggcc gcagtgttat cactcatggt tatggcagca ctgcataatt 4620
   ctcttactgt catgccatcc gtaagatgct tttctgtgac tggtgagtac tcaaccaagt 4680
   cattctgaga atagtgtatg cggcgaccga gttgctcttg cccggcgtca atacgggata 4740
   ataccgcgcc acatagcaga actttaaaag tgctcatcat tggaaaacgt tcttcggggc 4800
   gaaaactctc aaggatctta ccgctgttga gatccagttc gatgtaaccc actcgtgcac 4860
   ccaactgatc ttcagcatct tttactttca ccagcgtttc tgggtgagca aaaacaggaa 4920
   ggcaaaatgc cgcaaaaaag ggaataaggg cgacacggaa atgttgaata ctcatactct 4980
   tcctttttca atattattga agcatttatc agggttattg tctcatgagc ggatacatat 5040
   ttgaatgtat ttagaaaaat aaacaaatag gggttccgcg cacatttccc cgaaaagtgc 5100
   cacctgacgt ctaagaaacc attattatca tgacattaac ctataaaaat aggcgtatca 5160
   cgaggccctt tcgtctcgcg cgtttcggtg atgacggtga aaacctctga cacatgcagc 5220
   tcccggagac ggtcacagct tgtctgtaag cggatgccgg gagcagacaa gcccgtcagg 5280
   gcgcgtcagc gggtgttggc gggtgtcggg gctggcttaa ctatgcggca tcagagcaga 5340
   ttgtactgag agtgcaccat atcgacgctc tcccttatgc gactcctgca ttaggaagca 5400
   gcccagtagt aggttgaggc cgttgagcac cgccgccgca aggaatggtg catgcaagga 5460
   gatggcgccc aacagtcccc cggccacggg gcctgccacc atacccacgc cgaaacaagc 5520
   gctcatgagc ccgaagtggc gagcccgatc ttccccatcg gtgatgtcgg cgatataggc 5580
   gccagcaacc gcacctgtgg cgccggtgat gccggccacg atgcgtccgg cgtagaggat 5640
   ctggctagcg atgaccctgc tgattggttc gctgaccatt tccggggtgc ggaacggcgt 5700
   taccagaaac tcagaaggtt cgtccaacca aaccgactct gacggcagtt tacgagagag 5760
   atgatagggt ctgcttcagt aagccagatg ctacacaatt aggcttgtac atattgtcgt 5820
   tagaacgcgg ctacaattaa tacataacct tatgtatcat acacatacga tttaggtgac 5880
   actatagaat acaagcttgg gctgcaggtc gactctagag 5920
<210> 42
   <211> 4665
   <212> DNA
   <213> Homo sapiens
<400> 42

### SEQUENZPROTOKOLL

<110> Labor Dr. Koch - Dr. Merk
<120> Fusionsprotein zur gleichzeitigen Erkennung von humanen Autoantikörpern
<130> E 1207 DE
<140>
   <141>
<160> 42
<170> PatentIn Ver. 2.1
<210> 1
   <211> 585
   <212> PRT
   <213> Homo sapiens
<400> 1
   Met Ala Ser Pro Gly Ser Gly Phe Trp Ser Phe Gly Ser Glu Asp Gly
    1               5                  10                  15
   Ser Gly Asp Ser Glu Asn Pro Gly Thr Ala Arg Ala Trp Cys Gln Val
               20                  25                  30
   Ala Gln Lys Phe Thr Gly Gly Ile Gly Asn Lys Leu Cys Ala Leu Leu
           35                  40                  45
   Tyr Gly Asp Ala Glu Lys Pro Ala Glu Ser Gly Gly Ser Gln Pro Pro
       50                  55                  60
   Arg Ala Ala Ala Arg Lys Ala Ala Cys Ala Cys Asp Gln Lys Pro Cys
   65                  70                  75                  80
   Ser Cys Ser Lys Val Asp Val Asn Tyr Ala Phe Leu His Ala Thr Asp
                   85                  90                  95
   Leu Leu Pro Ala Cys Asp Gly Glu Arg Pro Thr Leu Ala Phe Leu Gln
               100                 105                 110
   Asp Val Met Asn Ile Leu Leu Gln Tyr Val Val Lys Ser Phe Asp Arg
           115                 120                 125
   Ser Thr Lys Val Ile Asp Phe His Tyr Pro Asn Glu Leu Leu Gln Glu
       130                 135                 140
   Tyr Asn Trp Glu Leu Ala Asp Gln Pro Gln Asn Leu Glu Glu Ile Leu
   145                 150                 155                 160
   Met His Cys Gln Thr Thr Leu Lys Tyr Ala Ile Lys Thr Gly His Pro
                   165                 170                 175
   Arg Tyr Phe Asn Gln Leu Ser Thr Gly Leu Asp Met Val Gly Leu Ala
               180                 185                 190
   Ala Asp Trp Leu Thr Ser Thr Ala Asn Thr Asn Met Phe Thr Tyr Glu
           195                 200                 205
   Ile Ala Pro Val Phe Val Leu Leu Glu Tyr Val Thr Leu Lys Lys Met
       210                 215                 220
   Arg Glu Ile Ile Gly Trp Pro Gly Gly Ser Gly Asp Gly Ile Phe Ser
   225 230 235 240
   Pro Gly Gly Ala Ile Ser Asn Met Tyr Ala Met Met Ile Ala Arg Phe
   245 250 255
   Lys Met Phe Pro Glu Val Lys Glu Lys Gly Met Ala Ala Leu Pro Arg
   260 265 270
   Leu Ile Ala Phe Thr Ser Glu His Ser His Phe Ser Leu Lys Lys Gly
   275 280 285
   Ala Ala Ala Leu Gly Ile Gly Thr Asp Ser Val Ile Leu Ile Lys Cys
   290 295 300
   Asp Glu Arg Gly Lys Met Ile Pro Ser Asp Leu Glu Arg Arg Ile Leu
   305 310 315 320
   Glu Ala Lys Gln Lys Gly Phe Val Pro Phe Leu Val Ser Ala Thr Ala
   325 330 335
   Gly Thr Thr Val Tyr Gly Ala Phe Asp Pro Leu Leu Ala Val Ala Asp
   340 345 350
   Ile Cys Lys Lys Tyr Lys Ile Trp Met His Val Asp Ala Ala Trp Gly
   355 360 365
   Gly Gly Leu Leu Met Ser Arg Lys His Lys Trp Lys Leu Ser Gly Val
   370 375 380
   Glu Arg Ala Asn Ser Val Thr Trp Asn Pro His Lys Met Met Gly Val
   385 390 395 400
   Pro Leu Gln Cys Ser Ala Leu Leu Val Arg Glu Glu Gly Leu Met Gln
   405 410 415
   Asn Cys Asn Gln Met His Ala Ser Tyr Leu Phe Gln Gln Asp Lys His
   420 425 430
   Tyr Asp Leu Ser Tyr Asp Thr Gly Asp Lys Ala Leu Gln Cys Gly Arg
   435 440 445
   His Val Asp Val Phe Lys Leu Trp Leu Met Trp Arg Ala Lys Gly Thr
   450 455 460
   Thr Gly Phe Glu Ala His Val Asp Lys Cys Leu Glu Leu Ala Glu Tyr
   465 470 475 480
   Leu Tyr Asn Ile Ile Lys Asn Arg Glu Gly Tyr Glu Met Val Phe Asp
   485 490 495
   Gly Lys Pro Gln His Thr Asn Val Cys Phe Trp Tyr Ile Pro Pro Ser
   500 505 510
   Leu Arg Thr Leu Glu Asp Asn Glu Glu Arg Met Ser Arg Leu Ser Lys
   515 520 525
   Val Ala Pro Val Ile Lys Ala Arg Met Met Glu Tyr Gly Thr Thr Met
   530 535 540
   Val Ser Tyr Gln Pro Leu Gly Asp Lys Val Asn Phe Phe Arg Met Val
   545 550 555 560
   Ile Ser Asn Pro Ala Ala Thr His Gln Asp Ile Asp Phe Leu Ile Glu
                   565                 570                 575
   Glu Ile Glu Arg Leu Gly Gln Asp Leu
               580                 585
<210> 2
   <211> 979
   <212> PRT
   <213> Homo sapiens
<400> 2
   Met Arg Arg Pro Arg Arg Pro Gly Gly Leu Gly Gly Ser Gly Gly Leu
   1               5                   10                  15
   Arg Leu Leu Leu Cys Leu Leu Leu Leu Ser Ser Arg Pro Gly Gly Cys
               20                  25                  30
   Ser Ala Val Ser Ala His Gly Cys Leu Phe Asp Arg Arg Leu Cys Ser
           35                  40                  45
   His Leu Glu Val Cys Ile Gln Asp Gly Leu Phe Gly Gln Cys Gln Val
       50                  55                  60
   Gly Val Gly Gln Ala Arg Pro Leu Leu Gln Val Thr Ser Pro Val Leu
   65                  70                  75                  80
   Gln Arg Leu Gln Gly Val Leu Arg Gln Leu Met Ser Gln Gly Leu Ser
                   85                  90                  95
   Trp His Asp Asp Leu Thr Gln Tyr Val Ile Ser Gln Glu Met Glu Arg
               100                 105                 110
   Ile Pro Arg Leu Arg Pro Pro Glu Pro Arg Pro Arg Asp Arg Ser Gly
           115                 120                 125
   Leu Ala Pro Lys Arg Pro Gly Pro Ala Gly Glu Leu Leu Leu Gln Asp
       130                 135                 140
   Ile Pro Thr Gly Ser Ala Pro Ala Ala Gln His Arg Leu Pro Gln Pro
   145                 150                 155                 160
   Pro Val Gly Lys Gly Gly Ala Gly Ala Ser Ser Ser Leu Ser Pro Leu
                   165                 170                 175
   Gln Ala Glu Leu Leu Pro Pro Leu Leu Glu His Leu Leu Leu Pro Pro
               180                 185                 190
   Gln Pro Pro His Pro Ser Leu Ser Tyr Glu Pro Ala Leu Leu Gln Pro
           195                 200                 205
   Tyr Leu Phe His Gln Phe Gly Ser Arg Asp Gly Ser Arg Val Ser Glu
       210                 215                 220
   Gly Ser Pro Gly Met Val Ser Val Gly Pro Leu Pro Lys Ala Glu Ala
   225                 230                 235                 240
   Pro Ala Leu Phe Ser Arg Thr Ala Ser Lys Gly Ile Phe Gly Asp His
                   245                 250                 255
   Pro Gly His Ser Tyr Gly Asp Leu Pro Gly Pro Ser Pro Ala Gln Leu
               260                 265                 270
   Phe Gln Asp Ser Gly Leu Leu Tyr Leu Ala Gln Glu Leu Pro Ala Pro
           275                 280                 285
   Ser Arg Ala Arg Val Pro Arg Leu Pro Glu Gln Gly Ser Ser Ser Arg
       290                 295                 300
   Ala Glu Asp Ser Pro Glu Gly Tyr Glu Lys Glu Gly Leu Gly Asp Arg
   305                 310                 315                 320
   Gly Glu Lys Pro Ala Ser Pro Ala Val Gln Pro Asp Ala Ala Leu Gln
                   325                 330                 335
   Arg Leu Ala Ala Val Leu Ala Gly Tyr Gly Val Glu Leu Arg Gln Leu
               340                 345                 350
   Thr Pro Glu Gln Leu Ser Thr Leu Leu Thr Leu Leu Gln Leu Leu Pro
           355                 360                 365
   Lys Gly Ala Gly Arg Asn Pro Gly Gly Val Val Asn Val Gly Ala Asp
       370                 375                 380
   Ile Lys Lys Thr Met Glu Gly Pro Val Glu Gly Arg Asp Thr Ala Glu
   385                 390                 395                 400
   Leu Pro Ala Arg Thr Ser Pro Met Pro Gly His Pro Thr Ala Ser Pro
                   405                 410                 415
   Thr Ser Ser Glu Val Gln Gln Val Pro Ser Pro Val Ser Ser Glu Pro
               420                 425                 430
   Pro Lys Ala Ala Arg Pro Pro Val Thr Pro Val Leu Leu Glu Lys Lys
           435                 440                 445
   Ser Pro Leu Gly Gln Ser Gln Pro Thr Val Ala Gly Gln Pro Ser Ala
       450                 455                 460
   Arg Pro Ala Ala Glu Glu Tyr Gly Tyr Ile Val Thr Asp Gln Lys Pro
   465                 470                 475                 480
   Leu Ser Leu Ala Ala Gly Val Lys Leu Leu Glu Ile Leu Ala Glu His
                   485                 490                 495
   Val His Met Ser Ser Gly Ser Phe Ile Asn Ile Ser Val Val Gly Pro
               500                 505                 510
   Ala Leu Thr Phe Arg Ile Arg His Asn Glu Gln Asn Leu Ser Leu Ala
           515                 520                 525
   Asp Val Thr Gln Gln Ala Gly Leu Val Lys Ser Glu Leu Glu Ala Gln
       530                 535                 540
   Thr Gly Leu Gln Ile Leu Gln Thr Gly Val Gly Gln Arg Glu Glu Ala
   545                 550                 555                 560
   Ala Ala Val Leu Pro Gln Thr Ala His Ser Thr Ser Pro Met Arg Ser
                   565                 570                 575
   Val Leu Leu Thr Leu Val Ala Leu Ala Gly Val Ala Gly Leu Leu Val
               580                 585                 590
   Ala Leu Ala Val Ala Leu Cys Val Arg Gln His Ala Arg Gln Gln Asp
   595 600 605
   Lys Glu Arg Leu Ala Ala Leu Gly Pro Glu Gly Ala His Gly Asp Thr
   610 615 620
   Thr Phe Glu Tyr Gln Asp Leu Cys Arg Gln His Met Ala Thr Lys Ser
   625 630 635 640
   Leu Phe Asn Arg Ala Glu Gly Pro Pro Glu Pro Ser Arg Val Ser Ser
   645 650 655
   Val Ser Ser Gln Phe Ser Asp Ala Ala Gln Ala Ser Pro Ser Ser His
   660 665 670
   Ser Ser Thr Pro Ser Trp Cys Glu Glu Pro Ala Gln Ala Asn Met Asp
   675 680 685
   Ile Ser Thr Gly His Met Ile Leu Ala Tyr Met Glu Asp His Leu Arg
   690 695 700
   Asn Arg Asp Arg Leu Ala Lys Glu Trp Gln Ala Leu Cys Ala Tyr Gln
   705 710 715 720
   Ala Glu Pro Asn Thr Cys Ala Thr Ala Gln Gly Glu Gly Asn Ile Lys
   725 730 735
   Lys Asn Arg His Pro Asp Phe Leu Pro Tyr Asp His Ala Arg Ile Lys
   740 745 750
   Leu Lys Val Glu Ser Ser Pro Ser Arg Ser Asp Tyr Ile Asn Ala Ser
   755 760 765
   Pro Ile Ile Glu His Asp Pro Arg Met Pro Ala Tyr Ile Ala Thr Gln
   770 775 780
   Gly Pro Leu Ser His Thr Ile Ala Asp Phe Trp Gln Met Val Trp Glu
   785 790 795 800
   Ser Gly Cys Thr Val Ile Val Met Leu Thr Pro Leu Val Glu Asp Gly
   805 810 815
   Val Lys Gln Cys Asp Arg Tyr Trp Pro Asp Glu Gly Ala Ser Leu Tyr
   820 825 830
   His Val Tyr Glu Val Asn Leu Val Ser Glu His Ile Trp Cys Glu Asp
   835 840 845
   Phe Leu Val Arg Ser Phe Tyr Leu Lys Asn Val Gln Thr Gln Glu Thr
   850 855 860
   Arg Thr Leu Thr Gln Phe His Phe Leu Ser Trp Pro Ala Glu Gly Thr
   865 870 875 880
   Pro Ala Ser Thr Arg Pro Leu Leu Asp Phe Arg Arg Lys Val Asn Lys
   885 890 895
   Cys Tyr Arg Gly Arg Ser Cys Pro Ile Ile Val His Cys Ser Asp Gly
   900 905 910
   Ala Gly Arg Thr Gly Thr Tyr Ile Leu Ile Asp Met Val Leu Asn Arg
   915 920 925
   Met Ala Lys Gly Val Lys Glu Ile Asp Ile Ala Ala Thr Leu Glu His
   930 935 940
   Val Arg Asp Gln Arg Pro Gly Leu Val Arg Ser Lys Asp Gln Phe Glu
   945 950 955 960
   Phe Ala Leu Thr Ala Val Ala Glu Glu Val Asn Ala Ile Leu Lys Ala
   965 970 975
   Leu Pro Gln
<210> 3
   <211> 2400
   <212> DNA
   <213> Homo sapiens
<400> 3
   agctcgcccg cagctcgcac tcgcaggcga cctgctccag tctccaaagc cgatggcatc 60
   tccgggctct ggcttttggt ctttcgggtc ggaagatggc tctggggatt ccgagaatcc 120
   cggcacagcg cgagcctggt gccaagtggc tcagaagttc acgggcggca tcggaaacaa 180
   actgtgcgcc ctgctctacg gagacgccga gaagccggcg gagagcggcg ggagccaacc 240
   cccgcgggcc gccgcccgga aggccgcctg cgcctgcgac cagaagccct gcagctgctc 300
   caaagtggat gtcaactacg cgtttctcca tgcaacagac ctgctgccgg cgtgtgatgg 360
   agaaaggccc actttggcgt ttctgcaaga tgttatgaac attttacttc agtatgtggt 420
   gaaaagtttc gatagatcaa ccaaagtgat tgatttccat tatcctaatg agcttctcca 480
   agaatataat tgggaattgg cagaccaacc acaaaatttg gaggaaattt tgatgcattg 540
   ccaaacaact ctaaaatatg caattaaaac agggcatcct agatacttca atcaactttc 600
   tactggtttg gatatggttg gattagcagc agactggctg acatcaacag caaatactaa 660
   catgttcacc tatgaaattg ctccagtatt tgtgcttttg gaatatgtca cactaaagaa 720
   aatgagagaa atcattggct ggccaggggg ctctggcgat gggatatttt ctcccggtgg 780
   cgccatatct aacatgtatg ccatgatgat cgcacgcttt aagatgttcc cagaagtcaa 840
   ggagaaagga atggctgctc ttcccaggct cattgccttc acgtctgaac atagtcattt 900
   ttctctcaag aagggagctg cagccttagg gattggaaca gacagcgtga ttctgattaa 960
   atgtgatgag agagggaaaa tgattccatc tgatcttgaa agaaggattc ttgaagccaa 1020
   acagaaaggg tttgttcctt tcctcgtgag tgccacagct ggaaccaccg tgtacggagc 1080
   atttgacccc ctcttagctg tcgctgacat ttgcaaaaag tataagatct ggatgcatgt 1140
   ggatgcagct tggggtgggg gattactgat gtcccgaaaa cacaagtgga aactgagtgg 1200
   cgtggagagg gccaactctg tgacgtggaa tccacacaag atgatgggag tccctttgca 1260
   gtgctctgct ctcctggtta gagaagaggg attgatgcag aattgcaacc aaatgcatgc 1320
   ctcctacctc tttcagcaag ataaacatta tgacctgtcc tatgacactg gagacaaggc 1380
   cttacagtgc ggacgccacg ttgatgtttt taaactatgg ctgatgtgga gggcaaaggg 1440
   gactaccggg tttgaagcgc atgttgataa atgtttggag ttggcagagt atttatacaa 1500
   catcataaaa aaccgagaag gatatgagat ggtgtttgat gggaagcctc agcacacaaa 1560
   tgtctgcttc tggtacattc ctccaagctt gcgtactctg gaagacaatg aagagagaat 1620
   gagtcgcctc tcgaaggtgg ctccagtgat taaagccaga atgatggagt atggaaccac 1680
   aatggtcagc taccaaccct tgggagacaa ggtcaatttc ttccgcatgg tcatctcaaa 1740
   cccagcggca actcaccaag acattgactt cctgattgaa gaaatagaac gccttggaca 1800
   agatttataa taaccttgct caccaagctg ttccacttct ctaggtagac aattaagttg 1860
   tcacaaactg tgtgaatgta tttgtagttt gttccaaagt aaatctattt ctatattgtg 1920
   gtgtcaaagt agagtttaaa aattaaacaa aaaagacatt gctcctttta aaagtccttt 1980
   cttaagttta gaatacctct ctaagaattc gtgacaaaag gctatgttct aatcaataag 2040
   gaaaagctta aaattgttat aaatacttcc cttactttta atatagtgtg caaagcaaac 2100
   tttattttca cttcagacta gtaggactga atagtgccaa attgcccctg aatcataaaa 2160
   ggttctttgg ggtgcagtaa aaaggacaaa gtaaatataa aatatatgtt gacaataaaa 2220
   actcttgcct ttttcatagt attagaaaaa aatttctaat ttacctatag caacatttca 2280
   aatgtattta aatacatata attttacaaa aggaaaatat atatattaaa aaagatatcc 2340
   tattttgtaa catatagatt tttattttat ataggttata caaactgcgg gggcggaatt 2400
<210> 4
   <211> 3613
   <212> DNA
   <213> Homo sapiens
<400> 4
   cagcccctct ggcaggctcc cgccagcgtc gctgcggctc cggcccggga gcgagcgccc 60
   ggagctcgga aagatgcggc gcccgcggcg gcctgggggt ctcgggggat ccgggggtct 120
   ccggctgctc ctctgcctcc tgctgctgag cagccgcccg gggggctgca gcgccgttag 180
   tgcccacggc tgtctatttg accgcaggct ctgctctcac ctggaagtct gtattcagga 240
   tggcttgttt gggcagtgcc aggtgggagt ggggcaggcc cggccccttt tgcaagtcac 300
   ctccccagtt ctccaacgct tacaaggtgt gctccgacaa ctcatgtccc aaggattgtc 360
   ctggcacgat gacctcaccc agtatgtgat ctctcaggag atggagcgca tccccaggct 420
   tcgcccccca gagccccgtc caagggacag gtctggcttg gcacccaaga gacctggtcc 480
   tgctggagag ctgcttttac aggacatccc cactggctcc gcccctgctg cccagcatcg 540
   gcttccacaa ccaccagtgg gcaaaggtgg agctggggcc agctcctctc tgtcccctct 600
   gcaggctgag ctgctcccgc ctctcttgga gcacctgctg ctgcccccac agcctcccca 660
   cccttcactg agttacgaac ctgccttgct gcagccctac ctgttccacc agtttggctc 720
   ccgtgatggc tccagggtct cagagggctc cccagggatg gtcagtgtcg gccccctgcc 780
   caaggctgaa gcccctgccc tcttcagcag aactgcctcc aagggcatat ttggggacca 840
   ccctggccac tcctacgggg accttccagg gccttcacct gcccagcttt ttcaagactc 900
   tgggctgctc tatctggccc aggagttgcc agcacccagc agggccaggg tgccaaggct 960
   gccagagcaa gggagcagca gccgggcaga ggactcccca gagggctatg agaaggaagg 1020
   actaggggat cgtggagaga agcctgcttc cccagctgtg cagccagatg cggctctgca 1080
   gaggctggcc gctgtgctgg cgggctatgg ggtagagctg cgtcagctga cccctgagca 1140
   gctctccaca ctcctgaccc tgctgcagct actgcccaag ggtgcaggaa gaaatccggg 1200
   aggggttgta aatgttggag ctgatatcaa gaaaacaatg gaggggccgg tggagggcag 1260
   agacacagca gagcttccag cccgcacatc ccccatgcct ggacacccca ctgccagccc 1320
   tacctccagt gaagtccagc aggtgccaag ccctgtctcc tctgagcctc ccaaagctgc 1380
   cagaccccct gtgacacctg tcctgctaga gaagaaaagc ccactgggcc agagccagcc 1440
   cacggtggca ggacagccct cagcccgccc agcagcagag gaatatggct acatcgtcac 1500
   tgatcagaag cccctgagcc tggctgcagg agtgaagctg ctggagatcc tggctgagca 1560
   tgtgcacatg tcctcaggca gcttcatcaa catcagtgtg gtgggaccag ccctcacctt 1620
   ccgcatccgg cacaatgagc agaacctgtc tttggctgat gtgacccaac aagcagggct 1680
   ggtgaagtct gaactggaag cacagacagg gctccaaatc ttgcagacag gagtgggaca 1740
   gagggaggag gcagctgcag tccttcccca aactgcgcac agcacctcac ccatgcgctc 1800
   agtgctgctc actctggtgg ccctggcagg tgtggctggg ctgctggtgg ctctggctgt 1860
   ggctctgtgt gtgcggcagc atgcgcggca gcaagacaag gagcgcctgg cagccctggg 1920
   gcctgagggg gcccatggtg acactacctt tgagtaccag gacctgtgcc gccagcacat 1980
   ggccacgaag tccttgttca accgggcaga gggtccaccg gagccttcac gggtgagcag 2040
   tgtgtcctcc cagttcagcg acgcagccca ggccagcccc agctcccaca gcagcacccc 2100
   gtcctggtgc gaggagccgg cccaagccaa catggacatc tccacgggac acatgattct 2160
   ggcatacatg gaggatcacc tgcggaaccg ggaccgcctt gccaaggagt ggcaggccct 2220
   ctgtgcctac caagcagagc caaacacctg tgccaccgcg cagggggagg gcaacatcaa 2280
   aaagaaccgg catcctgact tcctgcccta tgaccatgcc cgcataaaac tgaaggtgga 2340
   gagcagccct tctcggagcg attacatcaa cgccagcccc attattgagc atgaccctcg 2400
   gatgccagcc tacatagcca cgcagggccc gctgtcccat accatcgcag acttctggca 2460
   gatggtgtgg gagagcggct gcaccgtcat cgtcatgctg accccgctgg tggaggatgg 2520
   tgtcaagcag tgtgaccgct actggccaga tgagggtgcc tccctctacc acgtatatga 2580
   ggtgaacctg gtgtcggagc acatctggtg cgaggacttt ctggtgcgga gcttctacct 2640
   gaagaacgtg cagacccagg agacgcgcac gctcacgcag ttccacttcc tcagctggcc 2700
   ggcagagggc acaccggcct ccacgcggcc cctgctggac ttccgcagga aggtgaacaa 2760
   gtgctaccgg ggccgctcct gccccatcat cgtgcactgc agtgatggtg cggggaggac 2820
   cggcacctac atcctcatcg acatggtcct gaaccgcatg gcaaaaggag tgaaggagat 2880
   tgacatcgct gccaccctgg agcatgtccg tgaccagcgg cctggccttg tccgctctaa 2940
   ggaccagttt gaatttgccc tgacagccgt ggcggaggaa gtgaatgcca tcctcaaggc 3000
   cctgccccag tgagaccctg gggccccttg gcgggcagcc cagcctctgt ccctctttgc 3060
   ctgtgtgagc atctctgtgt acccactcct cactgcccca ccagccacct cttgggcatg 3120
   ctcagccctt cctagaagag tcaggaaggg aaagccagaa ggggcacgcc tgcccagcct 3180
   cgcatgccag agcctggggc atcccagagc ccagggcatc ccatgggggt gctgcagcca 3240
   ggaggagagg aaaggacatg ggtagcaatt ctacccagag ccttctcctg cctacattcc 3300
   ctggcctggc tctcctgtag ctctcctggg gttctgggag ttccctgaac atctgtgtgt 3360
   gtccccctat gctccagtat ggaagaatgg ggtggagggt cgccacaccc ggctccccct 3420
   gcttctcagc cccgggcctg cctctgactc acacttgggc gctctgccct ccctggcctc 3480
   acgcccagcc tggtcccacc accctcccac catgcgctgc tcaacctctc tccttctggc 3540
   gcaagagaac atttctagaa aaaactactt ttgtaccagt gtgaataaag ttagtgtgtt 3600
   gtctgtgcag ctg                                                        3613
<210> 5
   <211> 351
   <212> DNA
   <213> Homo sapiens
<220>
   <221> CDS
   <222> (1)..(351)
<400> 5
   tgc aca ggg tcc tgg gcc gag ctc gtg gtg act cag cct gcc tcc gtg    48
   Cys Thr Gly Ser Trp Ala Glu Leu Val Val Thr Gln Pro Ala Ser Val
     1               5                  10                  15
   tct ggg tct cct gga cag tcg atc acc atc tcc tgc act gga acc agc    96
   Ser Gly Ser Pro Gly Gln Ser Ile Thr Ile Ser Cys Thr Gly Thr Ser
                20                  25                  30
   agt gac gtt ggt ggt tat aac tat gtc tcc tgg tac cga cat cac cca   144
   Ser Asp Val Gly Gly Tyr Asn Tyr Val Ser Trp Tyr Arg His His Pro
            35                  40                  45
   gcc aaa gcc ccc aaa ctc atg att tat gat gtc agt aat cgg ccc tca   192
   Ala Lys Ala Pro Lys Leu Met Ile Tyr Asp Val Ser Asn Arg Pro Ser
        50                  55                  60
   ggg gtt tct aat cgc ttc tct ggc tcc aag tct ggc aac acg gcc tcc   240
   Gly Val Ser Asn Arg Phe Ser Gly Ser Lys Ser Gly Asn Thr Ala Ser
    65                  70                  75                  80
   ctg acc atc tct ggg ctc cag gct gag gac gag gct gat tat tac tgc   288
   Leu Thr Ile Ser Gly Leu Gln Ala Glu Asp Glu Ala Asp Tyr Tyr Cys
                    85                  90                  95
   agc tca tat aca agc agc agc act ctg gta ttc ggc gga ggg acc aag   336
   Ser Ser Tyr Thr Ser Ser Ser Thr Leu Val Phe Gly Gly Gly Thr Lys
               100                 105                 110
   ctg acc gtc cta ggt                                               351
   Leu Thr Val Leu Gly
           115
<210> 6
   <211> 117
   <212> PRT
   <213> Homo sapiens
<400> 6
   Cys Thr Gly Ser Trp Ala Glu Leu Val Val Thr Gln Pro Ala Ser Val
    1               5                  10                  15
   Ser Gly Ser Pro Gly Gln Ser Ile Thr Ile Ser Cys Thr Gly Thr Ser
               20                  25                  30
   Ser Asp Val Gly Gly Tyr Asn Tyr Val Ser Trp Tyr Arg His His Pro
           35                  40                  45
   Ala Lys Ala Pro Lys Leu Met Ile Tyr Asp Val Ser Asn Arg Pro Ser
       50                  55                  60
   Gly Val Ser Asn Arg Phe Ser Gly Ser Lys Ser Gly Asn Thr Ala Ser
   65                  70                  75                  80
   Leu Thr Ile Ser Gly Leu Gln Ala Glu Asp Glu Ala Asp Tyr Tyr Cys
                   85                  90                  95
   Ser Ser Tyr Thr Ser Ser Ser Thr Leu Val Phe Gly Gly Gly Thr Lys
               100                 105                 110
   Leu Thr Val Leu Gly
           115
<210> 7
   <211> 369
   <212> DNA
   <213> Homo sapiens
<220>
   <221> CDS
   <222> (1)..(369)
<400> 7
   nag gtg cag ctg ctc gag tcg ggc cca gga ctg gtg aag cct tcg gag    48
   Xaa Val Gln Leu Leu Glu Ser Gly Pro Gly Leu Val Lys Pro Ser Glu
    1               5                  10                  15
   acc ctg tcc ctc acc tgc act gtc tct ggt ggg tcc atc aat agt tac    96
   Thr Leu Ser Leu Thr Cys Thr Val Ser Gly Gly Ser Ile Asn Ser Tyr
               20                  25                  30
   tac tgg agc tgg atc cgg cag ccc cca ggg aaa gga ctg gag tgg att    144
   Tyr Trp Ser Trp Ile Arg Gln Pro Pro Gly Lys Gly Leu Glu Trp Ile
           35                  40                  45
   ggg ttc atc tct cac agt ggg atc acc aac tac aac ccg tcc cgc aag    192
   Gly Phe Ile Ser His Ser Gly Ile Thr Asn Tyr Asn Pro Ser Arg Lys
       50                  55                  60
   agt cga gtc acc ata tta gta gac acg tcc aag aac cag atc tcc ctg    240
   Ser Arg Val Thr Ile Leu Val Asp Thr Ser Lys Asn Gln Ile Ser Leu
   65                  70                  75                  80
   aag ctg agc tct gtg acc gct ggg gac acg gcc gtg tat tac tgt gcg    288
   Lys Leu Ser Ser Val Thr Ala Gly Asp Thr Ala Val Tyr Tyr Cys Ala
                   85                  90                  95
   aga gag ggg aaa gta tcc agt ggc tgg cga ctc tgg tac ttc gat ctc    336
   Arg Glu Gly Lys Val Ser Ser Gly Trp Arg Leu Trp Tyr Phe Asp Leu
               100                 105                 110
   tgg ggc cgt ggc acc ctg gtc act gtc tcc tca                        369
   Trp Gly Arg Gly Thr Leu Val Thr Val Ser Ser
           115                 120
<210> 8
   <211> 123
   <212> PRT
   <213> Homo sapiens
<400> 8
   Xaa Val Gln Leu Leu Glu Ser Gly Pro Gly Leu Val Lys Pro Ser Glu
   1               5                   10                  15
   Thr Leu Ser Leu Thr Cys Thr Val Ser Gly Gly Ser Ile Asn Ser Tyr
               20                  25                  30
   Tyr Trp Ser Trp Ile Arg Gln Pro Pro Gly Lys Gly Leu Glu Trp Ile
           35                  40                  45
   Gly Phe Ile Ser His Ser Gly Ile Thr Asn Tyr Asn Pro Ser Arg Lys
       50                  55                  60
   Ser Arg Val Thr Ile Leu Val Asp Thr Ser Lys Asn Gln Ile Ser Leu
   65                  70                  75                  80
   Lys Leu Ser Ser Val Thr Ala Gly Asp Thr Ala Val Tyr Tyr Cys Ala
                   85                  90                  95
   Arg Glu Gly Lys Val Ser Ser Gly Trp Arg Leu Trp Tyr Phe Asp Leu
               100                 105                 110
   Trp Gly Arg Gly Thr Leu Val Thr Val Ser Ser
           115                 120
<210> 9
   <211> 354
   <212> DNA
   <213> Homo sapiens
<220>
   <221> CDS
   <222> (1)..(354)
<400> 9
   tgc aca ggg tcc tgg gcc gag ctc gtg gtg act cag cca ccc tca gcg    48
   Cys Thr Gly Ser Trp Ala Glu Leu Val Val Thr Gln Pro Pro Ser Ala
    1               5                  10                  15
   tct ggg acc ccc ggg cag agg gtc acc atc tct tgt tct gga ggc aac    96
   Ser Gly Thr Pro Gly Gln Arg Val Thr Ile Ser Cys Ser Gly Gly Asn
               20                  25                  30
   tcc aac atc gga agt aat act gta aac tgg tac cag cag ctc cca gga    144
   Ser Asn Ile Gly Ser Asn Thr Val Asn Trp Tyr Gln Gln Leu Pro Gly
           35                  40                  45
   acg gcc ccc aag ctc ctc atc tat agt aat aat cag cgg ccc tca ggg    192
   Thr Ala Pro Lys Leu Leu Ile Tyr Ser Asn Asn Gln Arg Pro Ser Gly
       50                  55                  60
   gtc cct gac cga ttc tct ggc tcc aag tct ggc acc tca gcc tcc ctg    240
   Val Pro Asp Arg Phe Ser Gly Ser Lys Ser Gly Thr Ser Ala Ser Leu
    65                  70                  75                  80
   gcc atc agt ggg ctc cag tct gag gat gag gct gat tat tac tgt gca    288
   Ala Ile Ser Gly Leu Gln Ser Glu Asp Glu Ala Asp Tyr Tyr Cys Ala
                    85                  90                  95
   aca tgg gat gac agc ctg aat gcc tat gtg gta ttc ggc gga ggg acc    336
   Thr Trp Asp Asp Ser Leu Asn Ala Tyr Val Val Phe Gly Gly Gly Thr
               100                 105                 110
   aaa ctg acc gtc cta ggt                                            354
   Lys Leu Thr Val Leu Gly
           115
<210> 10
   <211> 118
   <212> PRT
   <213> Homo sapiens
<400> 10
   Cys Thr Gly Ser Trp Ala Glu Leu Val Val Thr Gln Pro Pro Ser Ala
    1               5                  10                  15
   Ser Gly Thr Pro Gly Gln Arg Val Thr Ile Ser Cys Ser Gly Gly Asn
               20                  25                  30
   Ser Asn Ile Gly Ser Asn Thr Val Asn Trp Tyr Gln Gln Leu Pro Gly
           35                  40                  45
   Thr Ala Pro Lys Leu Leu Ile Tyr Ser Asn Asn Gln Arg Pro Ser Gly
       50                  55                  60
   Val Pro Asp Arg Phe Ser Gly Ser Lys Ser Gly Thr Ser Ala Ser Leu
   65                  70                  75                  80
   Ala Ile Ser Gly Leu Gln Ser Glu Asp Glu Ala Asp Tyr Tyr Cys Ala
                   85                  90                  95
   Thr Trp Asp Asp Ser Leu Asn Ala Tyr Val Val Phe Gly Gly Gly Thr
               100                 105                 110
   Lys Leu Thr Val Leu Gly
           115
<210> 11
   <211> 351
   <212> DNA
   <213> Homo sapiens
<220>
   <221> CDS
   <222> (1)..(351)
<400> 11
   gtg caa ctg ctc gag tcg ggc cca gga ctg gtg aag cct tcg gag acc    48
   Val Gln Leu Leu Glu Ser Gly Pro Gly Leu Val Lys Pro Ser Glu Thr
    1               5                  10                  15
   ctg tcc ctc act tgc act gtc tct ggt ggc tcc atc act tct tac tac    96
   Leu Ser Leu Thr Cys Thr Val Ser Gly Gly Ser Ile Thr Ser Tyr Tyr
               20                  25                  30
   tgg agc tgg atc cgg cag ccc gcc ggg aag ggg ctg gac ttt att ggg    144
   Trp Ser Trp Ile Arg Gln Pro Ala Gly Lys Gly Leu Asp Phe Ile Gly
           35                  40                  45
   cgt gtc aac atc gct ggg agc acc aac tac aac ccc tcc ctc acg agt    192
   Arg Val Asn Ile Ala Gly Ser Thr Asn Tyr Asn Pro Ser Leu Thr Ser
        50                   55                   60
   cga gtc tcc atg tca gtg gac acg gcc aag aag cac ttc tcc ctg aga      240
   Arg Val Ser Met Ser Val Asp Thr Ala Lys Lys His Phe Ser Leu Arg
    65                  70                  75                  80
   ctg act tct gtg acc gcc gcg gac acg gcc gtt tac tac tgt gcg att      288
   Leu Thr Ser Val Thr Ala Ala Asp Thr Ala Val Tyr Tyr Cys Ala Ile
                    85                  90                  95
   tct ggt cgc tcc act ctg tta cct caa tac tgg ggc cag gga acc ctg      336
   Ser Gly Arg Ser Thr Leu Leu Pro Gln Tyr Trp Gly Gln Gly Thr Leu
               100                 105                 110
   gtc acc gtc tct tca                                                  351
   Val Thr Val Ser Ser
           115
<210> 12
   <211> 117
   <212> PRT
   <213> Homo sapiens
<400> 12
   Val Gln Leu Leu Glu Ser Gly Pro Gly Leu Val Lys Pro Ser Glu Thr
     1               5                  10                  15
   Leu Ser Leu Thr Cys Thr Val Ser Gly Gly Ser Ile Thr Ser Tyr Tyr
                20                  25                  30
   Trp Ser Trp Ile Arg Gln Pro Ala Gly Lys Gly Leu Asp Phe Ile Gly
            35                  40                  45
   Arg Val Asn Ile Ala Gly Ser Thr Asn Tyr Asn Pro Ser Leu Thr Ser
        50                  55                  60
   Arg Val Ser Met Ser Val Asp Thr Ala Lys Lys His Phe Ser Leu Arg
    65                  70                  75                  80
   Leu Thr Ser Val Thr Ala Ala Asp Thr Ala Val Tyr Tyr Cys Ala Ile
                    85                  90                  95
   Ser Gly Arg Ser Thr Leu Leu Pro Gln Tyr Trp Gly Gln Gly Thr Leu
               100                 105                 110
   Val Thr Val Ser Ser
           115
<210> 13
   <211> 351
   <212> DNA
   <213> Homo sapiens
<220>
   <221> CDS
   <222> (1)..(351)
<400> 13
   tgc aca ggg tcc tgg gcc gag ctc gtg gtg act cag cct ccc tcc gtg     48
   Cys Thr Gly Ser Trp Ala Glu Leu Val Val Thr Gln Pro Pro Ser Val
     1               5                  10                  15
   tcc ggg tct cct gga cag tca gtc acc atc tcc tgc act gga acc agc      96
   Ser Gly Ser Pro Gly Gln Ser Val Thr Ile Ser Cys Thr Gly Thr Ser
                20                  25                  30
   agt gac gtt ggt ggt tat aac cgt gtc tcc tgg tac cag cag ccc cca     144
   Ser Asp Val Gly Gly Tyr Asn Arg Val Ser Trp Tyr Gln Gln Pro Pro
            35                  40                  45
   ggc aca gcc ccc aaa ctc atg att tat gat gtc agt tat cgg ccc tca     192
   Gly Thr Ala Pro Lys Leu Met Ile Tyr Asp Val Ser Tyr Arg Pro Ser
        50                  55                  60
   ggg gtc cct gat cgc ttc tct ggg tcc aag tct ggc aac acg gcc tcc     240
   Gly Val Pro Asp Arg Phe Ser Gly Ser Lys Ser Gly Asn Thr Ala Ser
    65                  70                  75                  80
   ctg acc atc tct ggg ctc cag act gag gac gag gct gat tat tac tgc     288
   Leu Thr Ile Ser Gly Leu Gln Thr Glu Asp Glu Ala Asp Tyr Tyr Cys
                    85                  90                  95
   agc tca tat aca agc agc agt ctt tat gtc ttc gga act ggg acc aag     336
   Ser Ser Tyr Thr Ser Ser Ser Leu Tyr Val Phe Gly Thr Gly Thr Lys
               100                 105                 110
   gtc acc gtc cta ggt                                                 351
   Val Thr Val Leu Gly
           115
<210> 14
   <211> 117
   <212> PRT
   <213> Homo sapiens
<400> 14
   Cys Thr Gly Ser Trp Ala Glu Leu Val Val Thr Gln Pro Pro Ser Val
   1               5                   10                  15
   Ser Gly Ser Pro Gly Gln Ser Val Thr Ile Ser Cys Thr Gly Thr Ser
               20                  25                  30
   Ser Asp Val Gly Gly Tyr Asn Arg Val Ser Trp Tyr Gln Gln Pro Pro
           35                  40                  45
   Gly Thr Ala Pro Lys Leu Met Ile Tyr Asp Val Ser Tyr Arg Pro Ser
       50                  55                  60
   Gly Val Pro Asp Arg Phe Ser Gly Ser Lys Ser Gly Asn Thr Ala Ser
   65                  70                  75                  80
   Leu Thr Ile Ser Gly Leu Gln Thr Glu Asp Glu Ala Asp Tyr Tyr Cys
                   85                  90                  95
   Ser Ser Tyr Thr Ser Ser Ser Leu Tyr Val Phe Gly Thr Gly Thr Lys
               100                 105                 110
   Val Thr Val Leu Gly
           115
<210> 15
   <211> 366
   <212> DNA
   <213> Homo sapiens
<220>
   <221> CDS
   <222> (1)..(366)
<400> 15
   nag gtg cag ctg ctc gag tct ggg gct gag gtg aag aag cct ggg gcc    48
   Xaa Val Gln Leu Leu Glu Ser Gly Ala Glu Val Lys Lys Pro Gly Ala
    1               5                  10                  15
   tca gtg aag gtt tcc tgc aag gcc tct gga tac acc ttc acc aac tac    96
   Ser Val Lys Val Ser Cys Lys Ala Ser Gly Tyr Thr Phe Thr Asn Tyr
               20                  25                  30
   tat ata tac tgg gtg cga cag gcc cct gga caa ggg ctt gag tgg atg    144
   Tyr Ile Tyr Trp Val Arg Gln Ala Pro Gly Gln Gly Leu Glu Trp Met
           35                  40                  45
   gga ata atc aat cct agt ggt ggg agc aca agc tac gca gag gag ttc    192
   Gly Ile Ile Asn Pro Ser Gly Gly Ser Thr Ser Tyr Ala Glu Glu Phe
       50                  55                  60
   cag ggc aga gtc acc ttg acc agg gac acg tcc acg agc aca gtc tac    240
   Gln Gly Arg Val Thr Leu Thr Arg Asp Thr Ser Thr Ser Thr Val Tyr
   65                  70                  75                  80
   atg gaa ctg agc agc ctg aga tct gag gac acg gcc ctc tat tac tgt    288
   Met Glu Leu Ser Ser Leu Arg Ser Glu Asp Thr Ala Leu Tyr Tyr Cys
                   85                  90                  95
   gcg aga gac ttt caa gcg tac tac agg tat ggt tat ctc tac gcc tgg    336
   Ala Arg Asp Phe Gln Ala Tyr Tyr Arg Tyr Gly Tyr Leu Tyr Ala Trp
               100                 105                 110
   ggc cag gga acc ttg tcc tcc gtc tcc tca                            366
   Gly Gln Gly Thr Leu Ser Ser Val Ser Ser
           115                 120
<210> 16
   <211> 122
   <212> PRT
   <213> Homo sapiens
<400> 16
   Xaa Val Gln Leu Leu Glu Ser Gly Ala Glu Val Lys Lys Pro Gly Ala
    1               5                  10                  15
   Ser Val Lys Val Ser Cys Lys Ala Ser Gly Tyr Thr Phe Thr Asn Tyr
               20                  25                  30
   Tyr Ile Tyr Trp Val Arg Gln Ala Pro Gly Gln Gly Leu Glu Trp Met
           35                  40                  45
   Gly Ile Ile Asn Pro Ser Gly Gly Ser Thr Ser Tyr Ala Glu Glu Phe
       50                  55                  60
   Gln Gly Arg Val Thr Leu Thr Arg Asp Thr Ser Thr Ser Thr Val Tyr
   65                  70                  75                  80
   Met Glu Leu Ser Ser Leu Arg Ser Glu Asp Thr Ala Leu Tyr Tyr Cys
                   85                  90                  95
   Ala Arg Asp Phe Gln Ala Tyr Tyr Arg Tyr Gly Tyr Leu Tyr Ala Trp
               100                 105                 110
   Gly Gln Gly Thr Leu Ser Ser Val Ser Ser
           115                 120
<210> 17
   <211> 336
   <212> DNA
   <213> Homo sapiens
<220>
   <221> CDS
   <222> (1)..(336)
<400> 17
   gcc aga tgt gag ctc gtg atg acc cag tct cca gcc acc ctg tct gtg     48
   Ala Arg Cys Glu Leu Val Met Thr Gln Ser Pro Ala Thr Leu Ser Val
   1               5                   10                  15
   tct cca ggg gaa aga gcc acc ctc tcc tgc agg gcc agt cag agt gtt     96
   Ser Pro Gly Glu Arg Ala Thr Leu Ser Cys Arg Ala Ser Gln Ser Val
               20                  25                  30
   aac agc aac tta gcc tgg tac cag cag aaa cct ggc cag gct ccc agg     144
   Asn Ser Asn Leu Ala Trp Tyr Gln Gln Lys Pro Gly Gln Ala Pro Arg
           35                  40                  45
   ctc ctc atc tat aat aca tcc acc agg gcc act ggt atc cca gcc agg     192
   Leu Leu Ile Tyr Asn Thr Ser Thr Arg Ala Thr Gly Ile Pro Ala Arg
       50                  55                  60
   ttc agt ggc agt ggg tct ggg aca gag ttc act ctc acc atc agc agc     240
   Phe Ser Gly Ser Gly Ser Gly Thr Glu Phe Thr Leu Thr Ile Ser Ser
   65                  70                  75                  80
   ctg cag tct gaa gat ttt gca ctt tat tac tgt cag cag tat aat aac     288
   Leu Gln Ser Glu Asp Phe Ala Leu Tyr Tyr Cys Gln Gln Tyr Asn Asn
                   85                  90                  95
   tgg cct ccg agg gcc act ttc ggc gga ggg acc aag gtg gag atc aaa     336
   Trp Pro Pro Arg Ala Thr Phe Gly Gly Gly Thr Lys Val Glu Ile Lys
               100                 105                 110
<210> 18
   <211> 112
   <212> PRT
   <213> Homo sapiens
<400> 18
   Ala Arg Cys Glu Leu Val Met Thr Gln Ser Pro Ala Thr Leu Ser Val
    1               5                  10                  15
   Ser Pro Gly Glu Arg Ala Thr Leu Ser Cys Arg Ala Ser Gln Ser Val
               20                  25                  30
   Asn Ser Asn Leu Ala Trp Tyr Gln Gln Lys Pro Gly Gln Ala Pro Arg
           35                  40                  45
   Leu Leu Ile Tyr Asn Thr Ser Thr Arg Ala Thr Gly Ile Pro Ala Arg
       50                  55                  60
   Phe Ser Gly Ser Gly Ser Gly Thr Glu Phe Thr Leu Thr Ile Ser Ser
   65                  70                  75                  80
   Leu Gln Ser Glu Asp Phe Ala Leu Tyr Tyr Cys Gln Gln Tyr Asn Asn
                   85                  90                  95
   Trp Pro Pro Arg Ala Thr Phe Gly Gly Gly Thr Lys Val Glu Ile Lys
               100                 105                 110
<210> 19
   <211> 354
   <212> DNA
   <213> Homo sapiens
<220>
   <221> CDS
   <222> (1)..(354)
<400> 19
   nag gtg cag ctg ctc gag tcg gga gca gag gtg aaa aag ccc ggg gag    48
   Xaa Val Gln Leu Leu Glu Ser Gly Ala Glu Val Lys Lys Pro Gly Glu
    1               5                  10                  15
   tct ctg aag atc tcc tgt aag gct tct gga tac agg ctt gcc agc tat    96
   Ser Leu Lys Ile Ser Cys Lys Ala Ser Gly Tyr Arg Leu Ala Ser Tyr
               20                  25                  30
   tgg atc gcc tgg gtg cgc cag atg ccc ggg aaa ggc ctg gag tgg atg   144
   Trp Ile Ala Trp Val Arg Gln Met Pro Gly Lys Gly Leu Glu Trp Met
           35                  40                  45
   ggg atc atc tat cct gat gac tct gat acc aca tat agc ccg tcc ttc   192
   Gly Ile Ile Tyr Pro Asp Asp Ser Asp Thr Thr Tyr Ser Pro Ser Phe
       50                  55                  60
   caa ggc cat gtc acc atc tca gcc gac aag tcc atc aac acc gcc tac   240
   Gln Gly His Val Thr Ile Ser Ala Asp Lys Ser Ile Asn Thr Ala Tyr
   65                  70                  75                  80
   ctg cag tgg agc agc ctg agg gcc tcg gac acc gcc ata tat tac tgt   288
   Leu Gln Trp Ser Ser Leu Arg Ala Ser Asp Thr Ala Ile Tyr Tyr Cys
                   85                  90                  95
   gcg aga ctc gag ggt tta ggg tgg ttc gac ccc tgg ggc cag gga acc   336
   Ala Arg Leu Glu Gly Leu Gly Trp Phe Asp Pro Trp Gly Gln Gly Thr
               100                 105                 110
   ctg gtc acc gtc tcc tca                                           354
   Leu Val Thr Val Ser Ser
           115
<210> 20
   <211> 118
   <212> PRT
   <213> Homo sapiens
<400> 20
   Xaa Val Gln Leu Leu Glu Ser Gly Ala Glu Val Lys Lys Pro Gly Glu
   1               5                   10                  15
   Ser Leu Lys Ile Ser Cys Lys Ala Ser Gly Tyr Arg Leu Ala Ser Tyr
               20                  25                  30
   Trp Ile Ala Trp Val Arg Gln Met Pro Gly Lys Gly Leu Glu Trp Met
           35                  40                  45
   Gly Ile Ile Tyr Pro Asp Asp Ser Asp Thr Thr Tyr Ser Pro Ser Phe
       50                  55                  60
   Gln Gly His Val Thr Ile Ser Ala Asp Lys Ser Ile Asn Thr Ala Tyr
   65                  70                  75                  80
   Leu Gln Trp Ser Ser Leu Arg Ala Ser Asp Thr Ala Ile Tyr Tyr Cys
                   85                  90                  95
   Ala Arg Leu Glu Gly Leu Gly Trp Phe Asp Pro Trp Gly Gln Gly Thr
               100                 105                 110
   Leu Val Thr Val Ser Ser
           115
<210> 21
   <211> 345
   <212> DNA
   <213> Homo sapiens
<220>
   <221> CD5
   <222> (1)..(345)
<400> 21
   gcc aga tgt gag ctc gtg atg acc cag tct cca ctc tcc ctg ccc gtc     48
   Ala Arg Cys Glu Leu Val Met Thr Gln Ser Pro Leu Ser Leu Pro Val
    1               5                  10                  15
   acc ctt gga cag ccg gcc tcc atc tcc tgc agg tct agt caa ggc ctc     96
   Thr Leu Gly Gln Pro Ala Ser Ile Ser Cys Arg Ser Ser Gln Gly Leu
               20                  25                  30
   gta tac agt gat gga aaa tct tac ttg agt tgg ttc cag cag agg cca    144
   Val Tyr Ser Asp Gly Lys Ser Tyr Leu Ser Trp Phe Gln Gln Arg Pro
           35                  40                  45
   ggc caa tct cca agg cgc cta att tat aaa gtt tct agc cgg gac tct    192
   Gly Gln Ser Pro Arg Arg Leu Ile Tyr Lys Val Ser Ser Arg Asp Ser
       50                  55                  60
   ggg gtc cca gac aga ttc agc ggc agt gga tca ggc act gat ttc aca    240
   Gly Val Pro Asp Arg Phe Ser Gly Ser Gly Ser Gly Thr Asp Phe Thr
   65                  70                  75                  80
   ctg aaa atc agc agg gtg gag gct gag gat gtt ggg gtt tat tac tgc    288
   Leu Lys Ile Ser Arg Val Glu Ala Glu Asp Val Gly Val Tyr Tyr Cys
                   85                  90                  95
   atg caa ggt aca cac tgg cct ggc acg ttc ggc caa ggg acc agg gtg    336
   Met Gln Gly Thr His Trp Pro Gly Thr Phe Gly Gln Gly Thr Arg Val
               100                 105                 110
   gaa atc aaa                                                        345
   Glu Ile Lys
           115
<210> 22
   <211> 115
   <212> PRT
   <213> Homo sapiens
<400> 22
   Ala Arg Cys Glu Leu Val Met Thr Gln Ser Pro Leu Ser Leu Pro Val
   1               5                   10                  15
   Thr Leu Gly Gln Pro Ala Ser Ile Ser Cys Arg Ser Ser Gln Gly Leu
               20                  25                  30
   Val Tyr Ser Asp Gly Lys Ser Tyr Leu Ser Trp Phe Gln Gln Arg Pro
           35                  40                  45
   Gly Gln Ser Pro Arg Arg Leu Ile Tyr Lys Val Ser Ser Arg Asp Ser
       50                  55                  60
   Gly Val Pro Asp Arg Phe Ser Gly Ser Gly Ser Gly Thr Asp Phe Thr
   65                  70                  75                  80
   Leu Lys Ile Ser Arg Val Glu Ala Glu Asp Val Gly Val Tyr Tyr Cys
                   85                  90                  95
   Met Gln Gly Thr His Trp Pro Gly Thr Phe Gly Gln Gly Thr Arg Val
               100                 105                 110
   Glu Ile Lys
           115
<210> 23
   <211> 366
   <212> DNA
   <213> Homo sapiens
<220>
   <221> CDS
   <222> (1)..(366)
<400> 23
   cag gtt cac ctg ggg caa tct gga gct gag gtg aag aag cct ggg gcc     48
   Gln Val His Leu Gly Gln Ser Gly Ala Glu Val Lys Lys Pro Gly Ala
   1               5                   10                  15
   tca gtg aag gtc tcc tgc aag gct tct ggt tac aag ttt tac aat tat     96
   Ser Val Lys Val Ser Cys Lys Ala Ser Gly Tyr Lys Phe Tyr Asn Tyr
               20                  25                  30
   ggt atc agt tgg gtg cga cag gcc cct gga caa ggg ctt gag tgg atg    144
   Gly Ile Ser Trp Val Arg Gln Ala Pro Gly Gln Gly Leu Glu Trp Met
           35                  40                  45
   gga tgg atc agc ggt tac aat ggt aac aca aac tat gca cac aag ttc    192
   Gly Trp Ile Ser Gly Tyr Asn Gly Asn Thr Asn Tyr Ala His Lys Phe
       50                  55                  60
   ctg ggc aga gtc acc atg acc gca gac aca tcc acg agc aca gcc tac    240
   Leu Gly Arg Val Thr Met Thr Ala Asp Thr Ser Thr Ser Thr Ala Tyr
   65                  70                  75                  80
   atg gag ttg agg agc ctg aga tcg gac gac acg gcc gta tat tac tgt    288
   Met Glu Leu Arg Ser Leu Arg Ser Asp Asp Thr Ala Val Tyr Tyr Cys
                   85                  90                  95
   gcg aga gac act aca cta atg gcg tat gct agc ctc ttt gac cac tgg    336
   Ala Arg Asp Thr Thr Leu Met Ala Tyr Ala Ser Leu Phe Asp His Trp
               100                 105                 110
   ggc cag gga acc ctg gtc acc gtc tcc tca                            366
   Gly Gln Gly Thr Leu Val Thr Val Ser Ser
           115                 120
<210> 24
   <211> 122
   <212> PRT
   <213> Homo sapiens
<400> 24
   Gln Val His Leu Gly Gln Ser Gly Ala Glu Val Lys Lys Pro Gly Ala
   1               5                   10                  15
   Ser Val Lys Val Ser Cys Lys Ala Ser Gly Tyr Lys Phe Tyr Asn Tyr
               20                  25                  30
   Gly Ile Ser Trp Val Arg Gln Ala Pro Gly Gln Gly Leu Glu Trp Met
           35                  40                  45
   Gly Trp Ile Ser Gly Tyr Asn Gly Asn Thr Asn Tyr Ala His Lys Phe
       50                  55                  60
   Leu Gly Arg Val Thr Met Thr Ala Asp Thr Ser Thr Ser Thr Ala Tyr
   65                  70                  75                  80
   Met Glu Leu Arg Ser Leu Arg Ser Asp Asp Thr Ala Val Tyr Tyr Cys
                   85                  90                  95
   Ala Arg Asp Thr Thr Leu Met Ala Tyr Ala Ser Leu Phe Asp His Trp
               100                 105                 110
   Gly Gln Gly Thr Leu Val Thr Val Ser Ser
           115                 120
<210> 25
   <211> 351
   <212> DNA
   <213> Homo sapiens
<220>
   <221> CDS
   <222> (1)..(351)
<400> 25
   tgc aca ggg tcc tgg gcc gag ctc gtg gtg act cag cct ccc tcc gcg      48
   Cys Thr Gly Ser Trp Ala Glu Leu Val Val Thr Gln Pro Pro Ser Ala
    1               5                  10                  15
   tcc ggg tct cct gga cag tca gtc acc atc tcc tgc act gga acc agc      96
   Ser Gly Ser Pro Gly Gln Ser Val Thr Ile Ser Cys Thr Gly Thr Ser
               20                  25                  30
   agt gac gtt ggt ggt tat aac tat gtc tcc tgg tac caa cag cac cca     144
   Ser Asp Val Gly Gly Tyr Asn Tyr Val Ser Trp Tyr Gln Gln His Pro
           35                  40                  45
   ggc aaa gcc ccc aaa ctc atg att ttt gag gtc act aag cgg ccc tca     192
   Gly Lys Ala Pro Lys Leu Met Ile Phe Glu Val Thr Lys Arg Pro Ser
       50                  55                  60
   ggg gtc cct gat cgc ttc tct ggc tcc aag tct ggc aac acg gcc tcc     240
   Gly Val Pro Asp Arg Phe Ser Gly Ser Lys Ser Gly Asn Thr Ala Ser
   65                  70                  75                  80
   ctg acc gtc tct ggg ctc cag gct gag gat gag gct gat tat tac tgc     288
   Leu Thr Val Ser Gly Leu Gln Ala Glu Asp Glu Ala Asp Tyr Tyr Cys
                   85                  90                  95
   acc tca tat gca ggc agc aac aat ttg gtg ttc ggc gga ggg acc aag     336
   Thr Ser Tyr Ala Gly Ser Asn Asn Leu Val Phe Gly Gly Gly Thr Lys
               100                 105                 110
   ctg acc gtc cta ggt                                                 351
   Leu Thr Val Leu Gly
           115
<210> 26
   <211> 117
   <212> PRT
   <213> Homo sapiens
<400> 26
   Cys Thr Gly Ser Trp Ala Glu Leu Val Val Thr Gln Pro Pro Ser Ala
    1               5                  10                  15
   Ser Gly Ser Pro Gly Gln Ser Val Thr Ile Ser Cys Thr Gly Thr Ser
               20                  25                  30
   Ser Asp Val Gly Gly Tyr Asn Tyr Val Ser Trp Tyr Gln Gln His Pro
           35                  40                  45
   Gly Lys Ala Pro Lys Leu Met Ile Phe Glu Val Thr Lys Arg Pro Ser
       50                  55                  60
   Gly Val Pro Asp Arg Phe Ser Gly Ser Lys Ser Gly Asn Thr Ala Ser
   65                  70                  75                  80
   Leu Thr Val Ser Gly Leu Gln Ala Glu Asp Glu Ala Asp Tyr Tyr Cys
                   85                  90                  95
   Thr Ser Tyr Ala Gly Ser Asn Asn Leu Val Phe Gly Gly Gly Thr Lys
               100                 105                 110
   Leu Thr Val Leu Gly
           115
<210> 27
   <211> 381
   <212> DNA
   <213> Homo sapiens
<220>
   <221> CDS
   <222> (1)..(381)
<400> 27
   cag gtg caa ctg ctc gag tcg ggc cca gga ctg gtg aag cct tcg gag    48
   Gln Val Gln Leu Leu Glu Ser Gly Pro Gly Leu Val Lys Pro Ser Glu
    1               5                  10                  15
   acc ctg tcc ctc acc tgc act gtc tct ggt ggc tcc atc agc agt agg    96
   Thr Leu Ser Leu Thr Cys Thr Val Ser Gly Gly Ser Ile Ser Ser Arg
               20                  25                  30
   aat tac tac tgg ggc tgg atc cgc cag ccc cca ggg aag ggg ctg gag    144
   Asn Tyr Tyr Trp Gly Trp Ile Arg Gln Pro Pro Gly Lys Gly Leu Glu
           35                  40                  45
   tgg att gga agt att tat cat agt ggg aga acc tac tat aac ttg tcc    192
   Trp Ile Gly Ser Ile Tyr His Ser Gly Arg Thr Tyr Tyr Asn Leu Ser
       50                  55                  60
   ctc aag agt cga gtc acc ata tcc gta gac acg tcc aag aac cag ttc    240
   Leu Lys Ser Arg Val Thr Ile Ser Val Asp Thr Ser Lys Asn Gln Phe
    65                  70                  75                  80
   tcc ctg aag ctg agc tct gtg acc gcc gca gac acg gct gtg tat tac    288
   Ser Leu Lys Leu Ser Ser Val Thr Ala Ala Asp Thr Ala Val Tyr Tyr
                   85                  90                  95
   tgt gcg agg tca tat tgt agt gct gtt acc tgc tac cca gac tac tac    336
   Cys Ala Arg Ser Tyr Cys Ser Ala Val Thr Cys Tyr Pro Asp Tyr Tyr
               100                 105                 110
   ggt ctg gac atc tgg ggc caa ggg acc acg gtc acc gtc tcc tca        381
   Gly Leu Asp Ile Trp Gly Gln Gly Thr Thr Val Thr Val Ser Ser
           115                 120                 125
<210> 28
   <211> 127
   <212> PRT
   <213> Homo sapiens
<400> 28
   Gln Val Gln Leu Leu Glu Ser Gly Pro Gly Leu Val Lys Pro Ser Glu
    1               5                  10                  15
   Thr Leu Ser Leu Thr Cys Thr Val Ser Gly Gly Ser Ile Ser Ser Arg
               20                  25                  30
   Asn Tyr Tyr Trp Gly Trp Ile Arg Gln Pro Pro Gly Lys Gly Leu Glu
           35                  40                  45
   Trp Ile Gly Ser Ile Tyr His Ser Gly Arg Thr Tyr Tyr Asn Leu Ser
       50                  55                  60
   Leu Lys Ser Arg Val Thr Ile Ser Val Asp Thr Ser Lys Asn Gln Phe
   65                  70                  75                  80
   Ser Leu Lys Leu Ser Ser Val Thr Ala Ala Asp Thr Ala Val Tyr Tyr
                   85                  90                  95
   Cys Ala Arg Ser Tyr Cys Ser Ala Val Thr Cys Tyr Pro Asp Tyr Tyr
               100                 105                 110
   Gly Leu Asp Ile Trp Gly Gln Gly Thr Thr Val Thr Val Ser Ser
           115                 120                 125
<210> 29
   <211> 294
   <212> DNA
   <213> Homo sapiens
<220>
   <221> CDS
   <222> (1)..(294)
<400> 29
<210> 30
   <211> 98
   <212> PRT
   <213> Homo sapiens
<400> 30
   Ala Arg Cys Glu Leu Val Met Thr Gln Ser Pro Ser Thr Leu Ser Ala
    1               5                  10                  15
   Ser Val Gly Asp Arg Val Thr Ile Thr Cys Arg Ala Ser Gln Asn Ile
               20                  25                  30
   Asn Ser Trp Leu Ala Trp Tyr Gln Gln Lys Pro Gly Lys Ala Pro Asn
           35                  40                  45
   Leu Leu Ile Ser Lys Ala Ser Thr Leu Glu Ser Gly Val Pro Ser Arg
       50                  55                  60
   Phe Ser Gly Ser Gly Ser Gly Thr Glu Phe Thr Leu Thr Ile Ser Ser
   65                  70                  75                  80
   Leu Gln Pro Asp Asp Phe Ala Ser Tyr Tyr Cys Gln Gln Tyr Lys Asn
                   85                  90                  95
   Tyr Ser
<210> 31
   <211> 348
   <212> DNA
   <213> Homo sapiens
<220>
   <221> CDS
   <222> (1)..(348)
<400> 31
   gtg cag ctg ctc gag tct ggg gct gag gtg aag aag cct ggg tcc tcg    48
   Val Gln Leu Leu Glu Ser Gly Ala Glu Val Lys Lys Pro Gly Ser Ser
     1               5                  10                  15
   gtg aag gtc tcc tgc aag gct tct gga ggc acg ttc aac att tat tct    96
   Val Lys Val Ser Cys Lys Ala Ser Gly Gly Thr Phe Asn Ile Tyr Ser
                20                  25                  30
   ttc agt tgg gtg cga cag gcc cct gga caa ggg ctt gaa tgg atg gga   144
   Phe Ser Trp Val Arg Gln Ala Pro Gly Gln Gly Leu Glu Trp Met Gly
            35                  40                  45
   gga atc atc cct atc tat cgt cca gca aat tac gca cag aat ttc cag   192
   Gly Ile Ile Pro Ile Tyr Arg Pro Ala Asn Tyr Ala Gln Asn Phe Gln
        50                  55                  60
   ggc aga gtc acg att acc gcg gac gaa tcc acg agc aca gtc tac atg   240
   Gly Arg Val Thr Ile Thr Ala Asp Glu Ser Thr Ser Thr Val Tyr Met
    65                  70                  75                  80
   gac ctg agc agc ctg agg tct gac gac acg gcc atg tat tac tgt gcg   288
   Asp Leu Ser Ser Leu Arg Ser Asp Asp Thr Ala Met Tyr Tyr Cys Ala
                    85                  90                  95
   aga ggg aca ggg ggt tat tac aac tcc tgg ggc cag gga acc ctg gtc   336
   Arg Gly Thr Gly Gly Tyr Tyr Asn Ser Trp Gly Gln Gly Thr Leu Val
               100                 105                 110
   acc gtc tcc tca                                                   348
   Thr Val Ser Ser
           115
<210> 32
   <211> 116
   <212> PRT
   <213> Homo sapiens
<400> 32
   Val Gln Leu Leu Glu Ser Gly Ala Glu Val Lys Lys Pro Gly Ser Ser
    1               5                  10                  15
   Val Lys Val Ser Cys Lys Ala Ser Gly Gly Thr Phe Asn Ile Tyr Ser
               20                  25                  30
   Phe Ser Trp Val Arg Gln Ala Pro Gly Gln Gly Leu Glu Trp Met Gly
           35                  40                  45
   Gly Ile Ile Pro Ile Tyr Arg Pro Ala Asn Tyr Ala Gln Asn Phe Gln
       50                  55                  60
   Gly Arg Val Thr Ile Thr Ala Asp Glu Ser Thr Ser Thr Val Tyr Met
   65                  70                  75                  80
   Asp Leu Ser Ser Leu Arg Ser Asp Asp Thr Ala Met Tyr Tyr Cys Ala
                   85                  90                  95
   Arg Gly Thr Gly Gly Tyr Tyr Asn Ser Trp Gly Gln Gly Thr Leu Val
               100                 105                 110
   Thr Val Ser Ser
           115
<210> 33
   <211> 331
   <212> DNA
   <213> Homo sapiens
<220>
   <221> CDS
   <222> (1)..(330)
<400> 33
   cag tct gtg ctg act cag cca ccc tca gcg tct ggg acc ccc ggg cag     48
   Gln Ser Val Leu Thr Gln Pro Pro Ser Ala Ser Gly Thr Pro Gly Gln
    1               5                  10                  15
   agg gtc acc atc tct tgt tct gga agc agc tcc aac atc gga agt gat     96
   Arg Val Thr Ile Ser Cys Ser Gly Ser Ser Ser Asn Ile Gly Ser Asp
               20                  25                  30
   agt gtg tgc tgg tac cag cac ctc cca gga acg gcc ccc aaa ctc ctc     144
   Ser Val Cys Trp Tyr Gln His Leu Pro Gly Thr Ala Pro Lys Leu Leu
           35                  40                  45
   atc tat ggt aat aat ctg cgg ccc tca tgg gtc tct gac cga ttc tct     192
   Ile Tyr Gly Asn Asn Leu Arg Pro Ser Trp Val Ser Asp Arg Phe Ser
       50                  55                  60
   ggc tcc gag tct ggc acc tca gcc tcc ctg gcc atc agt ggg ctc cag     240
   Gly Ser Glu Ser Gly Thr Ser Ala Ser Leu Ala Ile Ser Gly Leu Gln
    65                 70                  75                  80
   tct gag gat gag gct gat tat tac tgt tca gca tgg gat gac tac ctg     288
   Ser Glu Asp Glu Ala Asp Tyr Tyr Cys Ser Ala Trp Asp Asp Tyr Leu
                   85                  90                  95
   aat gct gtg gta ttc ggc gga ggg acc aag ctg acc gtc cta g           331
   Asn Ala Val Val Phe Gly Gly Gly Thr Lys Leu Thr Val Leu
               100                 105                 110
<210> 34
   <211> 110
   <212> PRT
   <213> Homo sapiens
<400> 34
   Gln Ser Val Leu Thr Gln Pro Pro Ser Ala Ser Gly Thr Pro Gly Gln
    1               5                  10                  15
   Arg Val Thr Ile Ser Cys Ser Gly Ser Ser Ser Asn Ile Gly Ser Asp
               20                  25                  30
   Ser Val Cys Trp Tyr Gln His Leu Pro Gly Thr Ala Pro Lys Leu Leu
           35                  40                  45
   Ile Tyr Gly Asn Asn Leu Arg Pro Ser Trp Val Ser Asp Arg Phe Ser
       50                  55                  60
   Gly Ser Glu Ser Gly Thr Ser Ala Ser Leu Ala Ile Ser Gly Leu Gln
    65                 70                  75                  80
   Ser Glu Asp Glu Ala Asp Tyr Tyr Cys Ser Ala Trp Asp Asp Tyr Leu
                   85                  90                  95
   Asn Ala Val Val Phe Gly Gly Gly Thr Lys Leu Thr Val Leu
               100                 105                 110
<210> 35
   <211> 378
   <212> DNA
   <213> Homo sapiens
<220>
   <221> CDS
   <222> (1)..(378)
<400> 35
   cag ctg cag ctg cag gag tcg ggc cca gga ctg gtg aag cct tcg gag      48
   Gln Leu Gln Leu Gln Glu Ser Gly Pro Gly Leu Val Lys Pro Ser Glu
     1               5                  10                  15
   acc ctg tcc ctc acc tgc act gtc tct ggt ggc gcc gtc agc agt ggt      96
   Thr Leu Ser Leu Thr Cys Thr Val Ser Gly Gly Ala Val Ser Ser Gly
                20                  25                  30
   agt tac tac tgg ggc tgg atc cgc cag tcc cca ggg aag ggg ctg gag     144
   Ser Tyr Tyr Trp Gly Trp Ile Arg Gln Ser Pro Gly Lys Gly Leu Glu
            35                  40                  45
   tgg att ggg aat atc ttt tat agt ggg acc acc tac aaa aac ccg tcc     192
   Trp Ile Gly Asn Ile Phe Tyr Ser Gly Thr Thr Tyr Lys Asn Pro Ser
        50                  55                  60
   ctc agg agt cga gtc acc ata tcc gta gac acg tcc aag aac cag ttc     240
   Leu Arg Ser Arg Val Thr Ile Ser Val Asp Thr Ser Lys Asn Gln Phe
    65                  70                  75                  80
   tcc ctg aag gcg aac tct gtg acc gcc gca gac acg gct gtg tat ttc     288
   Ser Leu Lys Ala Asn Ser Val Thr Ala Ala Asp Thr Ala Val Tyr Phe
                    85                  90                  95
   tgt gcg aga caa cgg gtc gct ggg tgg tat agt ccg gcg acg aac tgg     336
   Cys Ala Arg Gln Arg Val Ala Gly Trp Tyr Ser Pro Ala Thr Asn Trp
               100                 105                 110
   ttc gac ccc tgg ggc cag gga acc ctg gtc atc gtc tcc tca             378
   Phe Asp Pro Trp Gly Gln Gly Thr Leu Val Ile Val Ser Ser
           115                 120                 125
<210> 36
   <211> 126
   <212> PRT
   <213> Homo sapiens
<400> 36
   Gln Leu Gln Leu Gln Glu Ser Gly Pro Gly Leu Val Lys Pro Ser Glu
   1               5                   10                  15
   Thr Leu Ser Leu Thr Cys Thr Val Ser Gly Gly Ala Val Ser Ser Gly
               20                  25                  30
   Ser Tyr Tyr Trp Gly Trp Ile Arg Gln Ser Pro Gly Lys Gly Leu Glu
           35                  40                  45
   Trp Ile Gly Asn Ile Phe Tyr Ser Gly Thr Thr Tyr Lys Asn Pro Ser
       50                  55                  60
   Leu Arg Ser Arg Val Thr Ile Ser Val Asp Thr Ser Lys Asn Gln Phe
   65                  70                  75                  80
   Ser Leu Lys Ala Asn Ser Val Thr Ala Ala Asp Thr Ala Val Tyr Phe
                   85                  90                  95
   Cys Ala Arg Gln Arg Val Ala Gly Trp Tyr Ser Pro Ala Thr Asn Trp
               100                 105                 110
   Phe Asp Pro Trp Gly Gln Gly Thr Leu Val Ile Val Ser Ser
           115                 120                 125
<210> 37
   <211> 331
   <212> DNA
   <213> Homo sapiens
<220>
   <221> CDS
   <222> (1)..(330)
<400> 37
   cag tct gtg gtg act cag ccg ccc tca gtg tct ggg gcc cca ggg cag    48
   Gln Ser Val Val Thr Gln Pro Pro Ser Val Ser Gly Ala Pro Gly Gln
   1               5                   10                  15
   agg gtc acc atc tcc tgc act ggg agc agc tcc aac atc ggg gca ggt    96
   Arg Val Thr Ile Ser Cys Thr Gly Ser Ser Ser Asn Ile Gly Ala Gly
                   20                  25                  30
   tat gat gta cac tgg tac cag cag ctt cca gga aca gcc ccc aaa ctc      144
   Tyr Asp Val His Trp Tyr Gln Gln Leu Pro Gly Thr Ala Pro Lys Leu
               35                  40                  45
   ctc atc tat ggt aac agc aat cgg ccc tca ggg gtc cct gac cga ttc      192
   Leu Ile Tyr Gly Asn Ser Asn Arg Pro Ser Gly Val Pro Asp Arg Phe
           50                  55                  60
   tct ggc tcc aag tct ggc acc tca gcc tcc ctg gcc atc act ggg ctc      240
   Ser Gly Ser Lys Ser Gly Thr Ser Ala Ser Leu Ala Ile Thr Gly Leu
    65                  70                  75                  80
   cag gct gag gat gag gct gat tat tac tgc cag tcc tat gac agc agc      288
   Gln Ala Glu Asp Glu Ala Asp Tyr Tyr Cys Gln Ser Tyr Asp Ser Ser
                    85                  90                  95
   ctg agt ggt tca ttc ggc gga cgg acc aag ctg acc gtc cta g            331
   Leu Ser Gly Ser Phe Gly Gly Arg Thr Lys Leu Thr Val Leu
               100                 105                 110
<210> 38
   <211> 110
   <212> PRT
   <213> Homo sapiens
<400> 38
   Gln Ser Val Val Thr Gln Pro Pro Ser Val Ser Gly Ala Pro Gly Gln
    1               5                  10                  15
   Arg Val Thr Ile Ser Cys Thr Gly Ser Ser Ser Asn Ile Gly Ala Gly
               20                  25                  30
   Tyr Asp Val His Trp Tyr Gln Gln Leu Pro Gly Thr Ala Pro Lys Leu
           35                  40                  45
   Leu Ile Tyr Gly Asn Ser Asn Arg Pro Ser Gly Val Pro Asp Arg Phe
       50                  55                  60
   Ser Gly Ser Lys Ser Gly Thr Ser Ala Ser Leu Ala Ile Thr Gly Leu
    65                  70                  75                  80
   Gln Ala Glu Asp Glu Ala Asp Tyr Tyr Cys Gln Ser Tyr Asp Ser Ser
                    85                  90                  95
   Leu Ser Gly Ser Phe Gly Gly Arg Thr Lys Leu Thr Val Leu
               100                 105                 110
<210> 39
   <211> 379
   <212> DNA
   <213> Homo sapiens
<220>
   <221> CDS
   <222> (1)..(378)
<400> 39
   gag gtg cag ctg gtg gag tct ggg gga ggc ttg gta aag cct ggg ggg    48
   Glu Val Gln Leu Val Glu Ser Gly Gly Gly Leu Val Lys Pro Gly Gly
   1               5                   10                  15
   tcc ctt aga ctc tcc tgt gca gcc tct gga ttc act ttc att aac gcc    96
   Ser Leu Arg Leu Ser Cys Ala Ala Ser Gly Phe Thr Phe Ile Asn Ala
               20                  25                  30
   tgg atg aac tgg gtc cgc cag gct cca ggg aag ggg ctg gag tgg gtt    144
   Trp Met Asn Trp Val Arg Gln Ala Pro Gly Lys Gly Leu Glu Trp Val
           35                  40                  45
   ggc cgt att aga agc aac att gat ggt ggg aca aca gac tac gct gca    192
   Gly Arg Ile Arg Ser Asn Ile Asp Gly Gly Thr Thr Asp Tyr Ala Ala
       50                  55                  60
   ccc gtg aaa ggc aga ttc acc atc tca aga gat gat tca aaa aac acg    240
   Pro Val Lys Gly Arg Phe Thr Ile Ser Arg Asp Asp Ser Lys Asn Thr
   65                  70                  75                  80
   ctg ttt ctg caa atg aac acc ctg aaa acc gag gac aca gcc gtg tat    288
   Leu Phe Leu Gln Met Asn Thr Leu Lys Thr Glu Asp Thr Ala Val Tyr
                   85                  90                  95
   tac tgt gtc aca gat ggg tat tac gag tct tgg agt ggt cct tat cct    336
   Tyr Cys Val Thr Asp Gly Tyr Tyr Glu Ser Trp Ser Gly Pro Tyr Pro
               100                 105                 110
   ttt gat gtc tgg ggc caa ggg aca atg gtc acc gtc tct tca g          379
   Phe Asp Val Trp Gly Gln Gly Thr Met Val Thr Val Ser Ser
           115                 120                 125
<210> 40
   <211> 126
   <212> PRT
   <213> Homo sapiens
<400> 40
   Glu Val Gln Leu Val Glu Ser Gly Gly Gly Leu Val Lys Pro Gly Gly
    1               5                  10                  15
   Ser Leu Arg Leu Ser Cys Ala Ala Ser Gly Phe Thr Phe Ile Asn Ala
               20                  25                  30
   Trp Met Asn Trp Val Arg Gln Ala Pro Gly Lys Gly Leu Glu Trp Val
           35                  40                  45
   Gly Arg Ile Arg Ser Asn Ile Asp Gly Gly Thr Thr Asp Tyr Ala Ala
       50                  55                  60
   Pro Val Lys Gly Arg Phe Thr Ile Ser Arg Asp Asp Ser Lys Asn Thr
   65                  70                  75                  80
   Leu Phe Leu Gln Met Asn Thr Leu Lys Thr Glu Asp Thr Ala Val Tyr
                   85                  90                  95
   Tyr Cys Val Thr Asp Gly Tyr Tyr Glu Ser Trp Ser Gly Pro Tyr Pro
               100                 105                 110
   Phe Asp Val Trp Gly Gln Gly Thr Met Val Thr Val Ser Ser
           115                 120                 125
<210> 41
   <211> 5920
   <212> DNA
   <213> Homo sapiens
<400> 41
   gatccgatgc agcaagacaa ggagcgcctg gcagccctgg ggcctgaggg ggcccatggt 60
   gacactacct ttgagtacca ggacctgtgc cgccagcaca tggccacgaa gtccttgttc 120
   aaccgggcag agggtccacc ggagccttca cgggtgagca gtgtgtcctc ccagttcagc 180
   gacgcagccc aggccagccc cagctcccac agcagcaccc cgtcctggtg cgaggagccg 240
   gcccaagcca acatggacat ctccacggga cacatgattc tggcatacat ggaggatcac 300
   ctgcggaacc gggaccgcct tgccaaggag tggcaggccc tctgtgccta ccaagcagag 360
   ccaaacacct gtgccaccgc gcagggggag ggcaacatca aaaagaaccg gcatcctgac 420
   ttcctgccct atgaccatgc ccgcataaaa ctgaaggtgg agagcagccc ttctcggagc 480
   gattacatca acgccagccc cattattgag catgaccctc ggatgccagc ctacatagcc 540
   acgcagggcc cgctgtccca taccatcgca gacttctggc agatggtgtg ggagagcggc 600
   tgcaccgtca tcgtcatgct gaccccgctg gtggaggatg gtgtcaagca gtgtgaccgc 660
   tactggccag atgagggtgc ctccctctac cacgtatatg aggtgaacct ggtgtcggag 720
   cacatctggt gcgaggactt tctggtgcgg agcttctacc tgaagaacgt gcagacccag 780
   gagacgcgca cgctcacgca gttccacttc ctcagctggc cggcagaggg cacaccggcc 840
   tccacgcggc ccctgctgga cttccgcagg aaggtgaaca agtgctaccg gggccgctcc 900
   tgccccatca tcgtgcactg cagtgatggt gcggggagga ccggcaccta catcctcatc 960
   gacatggtcc tgaaccgcat ggcaaaagga gtgaaggaga ttgacatcgc tgccaccctg 1020
   gagcatgtcc gtgaccagcg gcctggcctt gtccgctcta aggaccagtt tgaatttgcc 1080
   ctgacagccg tggcggagga agtgaatgcc atcctcaagg ccctgcccca gctcgagatg 1140
   gcatctccgg gctctggctt ttggtctttc gggtcggaag atggctctgg ggattccgag 1200
   aatcccggca cagcgcgagc ctggtgccaa gtggctcaga agttcacggg cggcatcgga 1260
   aacaaactgt gcgccctgct ctacggagac gccgagaagc cggcggagag cggcgggagc 1320
   caacccccgc gggccgccgc ccggaaggcc gcctgcgcct gcgaccagaa gccctgcagc 1380
   tgctccaaag tggatgtcaa ctacgcgttt ctccatgcaa cagacctgct gccggcgtgt 1440
   gatggagaaa ggcccacttt ggcgtttctg caagatgtta tgaacatttt acttcagtat 1500
   gtggtgaaaa gtttcgatag atcaaccaaa gtgattgatt tccattatcc taatgagctt 1560
   ctccaagaat ataattggga attggcagac caaccacaaa atttggagga aattttgatg 1620
   cattgccaaa caactctaaa atatgcaatt aaaacagggc atcctagata cttcaatcaa 1680
   ctttctactg gtttggatat ggttggatta gcagcagact ggctgacatc aacagcaaat 1740
   actaacatgt tcacctatga aattgctcca gtatttgtgc ttttggaata tgtcacacta 1800
   aagaaaatga gagaaatcat tggctggcca gggggctctg gcgatgggat attttctccc 1860
   ggtggcgcca tatctaacat gtatgccatg atgatcgcac gctttaagat gttcccagaa 1920
   gtcaaggaga aaggaatggc tgctcttccc aggctcattg ccttcacgtc tgaacatagt 1980
   catttttctc tcaagaaggg agctgcagcc ttagggattg gaacagacag cgtgattctg 2040
   attaaatgtg atgagagagg gaaaatgatt ccatctgatc ttgaaagaag gattcttgaa 2100
   gccaaacaga aagggtttgt tcctttcctc gtgagtgcca cagctggaac caccgtgtac 2160
   ggagcatttg accccctctt agctgtcgct gacatttgca aaaagtataa gatctggatg 2220
   catgtggatg cagcttgggg tgggggatta ctgatgtccc gaaaacacaa gtggaaactg 2280
   agtggcgtgg agagggccaa ctctgtgacg tggaatccac acaagatgat gggagtccct 2340
   ttgcagtgct ctgctctcct ggttagagaa gagggattga tgcagaattg caaccaaatg 2400
   catgcctcct acctctttca gcaagataaa cattatgacc tgtcctatga cactggagac 2460
   aaggccttac agtgcggacg ccacgttgat gtttttaaac tatggctgat gtggagggca 2520
   aaggggacta ccgggtttga agcgcatgtt gataaatgtt tggagttggc agagtattta 2580
   tacaacatca taaaaaaccg agaaggatat gagatggtgt ttgatgggaa gcctcagcac 2640
   acaaatgtct gcttctggta cattcctcca agcttgcgta ctctggaaga caatgaagag 2700
   agaatgagtc gcctctcgaa ggtggctcca gtgattaaag ccagaatgat ggagtatgga 2760
   accacaatgg tcagctacca acccttggga gacaaggtca atttcttccg catggtcatc 2820
   tcaaacccag cggcaactca ccaagacatt gacttcctga ttgaagaaat agaacgcctt 2880
   ggacaagatt tataataaga gctcccaaaa aaaaaaaaaa aaaaaaaaaa aaaaaaccga 2940
   attcgtaatc atggtcatag ctgtttcctg tgtgaaattg ttatccgctc acaattccac 3000
   acaacatacg agccggaagc ataaagtgta aagcctgggg tgcctaatga gtgagctaac 3060
   tcacattaat tgcgttgcgc tcactgcccg ctttccagtc gggaaacctg tcgtgccagc 3120
   tgcattaatg aatcggccaa cgcgcgggga gaggcggttt gcgtattggg cgctcttccg 3180
   cttcctcgct cactgactcg ctgcgctcgg tcgttcggct gcggcgagcg gtatcagctc 3240
   actcaaaggc ggtaatacgg ttatccacag aatcagggga taacgcagga aagaacatgt 3300
   gagcaaaagg ccagcaaaag gccaggaacc gtaaaaaggc cgcgttgctg gcgtttttcc 3360
   ataggctccg cccccctgac gagcatcaca aaaatcgacg ctcaagtcag aggtggcgaa 3420
   acccgacagg actataaaga taccaggcgt ttccccctgg aagctccctc gtgcgctctc 3480
   ctgttccgac cctgccgctt accggatacc tgtccgcctt tctcccttcg ggaagcgtgg 3540
   cgctttctca tagctcacgc tgtaggtatc tcagttcggt gtaggtcgtt cgctccaagc 3600
   tgggctgtgt gcacgaaccc cccgttcagc ccgaccgctg cgccttatcc ggtaactatc 3660
   gtcttgagtc caacccggta agacacgact tatcgccact ggcagcagcc actggtaaca 3720
   ggattagcag agcgaggtat gtaggcggtg ctacagagtt cttgaagtgg tggcctaact 3780
   acggctacac tagaaggaca gtatttggta tctgcgctct gctgaagcca gttaccttcg 3840
   gaaaaagagt tggtagctct tgatccggca aacaaaccac cgctggtagc ggtggttttt 3900
   ttgtttgcaa gcagcagatt acgcgcagaa aaaaaggatc tcaagaagat cctttgatct 3960
   tttctacggg gtctgacgct cagtggaacg aaaactcacg ttaagggatt ttggtcatga 4020
   gattatcaaa aaggatcttc acctagatcc ttttaaatta aaaatgaagt tttaaatcaa 4080
   tctaaagtat atatgagtaa acttggtctg acagttacca atgcttaatc agtgaggcac 4140
   ctatctcagc gatctgtcta tttcgttcat ccatagttgc ctgactcccc gtcgtgtaga 4200
   taactacgat acgggagggc ttaccatctg gccccagtgc tgcaatgata ccgcgagacc 4260
   cacgctcacc ggctccagat ttatcagcaa taaaccagcc agccggaagg gccgagcgca 4320
   gaagtggtcc tgcaacttta tccgcctcca tccagtctat taattgttgc cgggaagcta 4380
   gagtaagtag ttcgccagtt aatagtttgc gcaacgttgt tgccattgct acaggcatcg 4440
   tggtgtcacg ctcgtcgttt ggtatggctt cattcagctc cggttcccaa cgatcaaggc 4500
   gagttacatg atcccccatg ttgtgcaaaa aagcggttag ctccttcggt cctccgatcg 4560
   ttgtcagaag taagttggcc gcagtgttat cactcatggt tatggcagca ctgcataatt 4620
   ctcttactgt catgccatcc gtaagatgct tttctgtgac tggtgagtac tcaaccaagt 4680
   cattctgaga atagtgtatg cggcgaccga gttgctcttg cccggcgtca atacgggata 4740
   ataccgcgcc acatagcaga actttaaaag tgctcatcat tggaaaacgt tcttcggggc 4800
   gaaaactctc aaggatctta ccgctgttga gatccagttc gatgtaaccc actcgtgcac 4860
   ccaactgatc ttcagcatct tttactttca ccagcgtttc tgggtgagca aaaacaggaa 4920
   ggcaaaatgc cgcaaaaaag ggaataaggg cgacacggaa atgttgaata ctcatactct 4980
   tcctttttca atattattga agcatttatc agggttattg tctcatgagc ggatacatat 5040
   ttgaatgtat ttagaaaaat aaacaaatag gggttccgcg cacatttccc cgaaaagtgc 5100
   cacctgacgt ctaagaaacc attattatca tgacattaac ctataaaaat aggcgtatca 5160
   cgaggccctt tcgtctcgcg cgtttcggtg atgacggtga aaacctctga cacatgcagc 5220
   tcccggagac ggtcacagct tgtctgtaag cggatgccgg gagcagacaa gcccgtcagg 5280
   gcgcgtcagc gggtgttggc gggtgtcggg gctggcttaa ctatgcggca tcagagcaga 5340
   ttgtactgag agtgcaccat atcgacgctc tcccttatgc gactcctgca ttaggaagca 5400
   gcccagtagt aggttgaggc cgttgagcac cgccgccgca aggaatggtg catgcaagga 5460
   gatggcgccc aacagtcccc cggccacggg gcctgccacc atacccacgc cgaaacaagc 5520
   gctcatgagc ccgaagtggc gagcccgatc ttccccatcg gtgatgtcgg cgatataggc 5580
   gccagcaacc gcacctgtgg cgccggtgat gccggccacg atgcgtccgg cgtagaggat 5640
   ctggctagcg atgaccctgc tgattggttc gctgaccatt tccggggtgc ggaacggcgt 5700
   taccagaaac tcagaaggtt cgtccaacca aaccgactct gacggcagtt tacgagagag 5760
   atgatagggt ctgcttcagt aagccagatg ctacacaatt aggcttgtac atattgtcgt 5820
   tagaacgcgg ctacaattaa tacataacct tatgtatcat acacatacga tttaggtgac 5880
   actatagaat acaagcttgg gctgcaggtc gactctagag                      5920
<210> 42
   <211> 4665
   <212> DNA
   <213> Homo sapiens
<400> 42